(19) Europäisches Patentamt European Patent Office Office européen des brevets

(11) **EP 3 741 843 B1**

(12) **EUROPEAN PATENT SPECIFICATION**

(45) Date of publication and mention of the grant of the patent:
**26.06.2024 Bulletin 2024/26**

(21) Application number: **20160435.2**

(22) Date of filing: **02.01.2014**

(51) International Patent Classification (IPC):
**C12N 5/0784** *(2010.01)* **A61K 39/00** *(2006.01)*

(52) Cooperative Patent Classification (CPC):
**A61K 35/15; A61K 39/4615; A61K 39/464499; C12N 5/0639;** A61K 2035/124; C12N 2502/115; C12N 2521/00; C12N 2529/10

(54) **METHOD FOR OBTAINING IMMUNO-STIMULATORY DENDRITIC CELLS**

VERFAHREN ZUR GEWINNUNG VON IMMUNSTIMULATORISCHEN DENDRITISCHEN ZELLEN

PROCÉDÉ POUR L'OBTENTION DE CELLULES DENDRITIQUES IMMUNOSTIMULATRICES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.01.2013 GB 201300049**
**03.01.2013 US 201361748546 P**

(43) Date of publication of application:
**25.11.2020 Bulletin 2020/48**

(62) Document number(s) of the earlier application(s) in accordance with Art. 76 EPC:
**14700025.1 / 2 941 483**

(73) Proprietors:
• **Transimmune AG**
**40212 Düsseldorf (DE)**
• **Yale University**
**New Haven, CT 06511 (US)**

(72) Inventors:
• **Henco, Karsten**
**40629 Düsseldorf (DE)**
• **Bauer, Günter**
**24640 Schmalfeld (DE)**
• **Duckworth, Justin**
**Weybridge, Surrey KT13 8XB (GB)**
• **Hayday, Adrian**
**Orpington, Kent BR6 9DR (GB)**
• **Edelson, Richard**
**Westport, Connecticut 06880 (US)**
• **Tigelaar, Robert**
**New Haven, Connecticut 06512 (US)**
• **Girardi, Michael**
**Madison, Connecticut 06443 (US)**

(74) Representative: **Maiwald GmbH**
**Elisenhof**
**Elisenstraße 3**
**80335 München (DE)**

(56) References cited:
**WO-A1-2011/137365**

• **BERGER CAROLE ET AL: "Rapid generation of maturationally synchronized human dendritic cells: contribution to the clinical efficacy of extracorporeal photochemotherapy.", BLOOD 2 DEC 2010, vol. 116, no. 23, 2 December 2010 (2010-12-02), pages 4838-4847, XP055112768, ISSN: 1528-0020**
• **HOFFMANN K E ET AL: "Induction of immunomodulatory dendritic cells by transimmunization", JOURNAL OF INVESTIGATIVE DERMATOLOGY, vol. 127, no. Suppl. 1, April 2007 (2007-04), page S117, XP055113432, & 68TH ANNUAL MEETING OF THE SOCIETY-FOR-INVESTIGATIVE-DERMATOLOG Y; LOS ANGELES, CA, USA; MAY 09 -12, 2007 ISSN: 0022-202X**
• **ENGBERG A K ET AL: "Generation of tumor-specific anti-melanoma T-cell responses by extracorporeal photochemotherapy-induced dendritic cells", JOURNAL OF INVESTIGATIVE DERMATOLOGY, vol. 131, no. Suppl. 1, April 2011 (2011-04), page S94, XP055113416, & ANNUAL MEETING OF THE SOCIETY-FOR-INVESTIGATIVE-DERMATOLOG Y; PHOENIX, AZ, USA; MAY 04 -07, 2011 ISSN: 0022-202X**

- KHALIL D ET AL: "Conversion of monocytes to dendritic cells by platelet signaling", JOURNAL OF INVESTIGATIVE DERMATOLOGY, vol. 132, no. Suppl. 1, May 2012 (2012-05) , page S101, XP055113116, & 75TH ANNUAL MEETING OF THE SOCIETY-FOR-INVESTIGATIVE-DERMATOLOGY; RALEIGH, NC, USA; MAY 09 -12, 2012 ISSN: 0022-202X
- DURAZZO T S ET AL: "Platelet induction of monocyle-to-dendritic cell maturation", JOURNAL OF INVESTIGATIVE DERMATOLOGY, vol. 130, no. Suppl. 1, April 2010 (2010-04), page S121, XP055113123, & ANNUAL MEETING OF THE SOCIETY-FOR-INVESTIGATIVE-DERMATOLOGY; ATLANTA, GA, USA; MAY 05 -08, 2010 ISSN: 0022-202X
- SALSKOV-IVERSEN MARIA ET AL: "Rapid construction of a dendritic cell vaccine through physical perturbation and apoptotic malignant T cell loading", JOURNAL OF IMMUNE BASED THERAPIES AND VACCINES, BIOMED CENTRAL LTD., LONDON, GB, vol. 3, no. 1, 19 July 2005 (2005-07-19), page 4, XP021008598, ISSN: 1476-8518, DOI: 10.1186/1476-8518-3-4

Remarks:

The complete document including Reference Table(s) and the Sequence Listing(s) can be downloaded from the EPO website

**Description**

**FIELD OF THE INVENTION**

The present invention relates to methods for producing immuno-stimulatory dendritic cells. The present invention in particular relates to a method of preferentially producing immuno-stimulatory dendritic cells relative to immuno-suppressive dendritic cells

**BACKGROUND OF THE INVENTION**

Dendritic cells (DC) are recognized to be potent antigen presenting cells for the initiation and control of cellular immunologic responses in humans. Since DC can either be immuno-stimulatory or immuno-suppressive, depending on which set of their potential properties they express at the moment of interaction with responsive specific clones of T cells, they are considered profoundly important pivotal players in T cell-mediated immune reactions. As a broad, but widely held generalization, immature DC are more "tolerogenic" than their more mature counterparts, while mature DC are thought to be more "immunogenic" than their immature precursors. The capacity of DC, generated ex vivo from monocytes and armed with specific antigen, to function effectively in either immunologic direction, is dependent on their viability and vigor after being returned to the patient. It is logically concluded that the balance between counteractive immunostimulatory and immunosuppressive DC will be a major determinant of both the direction and potency of DC-dependent therapeutic immune responses.

The purpose of this invention is to facilitate production of DC populations particularly conducive to the generation of powerful and clinically relevant immune responses. Despite the tremendous promise of DC-based therapy, such as efforts to enhance anti-cancer immunity, clinical results have generally been disappointing. For example, Provenge, the recently first FDA-approved immunotherapy for a solid tumor, adapting the conventional method of ex vivo production of DC from blood monocytes, has yielded merely a four-month improvement in survival of patients with advanced prostate cancer. That conventional method of inducing DC, as well as Extracorporeal Photopheresis (ECP), an FDA-approved therapy for the "liquid" malignancy Cutaneous T Cell Lymphoma (CTCL), is encumbered by production of relatively heterogeneous DC populations under conditions which handicap the in vivo vigor, and viability, of the resulting DC. By employing more physiologic conditions to the production of therapeutic DC, the present invention enables production of more maturationally synchronized DC, whose survival and vigor are not inhibited by factors inherent to the method by which they are produced. Moreover, this method is applicable to both human and animal leukocytes.

DC prime both CD8$^+$ cytotoxic T-cell (CTL) and CD4$^+$ T helper (Th1) responses. DC are capable of capturing and processing antigens and migrating to the regional lymph nodes to present the captured antigens and induce T-cell responses. In humans, DC are a relatively rare component of peripheral blood (<1 % of leukocytes). However, large quantities of DC can be differentiated by laboratory procedures from CD34$^+$ precursors or blood monocytes.

For the afore-described properties, DC have been identified as important cellular agents for eliciting effective anti-tumor immune responses. The idea is to generate DC, which present tumor-specific antigens on their MHC Class I and MHC Class II complex and can be (re)introduced into a patient to thereby launch an immune-attack against the tumor. However, generation of such immuno-stimulatory DC usually requires differentiation of CD34$^+$ precursors or blood monocytes using complex and rather expensive cytokine cocktails. In those standard methods, the cytokines are employed at concentrations very much higher (often by orders of magnitude) than those encountered in vivo under physiological conditions. Therefore, one proffered reason for the overall disappointing clinical results from DC-based immunomodulation is that DC produced by the common cytokine method may not function effectively at the far lower cytokine concentrations actually in patients. DC produced ex vivo at markedly supra-physiologic concentrations of growth factors, such as cytokines, are selected to be dependent on conditions which are reproduced in the in vivo environment in patients.

Classical Extracorporeal Photopheresis (ECP) has been used successfully to treat cutaneous T-cell lymphoma (CTCL) in subsets of patients. In ECP, patients suffering from CTCL receive the photoactivatable compound 8-methoxypsoralen (8-MOP). Patients are then leukapheresed to obtain buffy coats and these buffy coats are passed through a continuous closed circuit ultraviolet exposure device to irradiate the leukapheresed buffy coats and thereby lethally damage exposed lymphocytes. In this manner, 8-MOP is induced to covalently crosslink base pairs of DNA. The concept of ECP is to destroy proliferating metastatic T-cells of CTCL and to then to intravenously re-introduce the dying cells to the patient. It has been learned that this process additionally leads to conversion of passaged blood monocytes to DC without the need for stimulation by addition of exogenous cytokines. These ECP-induced DC are furthermore assumed to internalize, process and display antigens from the tumor cells, which were destroyed by the combination of 8-MOP and UV irradiation. It has been hypothesized that reintroduction of these loaded dendritic cells to the patient account for at least part of the success of ECP when treating CTLC. In fact ECP-like processes, in which neither 8-MOP nor UV light irradiation are used, but in which extracorporeal blood sample comprising monocytes are passed under shear stress through an ECP device have also been assumed to initiate monocyte differentiation into DC.

However, it has also been found that the ECP or ECP-like process leads to truncated, i.e. immuno-suppressive or tolerogenic DC, likely contributing heavily to ECP's clinical efficacy in the treatment of Graft versus Host Disease which commonly follows post-bone marrow stem allotransplants. The precise mechanistic aspects of ECP on differentiation of monocytes into either immuno-stimulatory or immuno-suppressive DC have remained elusive (for review of the ECP process see Girardi et al. (2002), Transfusion and Apheresis Science, 26, 181-190).

WO 2011/137365 describes methods for treating blood monocytes to produce functional antigen presenting dendritic cells. An extracorporeal quantity of a subject's blood is treated with a photoactivatable agent and passed through a plastic treatment device which allows for transmittance of light to the interior of the plastic device. A light source then produces light of a wave length selected to activate the photoactivatable agent.

Berger et al. (2010, Blood, 116, 4838-4847) describes that ECP induces a high percentage of processed monocytes to enter the antigen-presenting dendritic cell differentiation pathway within a single day as determined by enhanced expression of different genes.

Hoffmann et al. (2007, Journal of Investigative Dermatology, S117) describes the induction of immunomodulatory dendritic cells by transimmunization.

Engberg et al. (2007, Journal of Investigative Dermatology, S117) relates to the generation of tumor-specific anti-melanoma T-cell responses by extracorporeal photochemotherapy-induced dendritic cells.

Khalil et al. (2012, Journal of investigative dermatology, S101) relates to the conversion of monocytes to dendritic cells by platelet signaling.

Durazzo et al. (2012, Journal of investigative dermatology, S121) describes that dendritic cell maturation is induced by platelets.

Salskov-Iversen et al. (2005, Journal of immune based therapies and vaccines, 3:4, 8518-3-4) relates to the production of dendritic cells from monocytes which are first differentiated to immature dendritic cells by adherence and release from a plastic surface and then further matured overnight in the presence of apoptotic malignant T cells.

The present ECP and ECP-like processes are thus conceived to lead to complex mixtures of immuno-stimulatory and immuno-suppressive DC. Of course, from *inter alia* a clinical perspective, it would be important to understand how the ECP and ECP-like processes can be modified to overcome these limitations and how one can obtain purposively and selectively preferentially immuno-stimulatory over immuno-suppressive DC and vice versa. Further, the classical ECP process is, in principle an in vivo method as the obtained dendritic cell mixtures are reinfused into the patient. It would, however, be desirable to have methods available that allow preferential production of immuno-stimulatory over immuno-suppressive DC and vice versa outside the human or animal body.

Thus, there is a continuing need for methods that allow predictable and reproducible production of individual-specific, i.e. autologous immuno-stimulatory dendritic cells, which can then be loaded with disease-specific antigens and which upon-reintroduction allow for treatment of e.g. hyper-proliferative diseases such as cancer.

## OBJECTIVES AND SUMMARY OF THE INVENTION

One objective of the present invention is to provide methods for producing immuno-stimulatory autologous dendritic cells.

Another objective of the present invention is to provide methods for producing immuno-stimulatory autologous antigen-presenting cells, preferably immuno-stimulatory autologous dendritic cells.

Another objective of the present invention is to provide methods for producing immuno-stimulatory autologous dendritic cells in an extracorporeal amount of blood obtained from a patient.

A further objective of the present invention is to provide methods for producing immuno-stimulatory autologous dendritic cells in an extracorporeal amount of blood obtained from a patient without the need for cytokine cocktails.

Yet another objective of the present invention is to provide methods for producing immuno-stimulatory autologous dendritic cells in an extracorporeal amount of blood obtained from an individual such as a patient over immuno-suppressive dendritic cells.

These and other objectives as they will become apparent from the ensuing description hereinafter are solved by the subject matter of the independent claims. Some of the preferred embodiments of the present invention form the subject matter of the dependent claims. Yet other embodiments of the present invention may be taken from the ensuing description.

The present invention is based to some extent on data presented hereinafter, which for a miniaturized and scalable device allowed (i) to mimic some aspects of the classical ECP procedure, (ii) to elucidate the cellular, molecular mechanism and biophysical conditions of induction of differentiation of monocytes into immuno-stimulatory autologous dendritic cells in an extracorporeal amount of blood. This data shows that the activation of platelets and binding of monocytes to such activated platelets under conditions of shear force is essential for obtaining immuno-stimulatory autologous dendritic cells. As is shown by the experiments described hereinafter, these immuno-stimulatory autologous dendritic cells can be characterized by expression of molecular markers indicative of immuno-stimulatory autologous dendritic cells. The data also shows that conditions that lead to an increased expression of Glucocorticoid-induced Leucine Zipper (GILZ) will favorably allow monocytes to differentiate into immuno-suppressive autologous dendritic cells. These findings thus allow for a rationalized approach to obtain immuno-stimulatory autologous dendritic cells by thus carefully selecting the properties of the devices to be used and the parameters of the process. The findings of the present invention allow to preferentially produce immuno-stimulatory dendritic cells over immuno-suppressive dendritic cells and thus overcome the limitations of the classical ECP procedure because, in the classical ECP, procedure the lack of understanding what type of dendritic cells are produced and how their production can be manipulated to some extent prevents the extension of using this method for other than the authorized applications (see Girardi et al. (2002), Transfusion and Apheresis Science, 26, 181-190). Moreover, other than for the classical ECP procedure as used in the device obtainable from Therakos, the present invention allows to obtain such immuno-stimulatory dendritic cells in an experimental setting, where the extracorporeal amount of blood is not in a continuous connection with the body. The data *inter alia* suggests that the process of obtaining immuno-stimulatory dendritic cells seems to include a global monocyte activation step and a subsequent monocyte to immuno-stimulatory antigen-presenting cell (e.g. dendritic cell) differentiation step. These steps seem to be initially dependent on physical activation of monocytes with the physical forces occurring during e.g. initial purification or enrichment of monocytes being sufficient for activation even though passage of e.g. initially activated monocytes through devices as described herein may allow improvement of activation and differentiation. Further, if activation and differentiation take place in the absence of photoactivatable agents and UV-A (as it is and was used in ECP processes), formation of immuno-suppressive dendritic cells seems to be favorably reduced as expression of GILZ is reduced. The present data further shed light on the nature of molecular markers that can be used to identifiy immuno-stimulatory dendritic cells.

Some of the embodiments, which are based on this data, are described in more detail hereinafter.

The present invention pertains to a method for inducing differentiation of monocytes contained in an extracorporeal quantity of a mammalian subject's blood sample into immuno-stimulatory dendritic cells, said method comprising at least the steps of:

a) subjecting said extracorporeal quantity of said mammalian subject's blood sample to a shear force by passing through a flow chamber;
b) wherein the method is performed in the absence of photoactivatable agents such as 8-MOP and UVA; and
c) wherein differentiation of monocytes from said blood sample into immunostimulatory dendritic cells is identified by increased expression of at least one molecular marker HLA-DR, CD83, CD86, ICAM-1 or PLAUR when comparing the immuno-stimulatory autologous dendritic cells to the monocytes within the extracorporeal quantity of a mammalian subject's blood sample.

Additional molecular markers are described hereinafter and may be taken from e.g. Table 1. These molecular markers may be grouped according to their know function as e.g. molecular markers of antigen-presenting cells, molecular markers of cellulara adhesion etc. Molecular markers the expression of which is considered indicative of immuno-stimulatory dendritic cells include PLAUR, NEU1, CD80, CCR7, LOX1, CD83, ADAM Decysin, FPRL2, GPNMB, ICAM-1, HLA-DR, and/or CD86. Markers like HLA-DR, PLAUR and ICAM-1 may be considered to be indicative of global monocyte activation while increased expression of e.g. CD83 and ADAM-Decysin seems indicative of monocyte to dendritic cell differentiation.

Activation of monocytes is *inter alia* achieved in that said extracorporeal quantity of said mammalian subject's blood sample is subjected to a physical force by passing or cycling said extracorporeal quantity of said mammalian subject's blood sample through a flow chamber of a device, which allows adjustment of the flow rate of said extracorporeal quantity of said mammalian subject's blood sample through said flow chamber of said device such that a shear force is applied to said monocytes contained within said mammalian subject's blood sample.

Thus, activation of monocytes and induction of differentiation into immuno-stimulatory autologous dendritic cells can be achieved and influenced by varying the flow forces of the extracorporeal quantity of the mammalian subject's blood sample through the flow chamber of such a device, by varying the geometry of the flow path of the flow chamber, by varying the dimensions of the flow chamber, by varying the temperature of the flow chamber and thus of the extracorporeal quantity of the mammalian subject's blood sample, by changing the biophysical and geometric surface properties of the flow path, etc.

As is shown hereinafter, activation of monocytes and induction of differentiation into immuno-stimulatory autologous dendritic cells may be optimized dependent on interaction of monocytes with activated platelets and/or specific plasma components in a situation where the monocytes experience physical force which may be provided by a device as described hereinafter.

The present disclosure thus describes activation of monocytes, which experience a physical force and which interact with activated platelets and/or plasma components such as fibrinogen or fibronectin. Activation may be a process of subsequent steps including the steps of (i) immobilizing plasma components such as fibrinogen or fibronectin either as isolated components or as part of the extracorporeal quantity of the mammalian subject's blood sample in the flow chamber of said device (ii) passing platelets, which may be obtained as a purified fraction from the extracorporeal quantity of the mammalian subject's blood sample or as part of the extracorporeal quantity of the mammalian subject's blood sample, through the flow chamber such that the platelets can interact with and become activated by the plasma components and (iii) passing monocytes, which may be obtained as a purified fraction from the extracorporeal quantity of the mammalian subject's blood sample or as part of the extracorporeal quantity of the mammalian subject's blood sample, through the flow chamber such that the monocytes can interact with and become activated by the activated platelets and/or the plasma components.

Thus, in addition and/or alternatively to the above described parameters and variable touching on the architecture of and the conditions under which the device is operated, activation of monocytes and induction of differentiation into immuno-stimulatory autologous dendritic cells can be achieved and influenced by varying the nature, purity and concentrations of the plasma components, the nature, purity and concentration of the platelets, the order of steps by which the plasma components and/or the platelets are passed through and/or disposed on the flow chamber, the density by which the flow chamber is coated with the plasma components and/or the platelets, the flow forces of the extracorporeal quantity of the mammalian subject's blood sample and in particular the platelets and/or the monocytes are passed through the flow chamber of such a device, the temperature and/or time at which the extracorporeal quantity of the mammalian subject's blood sample and in particular the platelets and/or the monocytes are passed through the flow chamber of such a device, etc..

It needs, however, to be understood that while such devices may be particularly effective in inducing monocyte activation and differentiation into dendritic cells, physical forces which monocytes experience during initial purification or enrichment such as during Ficoll-Hypaque enrichment as described hereinafter may already be sufficient to activate monocytes and to induce their differentiation into immuno-stimulatory antigen-presenting cells such as dendritic cells. Similarly even though activated platelets and/or specific plasma components may be helpful in increasing monocyte activation and differentiation into immuno-stimulatory dendritic cells they may not be absolutely necessary. In order to effect monocyte activation and differentiation into immuno-stimulatory dendritic cells the invention thus contemplates as a mimimal requirement the application of physical forces. In order to let this process proceed as uninfluenced as possible, the invention always considers to not apply molecular cocktails to achieve maturation and differentiation of monocytes into immuno-stimulatory autologous dendritic cells and to avoid conditions that lead to e.g. increased expression of GILZ such as co-application of photoactivatable agents and UV-A.

In the above and ensuing aspects and embodiment, the extracorporeal quantity of the mammalian subject's blood sample and in particular the monocytes thus may or may not be obtained by apheresis such as leukaphereses.

Additionally, the invention also relates to such methods which are conducted under conditions which avoid an increased expression of GILZ and/or an increased number of CD4+CD25+Foxp3+ cells and/or a down-regulations of CD80, CD86 and CD83.. The invention thus relates to e.g. methods, which are conducted in the absence of a photoactivatable agent such as 8-MOP and without exposure to light such as UV-A.

The present disclsoure also relates to methods as described hereinafter for obtaining autologous immuno-stimulatory antigen-presenting cells, which can be used e.g. in immunization against cancer antigens, viral antigens, bacterial antigens or fungal antigens.

Another embodiment relates to methods as described hereinafter for producing immune-stimulatory dendritic cells which may be mammalian cells. Even though the invention relates to producing human immuno-stimulatory allogeneic dendritic cells, the present invention considers also to use the methods for producing animal immuno-stimulatory dendritic cells such as for mice, rats, etc. These embodiments of the invention provide useful animal models and thus scalability of the methods and results described herein from e.g. mice to man. Moreover, as there are genetically identical lines of animals such as mice available, animal immuno-stimulatory dendritic cells such as mice immuno-stimulatory dendritic cells may be introduced either in the individual from which the extracorporeal amount of blood sample was taken and thus be autologous in the strict sense or be introduced in a genetically identical individual. This will allow e.g. testing for any unexpected effects of these cells.

It is to be understood that the methods described hereinafter have been shown to produce immune-stimulatory cells, which due to their molecular markers seem to be related to if not correspond to cells that are commonly named immune-stimulatory dendritic cells. Thus the immune-stimulatory cells according to the invention have been named immune-stimulatory dendritic cells.

The present disclosure also describes autologous immuno-stimulatory antigen-presenting cells obtainable by a method as described hereinafter for use in immunization against cancer antigens, viral antigens, bacterial antigens or fungal antigens.

Further embodiments will be described hereinafter.

**FIGURE LEGENDS**

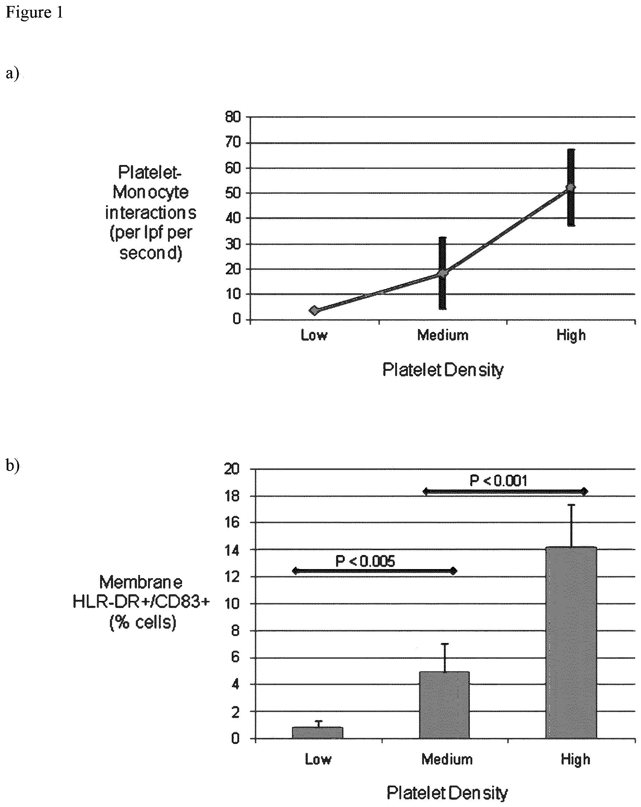

Figure 1    Effect of platelet density on number of monocyte-platelet interactions and subsequent monocyte phenotype. Monocytes were passed through parallel plates coated with platelets at low, medium, or high density. (A) The number of monocyte-platelet interactions increased substantially for plates coated with higher densities of platelets. (B) After overnight incubation, monocytes which were exposed to high levels of platelets were significantly more likely to develop a phenotype consistent with DC differentiation, as assessed by expression of membrane CD83 and HLA-DR (high versus medium or low density: p <0.0001; medium versus low density: p < 0.005). Data shown are the means (+/- SD) of at least 6 independent experiments. lpf, low power field.

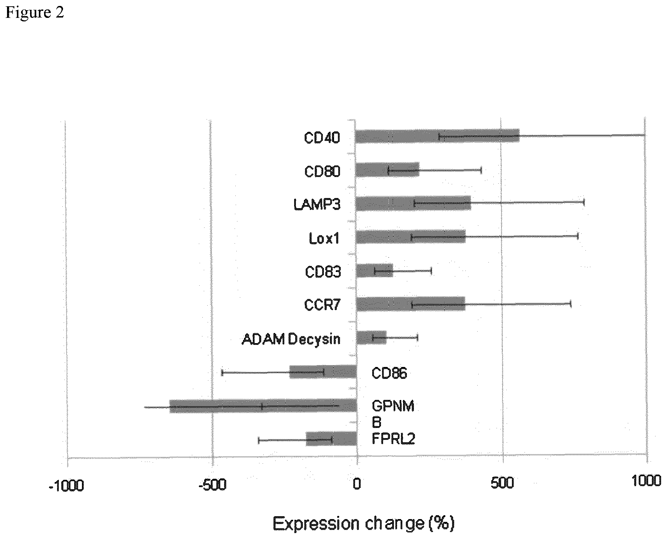

Figure 2    Gene expression following exposure to platelets. Monocytes were exposed to high or low levels of platelets in flow. Following overnight incubation, cells were assessed for differences in gene expression using RT-PCR. Figure 2 shows gene expression changes in monocytes exposed to high levels of platelets relative to those exposed to low levels. Seven genes associated with DC-differentiation and/or function were found to be upregulated, while three were downregulated. Of the genes downregulated, GPNMB and FPRL2 have known functions in decreasing cytokine production and inhibiting DC maturation, respectively. Of the genes upregulated, all have either pro-immune functions or miscellaneous roles in DC biology. See text for specific description of genes. Data shown are the means (+/- SD) of 2 independent experiments.

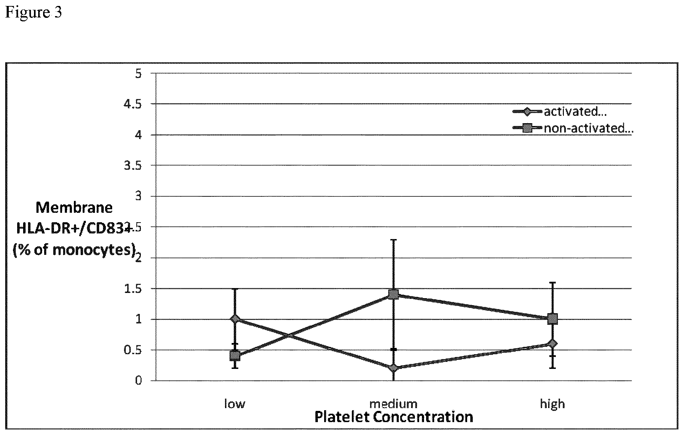

Figure 3    Platelet influence on monocyte differentiation in static conditions. Monocytes were co-cultured for 18 hours with low, medium, or high concentrations of platelets in static conditions lacking flow. Under these conditions, there was no observable platelet influence on DC differentiation; all conditions resulted in low, baseline levels of cells expressing DC markers. Furthermore, activating platelets with thrombin in culture (blue line) did not cause a discernible difference in monocyte differentiation relative to those cultures containing platelets not activated by thrombin (red line).

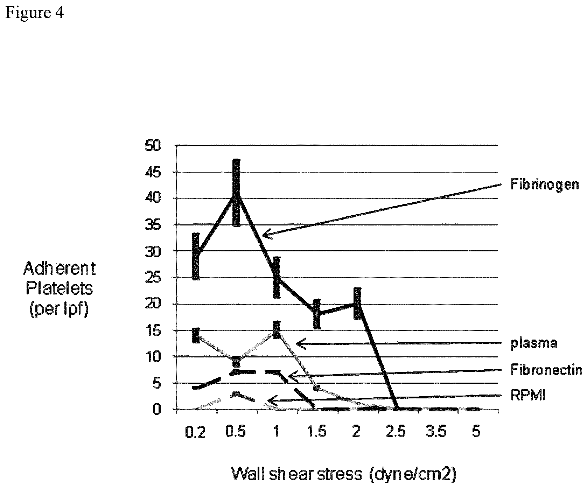

Figure 4    Plasma protein influence on platelet adhesion to plates. Platelets were passed through plates coated with fibrinogen, plasma, fibronectin, or RMPI at the shear stress level indicated by the x-axis. Platelets in flow adhered optimally to fibronectin. For all proteins, platelet adhesion occurred maximally between 0.5 and 1.0 dyne/cm$^2$ lpf, low power field. Data shown are the means (+/- SD) of at least 2 independent experiments.

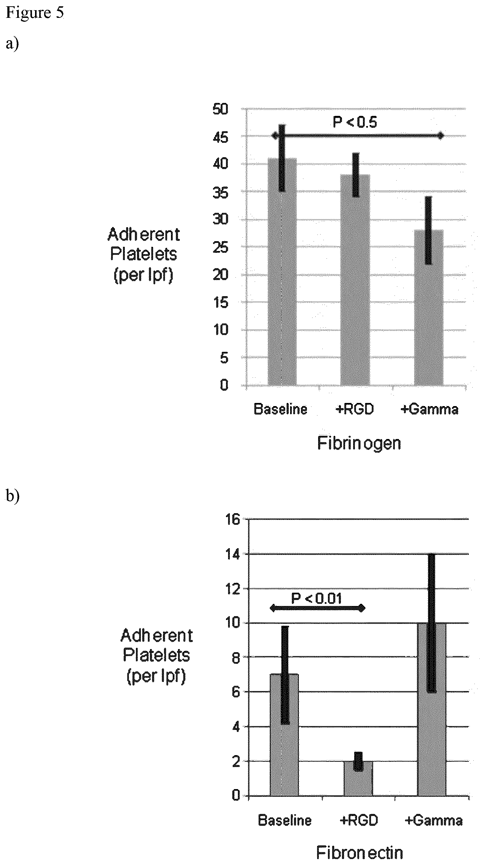

Figure 5    Plasma protein influence on platelet adhesion to plates coated with Fibrinogen (A) or Fibronectin (B). Platelets were either untreated (baseline), or pretreated with either RGD fragments (+RGD) or gamma fragments (+Gamma) and their subsequent adhesion to fibrinogen (left panel) and fibronectin (right panel) was assessed. Platelet binding to fibrinogen was decreased by gamma fragments (p < 0.05), while binding to fibronectin was decreased by RGD peptides (p < 0.001). lpf, low power field. Data shown are the means (+/- SD) of at least 2 independent experiments.

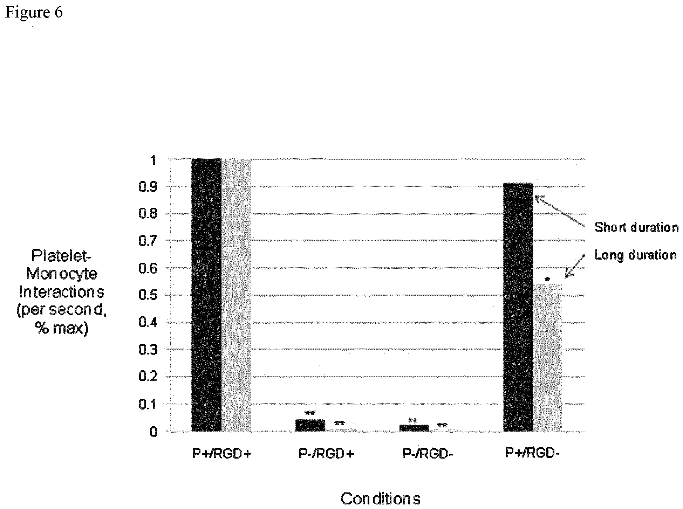

Figure 6    Proteins involved in monocyte-platelet interactions. Monocytes were passed between platelet-coated plates at a wall shear stress of 0.5 dyne/cm2 under the conditions indicated by the x-axis: platelets were either pretreated with anti-P-selectin (P-) or an isotype control (P+); monocytes were either pretreated with RGD peptides (RGD-) or a control fragment (RGD+). Monocyte-platelet interactions were quantified under each set of conditions using digital microscopy, and are expressed in the figure as a fraction of the maximum seen under conditions of P+/RGD+. Interactions were divided into those lasting less than 3 second (short duration, black bars) and those lasting greater than 3 seconds, including stable binding (long-duration, gray bars). All conditions which involved blocking with anti-P-selectin (P-) resulted in a significant decrease in both short and long duration interactions (**, p < 0.01); Blocking only RGD (RGD-) resulted in a significant decrease in long-duration interactions (*, p < 0.05) but no change in short-duration interactions. Data shown are the means (+/- SD) of 3 independent experiments.

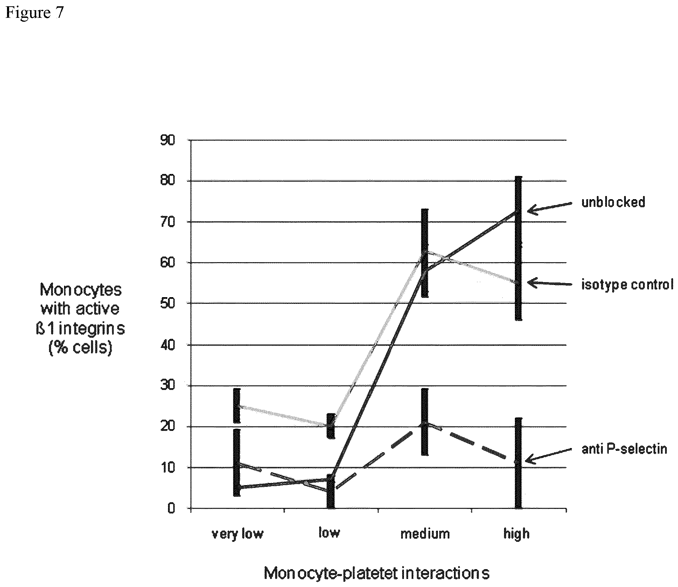

Figure 7    Effect of p-Selectin exposure on monocyte integrins. Plastic plates were coated with platelets at the relative density indicated by the x-axis. Platelets were then pretreated with anti p-selectin (dashed line) or an isotype control (gray line), or received no pretreatment (black line). Monocytes were passed through the plates at 0.5 dyne/cm2 and then immediately assessed by flow cytometry for expression of active β1 integrins. The y-axis indicates the percent of monocyte which bound an antibody directed at an epitope only exposed when the integrin is in the open confirmation. Data shown are the means (+/- SD) of 3 independent experiments.

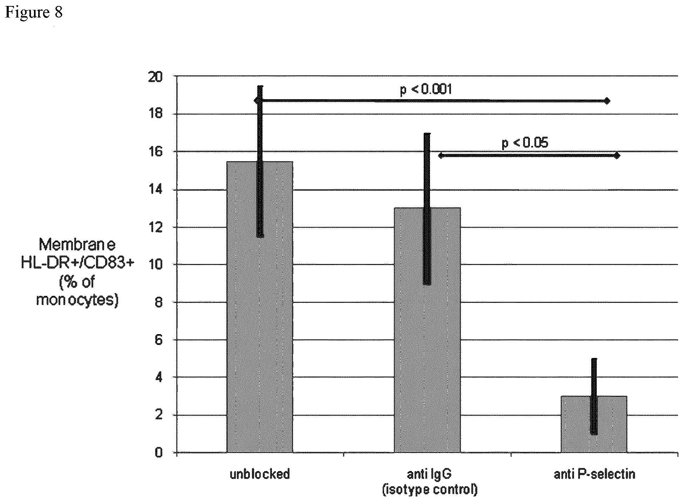

Figure 8    Effect of P-selectin exposure on monocyte phenotype after overnight incubation. Platelet-coated plates were either untreated (first column), or pretreated with an isotype control (second column) or anti-P-selectin (third column). Monocytes were passed through the plates at 0.5 dyne/cm2 then incubated overnight. The y-axis indicates the percent of monocytes which developed a phenotype consistent with DC differentiation, i.e., membrane HLA-DR+/CD83+. Data shown are the means (+/- SD) of 3 independent experiments.

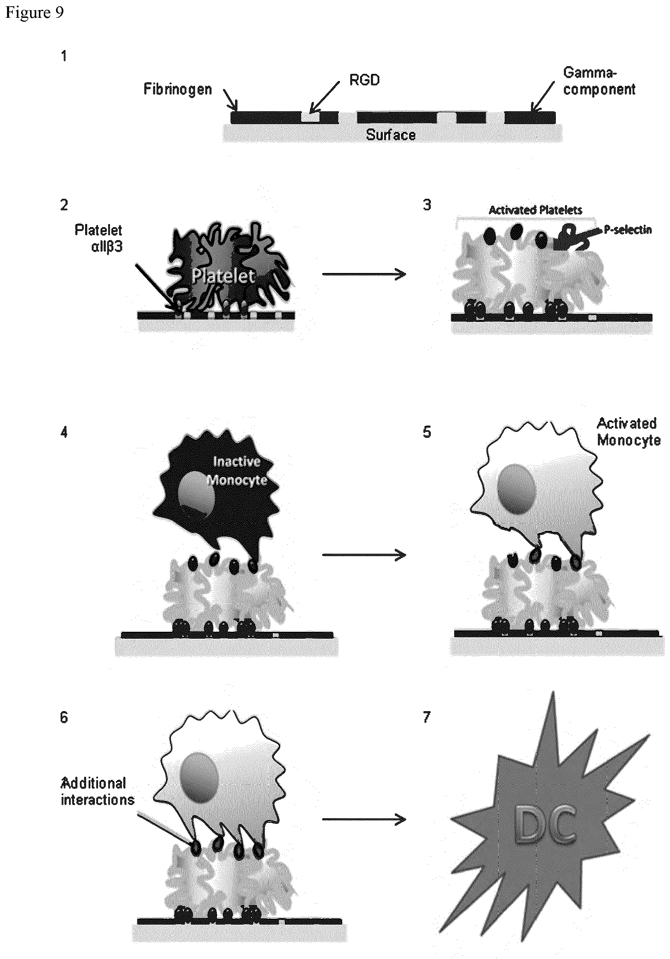

Figure 9    Proposed mechanism for induction of monocyte-to-DC differentiation. Based on data presented in this manuscript, the following sequence of events is postulated: (1) plasma fibrinogen coats the plastic surface of the flow chamber; (2) through their αIIβ3 receptor, unactivated platelets bind to the gamma-component of immobilized fibrinogen; (3) platelets become activated and instantaneously express preformed P-selectin and other surface proteins; (4) passaged monocytes transiently bind P-selectin via PSGL-1, causing partial monocyte activation and integrin receptor conformational changes; (5) partially-activated monocytes, now capable of further interactions, bind additional platelet-expressed ligands, including those containing RGD domains; (6) finally, so influenced, monocytes efficiently enter the DC maturational pathway within 18 hours. Note that, in-vivo, step (1) above may be replaced physiologically by inflammatory signals from tissue acting on local endothelium, causing it to recruit and activate platelets in a similar manner.

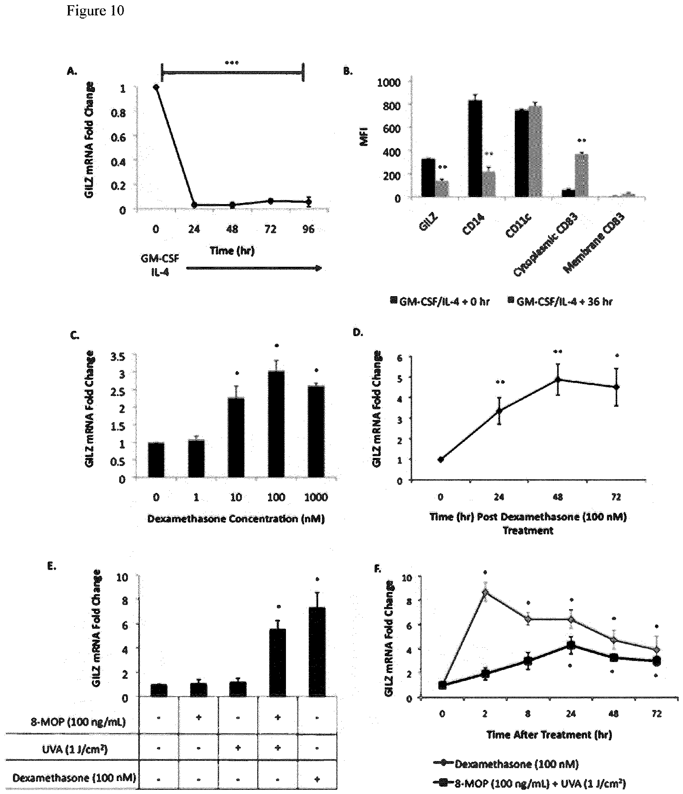

Figure 10:    Expression of GILZ is rapidly down-regulated as monocytes differentiate into immature MoDC, and up-regulated after exposure to dexamethasone. A.) GILZ mRNA expression in CD11c+ MoDC is presented as a fold change relative to freshly isolated monocytes. B.) Median fluorescence intensities for intracellular and cell surface markers after 0 and 36 hr. C.) GILZ mRNA expression in CD11c+ MoDC after 24 hr is presented as a fold change relative to MoDC receiving no dexamethasone. D.) GILZ mRNA expression in CD11c+ MoDC is presented as a fold change relative to MoDC at time 0 hr. E.) GILZ mRNA expression in CD11c+ MoDC after 24 hr is presented as a fold change relative to untreated MoDC. F.) GILZ mRNA expression in CD11c+ MoDC is presented as a fold change relative to untreated MoDC. All data are expressed as mean ± standard deviation for a minimum of 3 independent experiments. For differential gene expression: * ≥ 2.5-fold change and p < 0.05, ** ≥ 2.5-fold change and p < 0.01, *** ≥ 2.5-fold change and p < 0.001

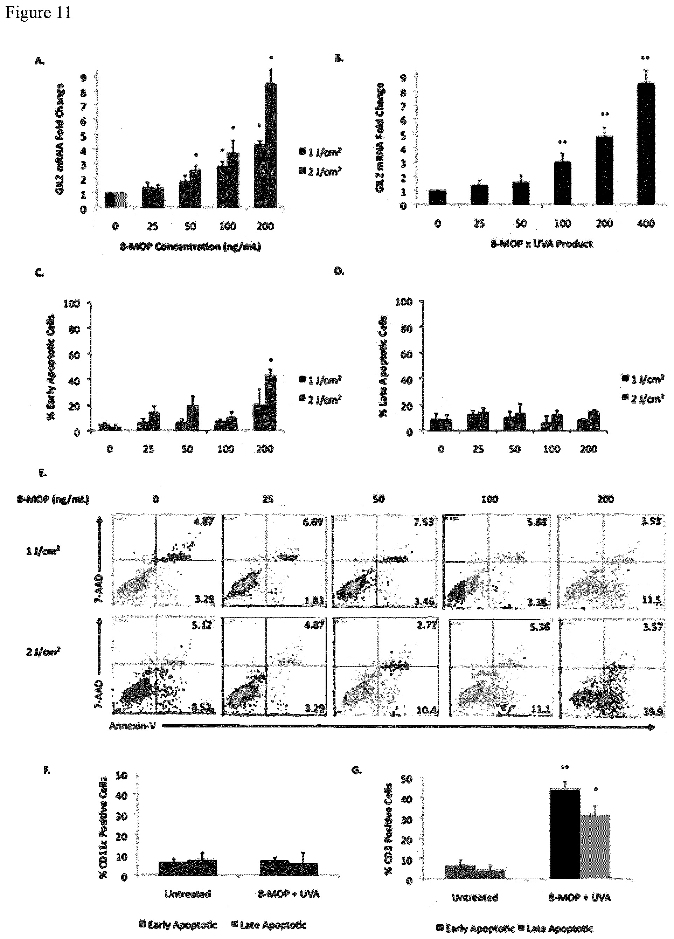

Figure 11:    8-MOP plus UVA light up-regulates GII,Z in immature MoDC in a dose-dependent fashion. A.) GILZ expression is presented as a function of the 8-MOP concentration at 1 J/cm2 and 2 J/cm2 of UVA light. GILZ mRNA expression in CD11c+ MoDC 24 hr after PUVA treatment is presented as a fold change relative to MoDC receiving no 8-MOP. B.) GILZ expression is presented as a function of the 8-MOP concentration multiplied by the UVA dose. C.) The percentage of early apoptotic CD11c+ cells after 24 hr. D.) The percentage of late apoptotic CD11c+ cells after 24 hr E.) Dot plots of CD11c+-gated cells for UVA doses of 1 J/cm2 and 2 J/cm2 are shown for 1 representative experiment of 4. The percentage of CD11c+ cells displaying Annexin-V+/7-AAD- or Annexin-V+/7-AAD+ phenotypes are indicated. The percentage of F.) CD11c+ cells and G.) CD3+ cells expressing early and late apoptotic markers were quantified 24 hr after treatment with 8-MOP (100 ng/mL) and UVA light (1 J/cm2). All data represent mean ± standard deviation of at least 4 independent experiments. For differential gene expression: * ≥ 2.5-fold change and p < 0.05, ** ≥ 2.5-fold change and p < 0.01

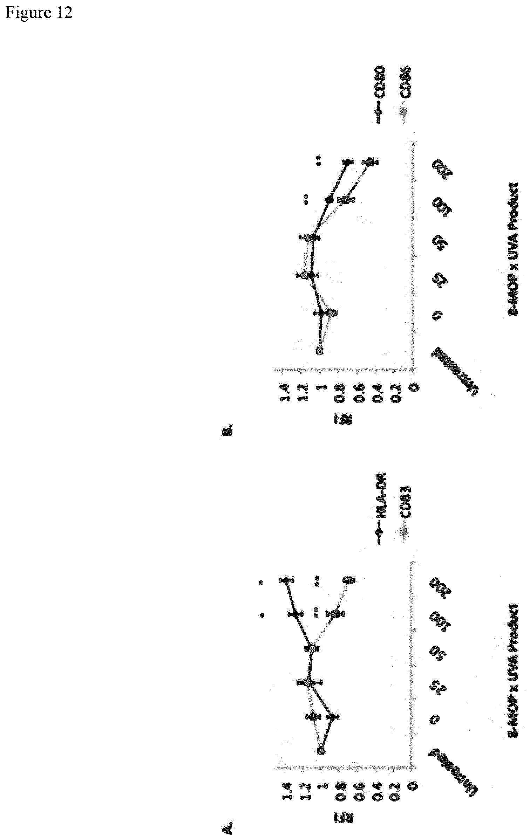

Figure 12:    8-MOP plus UVA light down-regulates CD83, CD80 and CD86 and up-regulates HLA-DR in immature MoDC in a dose-dependent manner. Relative fluorescence intensities for membrane expression of A.) HLA-DR and CD83, and B.) CD80 and CD86 are presented as a function of the 8-MOP concentration (0 to 200 ng/mL) multiplied by the UVA dose (1 or 2 J/cm2) 24 hr after PUVA treatment. Untreated MoDC served as controls and were assigned an RFI value of 1. Data represent mean ± standard deviation of 4 independent experiments. *p < 0.05, **p < 0.01

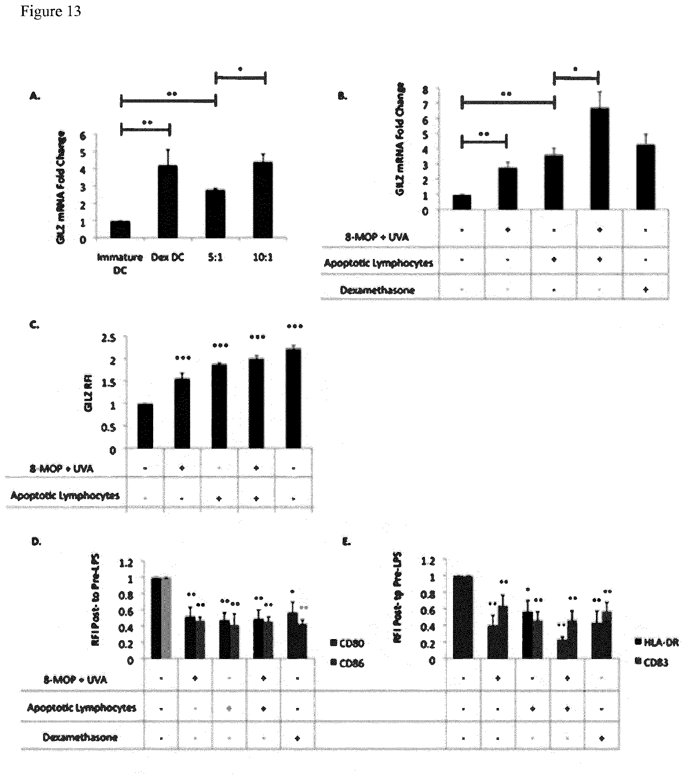

Figure 13:    Immature MoDC exposed to apoptotic lymphocytes up-regulate GILZ. A.) GII,Z mRNA expression in CD11c+ MoDC 24 hr after co-culture is presented as a fold change relative to untreated MoDC that were cultured alone. B.) GII,Z mRNA expression in CD11c+ MoDC 24 hr after co-culture is presented as a fold change relative to untreated MoDC that were cultured alone. C.) Relative fluorescence intensity for intracellular GILZ 24 hr after co-culture. Relative fluorescence intensities post- to pre-LPS stimulation for D.) CD80 and CD86 and E.) HLA-DR and CD83 were calculated as follows: (MFItreated after LPS - MFItreated before LPS) / (MFIuntreated after LPS - MFIuntreated before LPS). Data represent mean ± standard deviation for at least 4 independent experiments. For differential gene expression: * ≥ 2.5-fold change and p < 0.05

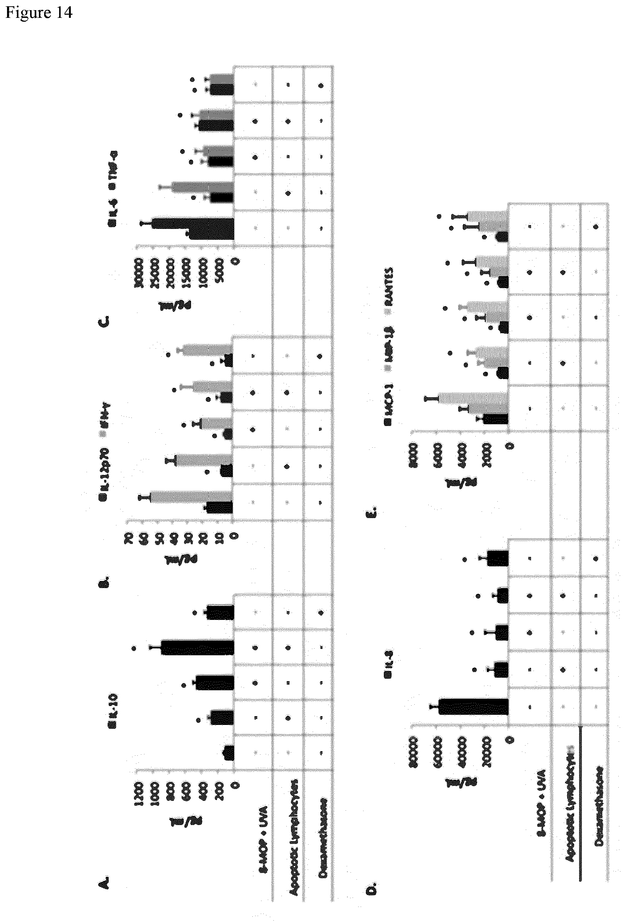

Figure 14: MoDC expressing GILZ increase production of IL-10, and decrease production of various pro-inflammatory cytokines and chemokines. 24 hr after LPS stimulation, culture supernatants were harvested for cytokine quantification by magnetic bead multiplex immunoassays for A.) IL-10, and the pro-inflammatory cytokines B.) IL-12p70 and IFN-$\gamma$, C.) IL-6 and TNF-$\alpha$. The same analysis was performed for the pro-inflammatory chemokines D.) IL-8, and E.) MCP-1, MIP-1$\beta$ and RANTES. Data are presented as mean ± standard deviation of 3 independent experiments. * p < 0.05 compared to the untreated MoDC group.

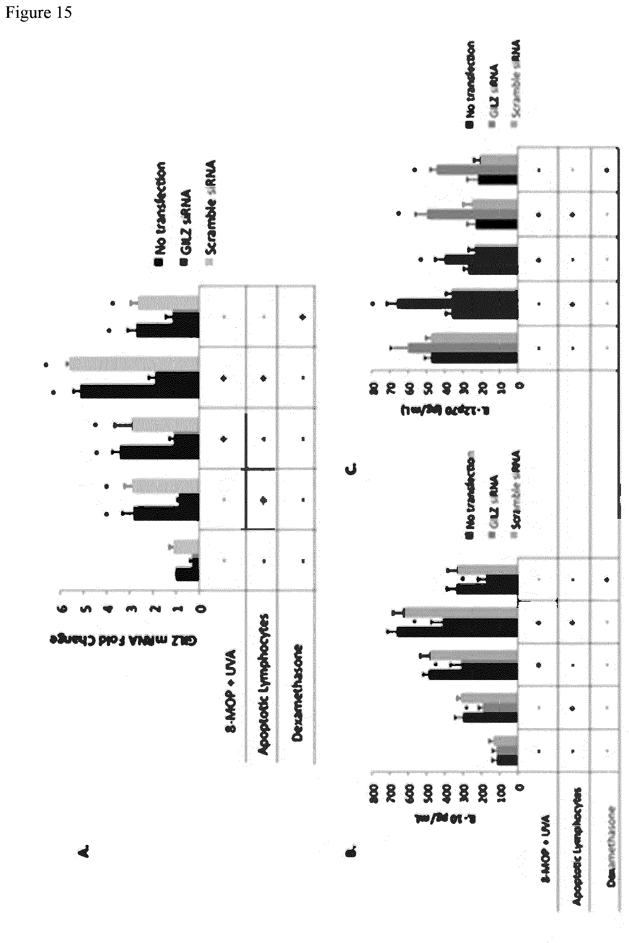

Figure 15: siRNA-mediated knockdown of GILZ abolishes the increased IL-10 to IL-12p70 ratio characteristic of tolerogenic DC. A.) GILZ mRNA expression is presented as fold change compared to untreated MoDC that were cultured alone. * ≥ 2.5-fold change and p < 0.05. B.) Quantification of IL-10 and IL-12p70 protein levels in culture supernatants after LPS stimulation. Data represent mean ± standard deviation of 3 independent experiments. *p < 0.05, compared to identically treated MoDC not transfected with siRNA.

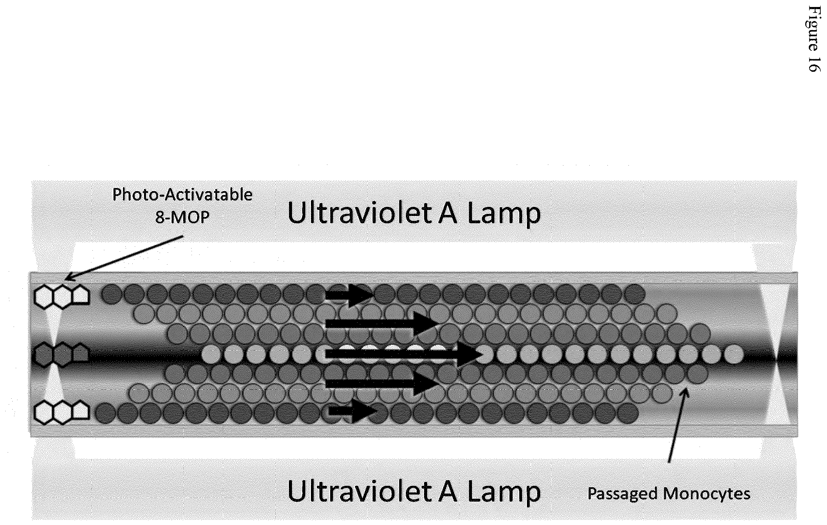

Figure 16: depicts the flow of monocytes in a classical ECP process in the presence of UVA and 8-MOP. The monocytes in the middle experience lower UVA exposure than the monocytes towards the surfaces of the channels.

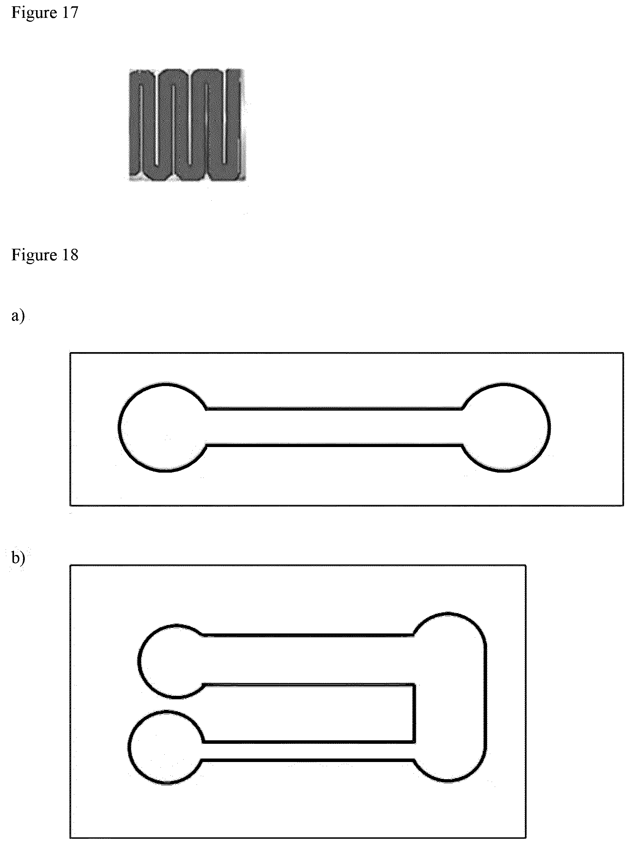

Figure 17: depicts the design of the channels of the device used in a classical ECP process.

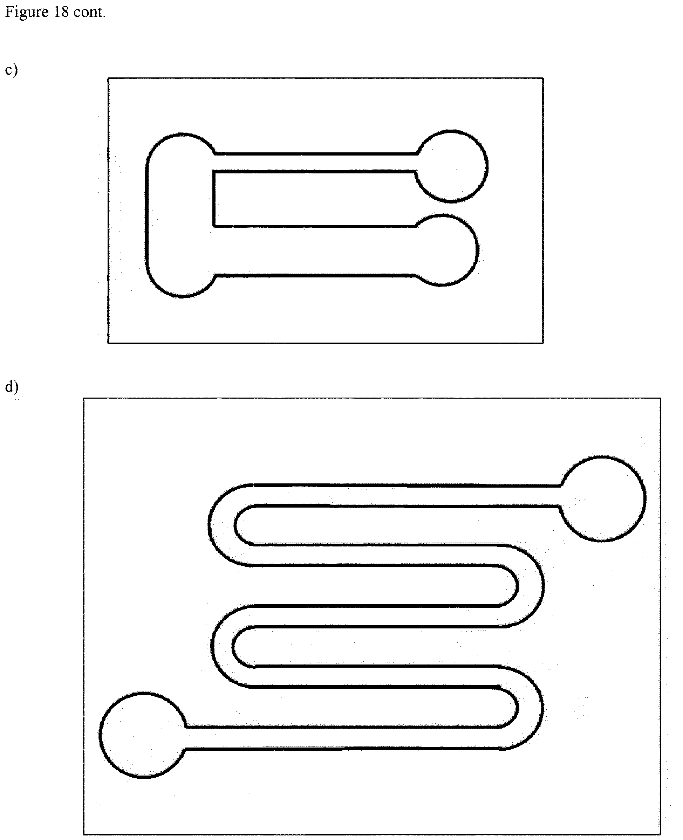

Figure 18: a) to d) depict different geometries of the flow chamber of a device that may be used for the methods of the invention.

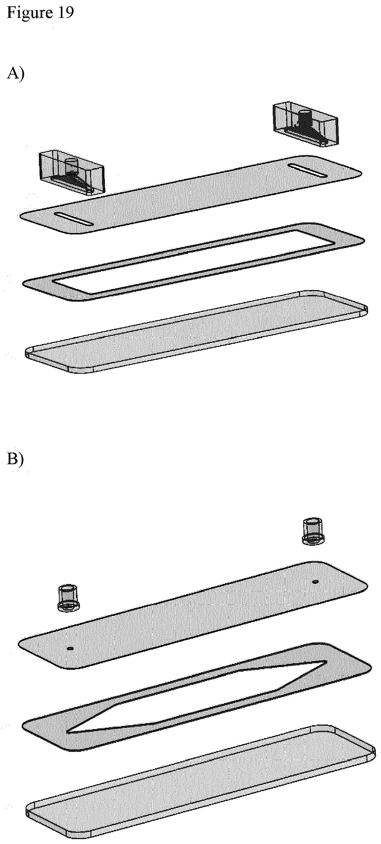

Figure 19: A) depicts the geometry of a device used in some of the examples. B) depicts the geometry of an alternative device.

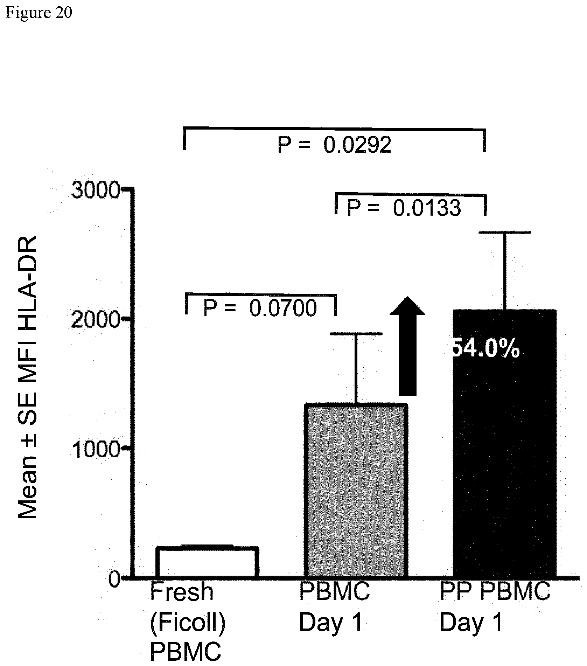

Figure 20: depicts increase of expression of HLA-DR upon physical activation of monocytes through a device of Figure 19

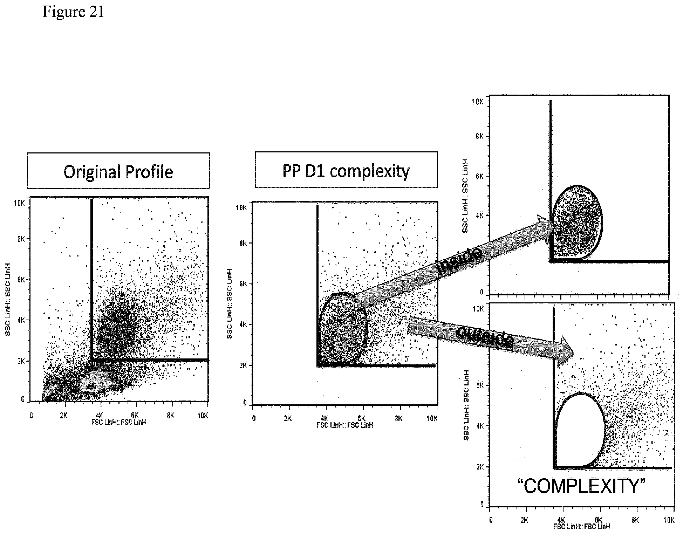

Figure 21: depicts increase of FSC/SSC complexity upon physical activation of monocytes through a device of Figure 19

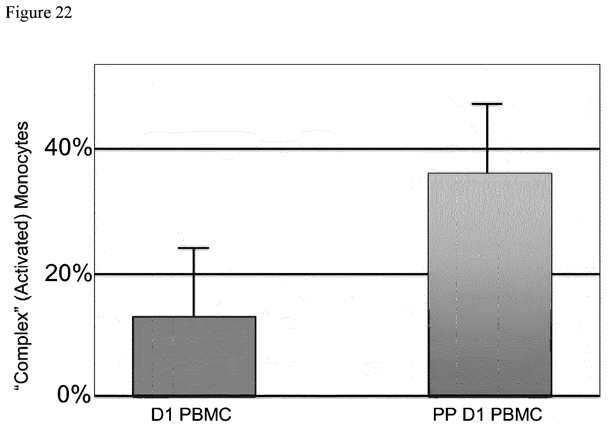

Figures 22: depicts increase of FSC/SSC complexity upon physical activation of monocytes by passing through a device of Figure 19

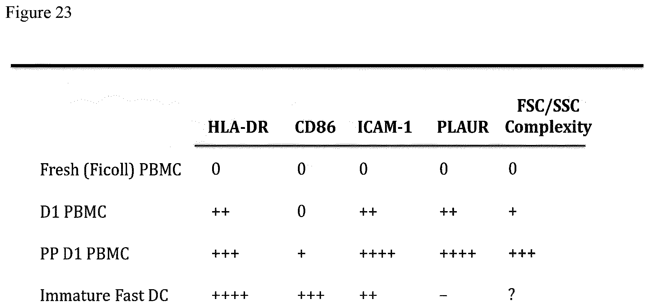

Figure 23: depicts increase of expression of HLA-DR, CD86, ICAM-1, PLAUR and or FSC/SSC complexity upon physical activation of monocytes through a device of Figure 19

## DETAILED DESCRIPTION OF THE INVENTION

Before the invention is described in detail with respect to some of its preferred embodiments, the following general definitions are provided.

The present invention as illustratively described in the following may suitably be practiced in the absence of any element or elements, limitation or limitations, not specifically disclosed herein.

The present invention will be described with respect to particular embodiments and with reference to certain figures but the invention is not limited thereto but only by the claims.

Where the term "comprising" is used in the present description and claims, it does not exclude other elements. For the purposes of the present invention, the term "consisting of" is considered to be a preferred embodiment of the term "comprising of". If hereinafter a group is defined to comprise at least a certain number of embodiments, this is also to be understood to disclose a group, which preferably consists only of these embodiments.

For the purposes of the present invention, the term "obtained" is considered to be a preferred embodiment of the term "obtainable". If hereinafter e.g. an antibody is defined to be obtainable from a specific source, this is also to be understood to disclose an antibody, which is obtained from this source.

Where an indefinite or definite article is used when referring to a singular noun, e.g. "a", "an" or "the", this includes a plural of that noun unless something else is specifically stated. The terms "about" or "approximately" in the context of the present invention denote an interval of accuracy that the person skilled in the art will understand to still ensure the technical effect of the feature in question. The term typically indicates deviation from the indicated numerical value of $\pm 20$ %, preferably $\pm 15$ %, more preferably $\pm 10$ %, and even more preferably $\pm 5$ %.

Furthermore, the terms "first", "second", "third" or "(a)", "(b)", "(c)", "(d)" or "(i)", "(ii)", "(iii)", "(iv)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In case the terms "first", "second", "third" or "(a)", "(b)", "(c)", "(d)" or "(i)", "(ii)", "(iii)", "(iv)" etc. relate to steps of a method or use or assay there is no time or time interval coherence between the steps unless indicated otherwise, i.e. the steps may be carried out simultaneously or there may be time intervals of seconds, minutes, hours, days, weeks, months or even years between such steps, unless otherwise indicated in the application as set forth herein above or below.

Technical terms are used by their common sense. If a specific meaning is conveyed to certain terms, definitions of terms will be given in the following in the context of which the terms are used.

As already mentioned, the present invention is based to some extent on data presented hereinafter, which for a miniaturized device allowed (i) to mimic some aspects of the classical ECP procedure and (ii) to elucidate the cellular and molecular mechanism of induction of differentiation of monocytes into immuno-stimulatory dendritic cells in an extracorporeal amount of blood. As is shown by the experiments described hereinafter, these immuno-stimulatory autologous dendritic cells can be characterized by expression of molecular markers indicative of immuno-stimulatory autologous dendritic cells. The data also shows that conditions that lead to an increased expression of Glucocorticoid-induced Leucine Zipper (GILZ) will favorably allow monocytes to differentiate into immuno-suppressive autologous dendritic cells. For the purposes of the present invention such immuno-suppressive autologous dendritic cells are also designated as immuno-inhibiting autologous dendritic cells, tolerogenic autologous dendritic cells or truncated autologous dendritic cells. This data shows that the sequential activation of platelets and binding of monocytes to such activated platelets under conditions of shear force is essential for obtaining immuno-stimulatory dendritic cells. Further, these findings immediately allow for a rationalized approach to obtain immuno-stimulatory dendritic cells. Given that one can mimic and dissect the series of molecular events leading to the formation of immuno-stimulatory autologous dendritic cells and immuno-suppressive autologous dendritic cells obtained in the classical ECP process, one can now design devices and more particularly flow chambers, which allow to further dissect the molecular events leading to differentiation of monocytes into immuno-stimulatory autologous dendritic cells on a scale suitable for research purposes, but also which allow to obtain such immuno-stimulatory autologous dendritic cells for therapeutic purposes. This will be explained in further detail.

In the classical ECP procedure, 2.5 L to 6 L blood is typically obtained from patients suffering from CTLC by apheresis such as leukaphereses. This extracorporeal amount of blood, which typically is processed by apheresis such as leukaphereses to give a final volume of about 200 ml to 500 ml comprising leukocytes including monocytes as well as plasma components, platelets and cancerous T-cells, is then passed under shear stress through a Photopheresis device having transparent plastic channels together with the photoactivatable drug 8-MOP. This extracorporeal amount of blood comprising 8-MOP is then irradiated by exposing the transparent channels to UV-A having a wavelength of 315 to 380 nm. The irradiated extracorporeal amount of blood is then re-introduced into the patient. The beneficial effects of this procedure on the course of CTLC in some of the treated patients was originally hypothesized to result from the destruction of cancerous T-cells. Based on this hypothesis, it was assumed that patients would have to undergo repeated cycles of ECP. However, for some of the patients beneficial long-term effects were observed making repeated treatment superfluous and, in the following, interesting and partially non-reconcilable effects were found, which could explain some of the positive outcomes of ECP for CTLC treatment.

For example, as is described in US 6,524,855 induction of DC was observed in the extracorporeal amount of blood and it was hypothesized that some of the beneficial effects of ECP on CTLC resulted from cancer-specific antigens that were shed by cancerous T-cells as a consequence of the 8-MOP induced apoptosis of these cells and loading of DC, which had started to differentiate, with these antigens. The reintroduction of the extracorporeal amount of blood comprising such cancer-antigen loaded autologous DC was assumed to provide a vaccination-like long-term lasting therapeutic effect. However, at the same it was observed that so-called "truncated" DC were formed during the ECP procedure, which did not provide an immuno-stimulatory effect, but rather the opposite, namely an immuno-suppressant effect. The induction of such different types of DC with opposing effects by the same process was puzzling and, from a practical perspective, posed hurdles as to a rationalized use of ECP for obtaining immuno-stimulatory or immuno-suppressant DC. Further, the need for apheresis such as leukaphereses to obtain a sufficient amount of extracorporeal blood is another factor negatively affecting treatment quality for patients.

The data presented hereinafter suggest that shear stress is in principle responsible for global monocyte activation and the induction of DC. By using e.g. the miniaturized model device as described hereinafter, it was shown that induction of immuno-stimulatory DC occurs even if substantially lower amounts of extracorporeal blood, which has not been obtained by apheresis such as leukaphereses, are used, even if 8-MOP is not added to the extracorporeal amount of blood and even if no irradiation with UV-A takes place. Thus, induction of DC occurred despite omission of central steps of the classical ECP procedure. However, shear stress seems to be one factor that is crucial for obtaining immuno-stimulatory DC. Other steps with a positive influence for the induction of DC formation seem to be the activation of platelets by plasma components and the activation of monocytes by such activated platelets. The data further suggests that, if shear-stress induced induction of DC formation takes place in the presence of 8-MOP and irradiation with UVA, expression of the Glucocorticoid-induced Leucine Zipper (GILZ) is increased, which in turn activates a pathway leading to formation of truncated, i.e. immuno-suppressant tolerogenic DC (see Example 2). The fact that shear-stress induced induction of immuno-stimulatory DC could be achieved by applying shear stress without the addition of 8-MOP and without irradiation with UV-A further suggests that in the classical ECP procedure due to the dimensions of the plastic channels some of the initially shear-stress induced DC were not effectively irradiated with the consequence that these DC could further develop into immuno-stimulatory DC (see Figure 16). This previous data was obtained using a device having the general architecture of Figure 17. However, in the classical ECP and ECP-like procedures, mixtures of immuno-stimulatory autologous and immuno-suppressive autologous dendritic cells were obtained. Based on the data presented hereinafter, it is now possible to e.g. dispense with some of the requirements of the ECP and ECP-like processes of the prior art, e.g. to use large amounts of blood which needs to be processed by apheresis such as leukaphereses. Further, one can now deliberately adapt the process parameters and the design of the device, which is used to exert a physical force on monocytes, to deliberately obtain either immuno-stimulatory autologous or immuno-suppressive autologous dendritic cells.

The method as described hereinafter is performed without the need of molecular cocktails to achieve maturation and differentiation of monocytes into immuno-stimulatory autologous dendritic cells. Further, as the invention is based on inducing differentiation of monocytes contained in an extracorporeal quantity of mammalian subject's blood sample, the differentiation process is not limited to the molecular events which can be triggered by typical cytokine cocktails. Rather, dendritic cells as obtainable with the methods described hereinafter seem to have more complex molecular, albeit synchronized patterns, which seem representative of a broader functionality of these dendritic cells.

The present invention relates to a method for inducing differentiation of monocytes contained in an extracorporeal quantity of a mammalian subject's blood sample into immuno-stimulatory dendritic cells, said method comprising at least the steps of:

> a) subjecting said extracorporeal quantity of said mammalian subject's blood sample to a shear force by passing through a flow chamber;
> b) wherein the method is performed in the absence of photoactivatable agents such as 8-MOP and UVA; and
> c) wherein differentiation of monocytes from said blood sample into immuno-stimulatory dendritic cells is identified by increased expression of at least one molecular marker HLA-DR, CD83, CD86, ICAM-1 or PLAUR when comparing the immuno-stimulatory autologous dendritic cells to the monocytes within the extracorporeal quantity of a mammalian subject's blood sample. a)

It is to be understood that the invention as well as all the embodiments described hereinafter can preferably be used to provide immuno-stimulatory autologous dendritic cells, namely if the obtained immuno-stimulatory dendritic cells are later re-introduced into the same donor. This can be done in a continous or dis-continous fashion where the dendritic cells are cultivated for extended periods of time before they are re-introduced into the donor. As this will be the preferred application, all of the embodiments discussed hereinafter refer to immuno-stimulatory autologous dendritic cells. It is, however, to be understood that the discussion of such embodiments always includes the scenario where the invention is used to make immuno-stimulatory dendritic cells as such and where only the later administration of these cells will make them potentially immuno-stimulatory autologous dendritic cells.

As has already been mentioned the methods described hereinafter have been shown to produce immune-stimulatory and immune-suppressive cells, which due to their molecular markers seem to be related to if not correspond to cells that are commonly named dendritic cells. Thus the immune-stimulatory cells according to the invention have been named immune-stimulatory dendritic cells.

The term "immuno-stimulatory autologous dendritic cells" thus refers to cells derivable from monocytes by treating the monocytes contained in an extracorporeal quantity of said mammalian subject's blood sample as it is described herein and identified by at least one of the molecular markers HLA-DR, CD83, CD86, ICAM-1 or PLAUR as described in the following. These molecular markers have been discussed in the literature for dendritic cells which can present antigens by way of MHC I and MHC II. It is to be understood that the immuno-stimulatory autologous dendritic cells as obtainable by the methods described herein and identified by the molecular markers described herein may be considered as dendritic cells which have already differentiated enough and internalized and even display e.g. tumor-specific antigens from apoptotic cells such as cytotoxic T-cells, which are contained in the extracorporeal quantity of a respective mammalian subject's blood sample, or e.g. viral or bacterial antigens, which are contained in the extracorporeal quantity of a respective mammalian subject's blood sample, such that they can be considered o be immuno-stimulatory autologous antigen-presenting dendritic cells. However, the process can also be conducted in a way such that the dendritic cells express molecular markers indicative of immuno-stimulatory dendritic cells, which have not yet internalized and display antigens. It needs to be understood that where dendritic cells are mentioned herein, this refers to immuno-stimulatory antigen-presenting cells which have the capacity of displaying e.g. disease-specific antigens in their surfaces after these cells have been contacted with such antigens.

The present invention allows preferential production of immuno-stimulatory DC relative to immuno-suppressive DC. The preferential production of immuno-stimulatory dendritic cells over immuno-suppressive dendritic cells means that starting from an extracorporeal amount of blood sample, more immuno-stimulatory dendritic cells than immuno-suppressive DC can be selectively obtained compared to a situation where e.g. the same extracorporeal amount of blood sample was subjected to a classical ECP procedure. Even though production of immuno-stimulatory DC will be produced preferentially over produce immuno-suppressive DC, immuno-suppressive DC may be still present after the methods in accordance with the invention have been performed. Nevertheless, the present invention provides the parameters and variables that can be manipulated to skew production of one dendritic cell population over the other. Production of immuno-stimulatory dendritic cells is achieved by not using 8-MOP and UVA and by culturing the obtained immuno-stimulatory dendritic cells for extended periods of time such as 1, 2, 3, or 4 days. In this way, formation of immuno-suppressive dendritic cells may be reduced to a clinically acceptable level. Further, the eventually remaining immuno-suppressive dendritic cells may be removed by e.g. affinity purification against molecular markers that are indicative of immuno-suppressive dendritic cells.

As is described in the examples, molecular markers which are indicative of immuno-stimulatory autologous dendritic cells obtainable by the methods described herein were identified by subjecting monocytes contained in the extracorporeal quantity of mammalian subjects' blood samples derived either from healthy volunteers to the process using a miniaturized device (see markers 88 to 99 of Table 1). Further, as is also described in the example, molecular markers, which are indicative of immuno-stimulatory autologous dendritic cells, were identified by subjecting monocytes contained in the extracorporeal quantity of mammalian subjects' blood samples derived either from healthy volunteers or from patients suffering from CTCL or from GvH disease (GvHD) to an ECP process (see markers 1 to 87 of Table 1). The dendritic cells were then isolated and up-regulated expression of molecular markers, which are known or suspected to play a role in immuno-stimulatory dendritic cells, was analyzed. Some of the markers identified for the ECP process, which is assumed to lead to a complex mixture of immune-stimulatory and immune-suppressive dendritic cells, are the same as they were observed for the dendritic cells obtained by the process with the miniaturized device, which should lead to immune-stimulatory dendritic cells only. Thus to the extents that the ECP process leads to up-regulation of molecular markers, which can be associated with dendritic cell function, it seems justified to assume that these markers will also be suitable to identify immune-stimulatory dendritic cells as they are obtainable by the processes described herein such as with the miniaturized device. A set of overall 99 molecular markers was identified as being upregulated for immuno-stimulatory autologous dendritic cells obtainable by methods described herein. This set may be extended by further molecular markers in the future through comparable analysis

Thus, the data of examples 1 and 3 lead to a set of 99 markers, which are considered indicative of immuno-stimulatory autologous dendritic cells. These markers are summarized in Table 1.

Table 1

| No. | Marker | NCBI Gene ID No. | mRNA REF | SEQ ID No. |
|-----|--------|------------------|----------|------------|
| 1 | ABCA1 | 19 | NM_005502.3 | 1 |
| 2 | ACVR1B | 91 | NM_004302.4 | 2 |
| 3 | ANPEP | 290 | NM_001150.2 | 3 |
| 4 | AQP9 | 366 | NM_020980.3 | 4 |
| 5 | ATP6V0B | 533 | NM_001039457.1 | 5 |
| 6 | BASP1 | 10409 | NM_001271606.1 | 6 |
| 7 | BEST1 | 7439 | NM_001139443.1 | 7 |
| 8 | CD63 | 967 | NM_001257389.1 | 8 |

(continued)

| No. | Marker | NCBI Gene ID No. | mRNA REF | SEQ ID No. |
|-----|--------|------------------|----------|------------|
| 9 | CD68 | 968 | NM_001040059.1 | 9 |
| 10 | CDCP1 | 64866 | NM_022842.3 | 10 |
| 11 | CPM | 1368 | NM_001005502.2 | 11 |
| 12 | CRK | 1398 | NM_005206.4 | 12 |
| 13 | CSF2RA | 1438 | NM_001161529.1 | 13 |
| 14 | CTNND1 | 1500 | NM_001085458.1 | 14 |
| 15 | CTSB | 1508 | NM_001908.3 | 15 |
| 16 | CXCL16 | 58191 | NM_001100812.1 | 16 |
| 17 | EMP1 | 2012 | NM_001423.2 | 17 |
| 18 | ENG | 2022 | NM_000118.2 | 18 |
| 19 | EPB41L3 | 23136 | NM_012307.2 | 19 |
| 20 | FLOT1 | 10211 | NM_005803.2 | 20 |
| 21 | GNA15 | 2769 | NM_002068.2 | 21 |
| 22 | GPNMB | 93695 | NM_053110.4 | 22 |
| 23 | GPR137B | 83924 | NM_031999.2 | 23 |
| 24 | GPR157 | 269604 | NM_177366.3 | 24 |
| 25 | HEXB | 3074 | NM_000521.3 | 25 |
| 26 | HOMER3 | 9454 | NM_001145721.1 | 26 |
| 27 | ICAM1 | 3383 | NM_000201.2 | 27 |
| 28 | IL1R1 | 3554 | NM_000877.2 | 28 |
| 29 | IRAK1 | 3654 | NM_001025242.1 | 29 |
| 30 | ITGA5 | 3678 | NM_002205.2 | 30 |
| 31 | ITGB8 | 3696 | NM_002214.2 | 31 |
| 32 | KCTD11 | 147040 | NM_001002914.2 | 32 |
| 33 | LAMP2 | 3920 | NM_001122606.1 | 33 |
| 34 | LEPROT | 54741 | NM_001198681.1 | 34 |
| 35 | LGALS3 | 3958 | NM_001177388.1 | 35 |
| 36 | LILRB4 | 11006 | NM_001081438.1 | 36 |
| 37 | MARCKSL1 | 65108 | NM_023009.6 | 37 |
| 38 | MCOLN1 | 57192 | NM_020533.2 | 38 |
| 39 | MFAP3 | 4238 | NM_001135037.1 | 39 |
| 40 | MGAT4B | 11282 | NM_014275.4 | 40 |
| 41 | MR1 | 3140 | NM_001194999.1 | 41 |
| 42 | MRAS | 22808 | NM_001085049.2 | 42 |
| 43 | MSR1 | 4481 | NM_002445.3 | 43 |
| 44 | NEU1 | 4758 | NM_000434.3 | 44 |
| 45 | NPC1 | 4864 | NM_000271.4 | 45 |
| 46 | OLR1 (LOX1) | 4973 | NM_001172632.1 | 46 |

(continued)

| No. | Marker | NCBI Gene ID No. | mRNA REF | SEQ ID No. |
|---|---|---|---|---|
| 47 | OMG | 4974 | NM_002544.4 | 47 |
| 48 | P2RX4 | 5025 | NM_001256796.1 | 48 |
| 49 | PI4K2A | 55361 | NM_018425.2 | 49 |
| 50 | PLAUR | 5329 | NM_001005376.2 | 50 |
| 51 | PMP22 | 5376 | NM_000304.2 | 51 |
| 52 | PPAP2B | 8613 | NM_003713.4 | 52 |
| 53 | PSEN1 | 5663 | NM_000021.3 | 53 |
| 54 | PVRL2 | 5819 | NM_001042724.1 | 54 |
| 55 | RAB13 | 5872 | NM_002870.2 | 55 |
| 56 | RAB8B | 51762 | NM_016530.2 | 56 |
| 57 | RAB9A | 9367 | NM_001195328.1 | 57 |
| 58 | RALA | 5898 | NM_005402.3 | 58 |
| 59 | RHEB | 6009 | NM_005614.3 | 59 |
| 60 | RNASE1 | 6035 | NM_002933.4 | 60 |
| 61 | SC5DL | 6309 | NM_001024956.2 | 61 |
| 62 | SDC2 | 6383 | NM_002998.3 | 62 |
| 63 | SEMA6B | 10501 | NM_032108.3 | 63 |
| 64 | SIRPA | 140885 | NM_001040022.1 | 64 |
| 65 | SLC17A5 | 26503 | NM_012434.4 | 65 |
| 66 | SLC1A4 | 6509 | NM_001193493.1 | 66 |
| 67 | SLC22A4 | 6583 | NM_003059.2 | 67 |
| 68 | SLC31A1 | 1317 | NM_001859.3 | 68 |
| 69 | SLC35E3 | 55508 | NM_018656.2 | 69 |
| 70 | SLC39A6 | 25800 | NM_001099406.1 | 70 |
| 71 | SLC6A6 | 6533 | NM_001134367.1 | 71 |
| 72 | SLC6A8 | 6535 | NM_001142805.1 | 72 |
| 73 | SLC7A11 | 23657 | NM_014331.3 | 73 |
| 74 | STX3 | 6809 | NM_001178040.1 | 74 |
| 75 | STX6 | 10228 | NM_005819.4 | 75 |
| 76 | TM9SF1 | 10548 | NM_001014842.1 | 76 |
| 77 | TMBIM1 | 64114 | NM_022152.4 | 77 |
| 78 | TMEM33 | 55161 | NM_018126.2 | 78 |
| 79 | TNFRSF10B | 8795 | NM_003842.4 | 79 |
| 80 | TNFRSF11A | 8792 | NM_001270949.1 | 80 |
| 81 | TNFRSF1A | 7132 | NM_001065.3 | 81 |
| 82 | TNFRSF1B | 7133 | NM_001066.2 | 82 |
| 83 | TNFSF14 | 8740 | NM_003807.3 | 83 |
| 84 | TNFSF9 | 8744 | NM_003811.3 | 84 |

(continued)

| No. | Marker | NCBI Gene ID No. | mRNA REF | SEQ ID No. |
|---|---|---|---|---|
| 85 | TRIP10 | 9322 | NM_004240.2 | 85 |
| 86 | TRIP6 | 7205 | NM_003302.2 | 86 |
| 87 | YKT6 | 10652 | NM_006555.3 | 87 |
| 88 | DC-LAMP (LAMP3) | 27074 | NM_014398.3 | 88 |
| 89 | CLEC5A | 23601 | NM_013252.2 | 89 |
| 90 | SPC2 (PCSK2) | 5126 | NM_002594.3 | 90 |
| 91 | THBS1 | 7057 | NM_003246.2 | 91 |
| 92 | CD14 | 929 | NM_000591.3 | 92 |
| 93 | CD40 | 958 | NM_001250.4 | 93 |
| 94 | CD80 | 941 | NM_005191.3 | 94 |
| 95 | CCR7 | 1236 | NM_001838.3 | 95 |
| 96 | CD83 | 9308 | NM_001251901.1 | 96 |
| 97 | ADAM Decysin | 27299 | NM_014479.3 | 97 |
| 98 | FPRL2 (FPR3) | 2359 | NM_002030.3 | 98 |
| 99 | CD86 | 942 | NM_006889.4 | 99 |

Of the 87 genes (markers 1 to 87 of Table 1) that represent surface markers/functional mediators of immunostimulatory DC function, 66 were found to be uniquely identified in the ECP-induced process (plate passaged, overnight cultured, see example) dendritic cells, after comparison to expression databases for "classical" dendritic cells. These are: ABCA1 , ACVR1B, ATP6V0B, BASP1, BEST1, CPM, CRK, CSF2RA, CTNND1, CTSB, CXCL16, ENG, FLOT1, GNA15, GPR137B, GPR157, HEXB, HOMER3, ICAM1, IRAK1, ITGA5, ITGB8, KCTD11, LAMP2, LEPROT, MARCKSL1, MCOLN1, MFAP3, MGAT4B, MR1, MRAS, MSR1, NEU1, OLR1, OMG, PI4K2A, PLAUR, PMP22, PVRL2, RAB13, RAB8B, RAB9A, RALA, RNASE1, SC5DL, SEMA6B, SIRPA, SLC1A4, SLC22A4, SLC31A1, SLC35E3, SLC39A6, SLC6A6, SLC6A8, STX3, STX6, TM9SF1, TMBIM1, TMEM33, TNFRSF10B, TNFRSF11A, TNFRSF1A, TNFRSF1B, TNFSF14, TNFSF9, YKT6.

Immuno-stimulatory autologous dendritic cells are thus identified by determining expression of at least one molecular marker of HLA-DR, CD83, CD86, ICAM-1 or PLAUR for the immuno-stimulatory autologous dendritic cells obtainable by the methods described herein and by comparing its expression for monocytes contained within the extracorporeal quantity of a mammalian subject's blood sample. If an increased expression for immuno-stimulatory autologous dendritic cells vs. monocytes is observed, this is indicative of the differentiation of monocytes to immuno-stimulatory autologous dendritic cells.

According to the present disclosure, immuno-stimulatory autologous dendritic cells are identifiable by determining expression for at least 2, 3, 4, 5, 6, 7, 8, 9, 10, 15, 20, 25, 30, 35, 40, 45, 50, or more additional molecular markers selectable from Table 1. For example, one may identify immuno-stimulatory autologous dendritic cells by determining expression for at least 1, 2, 3, 4, 5, 6, 7, 8, 9, 10, 11, 12, 13, 14, 15, 16, 17, 18, 19, 20, 21 or 22 additional molecular markers selectable from the group comprising PLAUR, NEU1, CTSB, CXCL16, ICAM1, MSR1, OLR1, SIRPA, TNFRSF1A, TNFSF14, TNFSF9, PMB22, CD40, LAMP3, CD80, CCR7, LOX1, CD83, ADAM Decysin, FPRL2, GPNMB and/or CD86. More preferably, one may identify immuno-stimulatory autologous dendritic cells according to the present disclosure by determining expression for at least 1, 2, 3, 4, 5, 6, 7, 8, 9, or 10 molecular markers selectable from the group comprising PLAUR, NEU1, CD80, CCR7, LOX1, CD83, ADAM Decysin, FPRL2, GPNMB and/or CD86. The most preferred markers, which are considered indicative of immuno-stimulatory autologous dendritic cells according to the present disclosure are PLAUR, NEU1, CD80, CD83, and/or CD86.

The data and conclusions presented herein suggest that the process of obtaining immuno-stimulatory dendritic cells seems to include a global monocyte activation step and a monocyte to immuno-stimulatory antigen-presenting cell (e.g. dendritic cell) differentiation step. These different steps seem to be traceable by molecular markers as described above and by Forward Scattering/Side Scattering Complexity (FSC/SSC Complexity) which is determinable by FACS analysis. The molecular markers may moreover be be grouped according to their know function as e.g. molecular markers of antigen-presentation, molecular markers of cellular adhesion etc.. HLA-DR, CD86, and CD 80 may be considered to representative of antigen-presentation. PLAUR, and ICAM-1 may be considered to representative of cell adhesion. Markers like HLA-DR, PLAUR and ICAM-1 as well as FSC/SSC complexity may be moreover considered to be indicative of global monocyte activation while increased expression of e.g. CD83, ADAM-Decysin, CD40, CD80, LAMP-3, and CCR7 seems indicative of monocyte to dendritic cell differentiation.

As is described herein, if the methods are conducted to allow an increased expression of GILZ (SEQ ID No.: 100), IDO (Indoleamine) (SEQ ID No.: 101), KMO (kynurenine 3-hydroxylase) (SEQ ID No.: 102), transforming growth factor-beta (TGFβ) (SEQ ID No.: 103), and/or IL-10 (Interleukin 10) (SEQ ID No.: 104), monocytes contained within the extracorporeal quantity of a mammalian subject's blood sample will not differentiate into immuno-stimulatory autologous dendritic cells, but rather into immature, so-called truncated or immuno-suppressive dendritic cells. According to the present disclosure, immuno-stimulatory autologous dendritic cells are identifiable not only by determining expression of the afore-mentioned molecular markers, but also by determining that expression of GILZ, IDO, KMO, TGFβ, and/or IL-10 is not increased for immuno-stimulatory autologous dendritic cells vs. monocytes. If increased GILZ, IDO, KMO, TGFβ and/or IL-10 expression was determined, this would be considered indicative of at least some for immuno-suppressive dendritic cells having formed. The preferred molecular marker, which is considered indicative for immune-suppressive dendritic cells, is currently GILZ.

As mentioned above, the method as described hereinafter is performed without the need of molecular cocktails to achieve maturation and differentiation of monocytes into immuno-stimulatory autologous dendritic cells. Such cocktails may comprise factors such as e.g. IL-4, GM-CSF, LPS, IFN-γ, IL-1β and TNF-α.

According to the present disclosure, the immuno-stimulatory autologous dendritic cells as they are obtainable by the methods described herein can thus not only be positively identified by molecular markers, which are indicative of immune-stimulatory dendritic cells such as PLAUR, CD80 and CD83, but also by the absence of up-regulation of molecular markers, which are indicative of immune-suppressive dendritic cells such as GILZ. Further both of these cell types, i.e. immune-stimulatory and immune-suppressive dendritic cells can be distinguished from the monocytes, which are subjected to a shear force to induce the differentiation process thereof, by determining the expression of molecular markers which are considered indicative of monocytes such as CD33 , CD36, and/or FCGRIa (Receptor for IgGFc fragment 1A). If it is found that the expression of these factors is down-regulated compared to expression of the monocytes, before they have been subjected to a shear force and process as described herein, then this is considered indicative that the monocytes have entered the maturation and differentiation pathway towards immune-stimulatory and/or immuno-suppressive dendritic cells. According to the present disclosure, the distinction between these later two dendritic cell population can then be made by determining expression of molecular markers such as PLAUR, ICAM-1, CD80, CD83 and GILZ.

Given that one now has the understanding and correspondingly the tools, e.g. the molecular markers at hand to distinguish between immuno-stimulatory autologous dendritic cells and the immuno-suppressive autologous dendritic cells, one can now deliberately vary both the design of the device and the flow chamber through which the extracorporeal quantity of a mammalian subject's blood sample and thus the monocytes are passed to experience a shear force, and the parameters at which the process of inducing differentiation of monocytes into immuno-stimulatory autologous dendritic cells is performed, to purposively enable differentiation of monocytes into immuno-stimulatory autologous dendritic cells.

As mentioned above, an extracorporeal quantity of a mammalian subject's blood sample is passed through a flow chamber of a device, such that a shear force is applied to said monocytes contained within said mammalian subject's blood sample. Alterations of the design of the device and the flow chamber which have an influence on the differentiation of monocytes into immuno-stimulatory autologous dendritic cells include variation of flow forces, variation of the geometry of the flow path of the flow chamber, variation of the dimensions of the flow chamber, the possibility to adjust temperature, the possibility of exposure of the extracorporeal quantity of the mammalian subject's blood sample in the flow chamber to visible light, etc.. Application of a shear force may not only be achieved by e.g. passing an extracorporeal amount of blood sample through a flow chamber, but also by placing such an extracorporeal amount of blood sample in e.g. an EVA plastic bag as obtainable from Macopharma and gently moving or shaking this blood sample-filled bag (see e.g. Andreu et al., (1994), Trans. Sci., 15(4), 443-454)

As also mentioned above and shown hereinafter, activation of monocytes and induction of differentiation into immuno-stimulatory autologous dendritic cells is dependent on interaction of monocytes with activated platelets and/or specific plasma components in a situation where the monocytes experience shear force, which may be provided by a device as described hereinafter. Variation of process parameters thus include varying the nature, purity and concentrations of plasma components; the nature, purity and concentration of platelets; the order of steps by which plasma components and/or platelets are passed through and/or disposed on the flow chamber; the density by which the flow chamber is coated with plasma components and/or platelets, the flow forces of the extracorporeal quantity of the mammalian subject's blood sample and in particular the platelets and/or the monocytes are passed through the flow chamber of such a flow chamber, the temperature and/or time at which the extracorporeal quantity of the mammalian subject's blood sample and in particular the platelets and/or the monocytes are passed through the flow chamber of such a device, etc.

Factors relating to the design of the device and the flow chamber as well as to process parameter will now be discussed in more detail as regards their relevance for the differentiation of monocytes into immuno-stimulatory autologous dendritic cells. It is to be understood that for any of the embodiments discussed in the following differentiation of monocytes into immuno-stimulatory autologous dendritic cells is achieved wherein immuno-stimulatory autologous dendritic cells are identified by determining expression of molecular markers described above . Further, for all embodiments discussed in the following it is to be understood that monocytes that are contained in an extracorporeal quantity of a mammalian subject's blood sample are subjected to a shear force in order to allow them to differentiate into immuno-stimulatory autologous dendritic cells, e.g. upon interaction with activated platelets and/or plasma components.

The invention relates to a method of inducing differentiation of monocytes contained in an extracorporeal quantity of a mammalian subject's blood sample into immuno-stimulatory autologous dendritic cells, wherein according to claim 1 said extracorporeal quantity of said mammalian subject's blood sample is subjected to a physical force by passing said extracorporeal quantity of said mammalian subject's blood sample through a flow chamber of a device, which allows adjustment of the flow rate of said extracorporeal quantity of said mammalian subject's blood sample through said flow chamber of said device such that a shear force is applied to said monocytes contained within said mammalian subject's blood sample.

In an embodiment, the invention of claim 1 relates to a method of inducing differentiation of monocytes contained in an extracorporeal quantity of a mammalian subject's blood sample into immuno-stimulatory autologous dendritic cells, wherein said extracorporeal quantity of said mammalian subject's blood sample is subjected to a physical force by passing said extracorporeal quantity of said mammalian subject's blood sample through a flow chamber of a device, which allows adjustment of the flow rate of said extracorporeal quantity of said mammalian subject's blood sample through said flow chamber of said device such that a shear force is applied to said monocytes contained within said mammalian subject's blood sample, and wherein said flow chamber of said device has a design allowing to apply a shear force to said monocytes contained within said mammalian subject's blood sample.

In another embodiment, the invention of claim 1 relates to a method of inducing differentiation of monocytes contained in an extracorporeal quantity of a mammalian subject's blood sample into immuno-stimulatory autologous dendritic cells, wherein said extracorporeal quantity of said mammalian subject's blood sample is subjected to a physical force by passing said extracorporeal quantity of said mammalian subject's blood sample through a flow chamber of a device, which allows adjustment of the flow rate of said extracorporeal quantity of said mammalian subject's blood sample through said flow chamber of said device such that a shear force is applied to said monocytes contained within said mammalian subject's blood sample, and wherein said device additionally allows for adjustment of at least one parameter selected from the group comprising temperature, and light exposure.

In another embodiment, the invention of claim 1 relates to a method of inducing differentiation of monocytes contained in an extracorporeal quantity of a mammalian subject's blood sample into immuno-stimulatory autologous dendritic cells, wherein said extracorporeal quantity of said mammalian subject's blood sample is subjected to a shear force by passing said extracorporeal quantity of said mammalian subject's blood sample through a flow chamber of a device as mentioned before and wherein said monocytes are activated and induced to differentiate into immuno-stimulatory autologous dendritic cells through interaction with activated platelets and/or plasma components.

For example, in one embodiment, the invention of claim 1 relates to a method of inducing differentiation of monocytes contained in an extracorporeal quantity of a mammalian subject's blood sample into immuno-stimulatory autologous dendritic cells, wherein said method comprises at least the steps of:

   a) applying said extracorporeal quantity of said mammalian subject's blood sample comprising at least monocytes to a device, which is configured to provide for a flow chamber through which said extracorporeal quantity of said mammalian subject's blood sample can be passed,
   b) activating platelets, which may be comprised within said extracorporeal quantity of said mammalian subject's blood or which may be provided separate from said mammalian subject's blood sample comprising at least monocytes,
   c) treating said extracorporeal quantity of said mammalian subject's blood sample comprising at least monocytes in said device by applying a physical force to the monocytes contained within said extracorporeal quantity of said mammalian subject's blood sample such that said monocytes are activated and induced to differentiate into immuno-stimulatory autologous dendritic cells by binding to said activated platelets obtained in step b).

In another embodiment, the invention of claim 1 relates to a method of inducing differentiation of monocytes contained in an extracorporeal quantity of a mammalian subject's blood sample into immuno-stimulatory autologous dendritic cells, wherein said method comprises at least the steps of:

   a) applying said extracorporeal quantity of said mammalian subject's blood sample comprising at least monocytes to a device, which is configured to provide for a flow chamber through which said extracorporeal quantity of said mammalian subject's blood sample can be passed,

b) passing plasma components, which may be comprised within said extracorporeal quantity of said mammalian subject's blood sample or which may be provided separate from said mammalian subject's blood sample,

c) treating said extracorporeal quantity of said mammalian subject's blood sample comprising at least monocytes in said device by applying a physical force to the monocytes contained within said extracorporeal quantity of said mammalian subject's blood sample such that said monocytes are activated and induced to differentiate into immuno-stimulatory autologous dendritic cells by binding to said plasma components obtained in step b).

In yet another embodiment, the invention of claim 1 relates to a method of inducing differentiation of monocytes contained in an extracorporeal quantity of a mammalian subject's blood sample into immuno-stimulatory autologous dendritic cells, wherein said method comprises at least the steps of:

a) applying said extracorporeal quantity of said mammalian subject's blood sample comprising at least monocytes to a device, which is configured to provide for a flow chamber through which said extracorporeal quantity of said mammalian subject's blood sample can be passed,

b) passing plasma components, which may be comprised within said extracorporeal quantity of said mammalian subject's blood or which may be provided separate from said mammalian subject's blood sample,

c) activating platelets, which may be comprised within said extracorporeal quantity of said mammalian subject's blood sample or which may be provided separate from said mammalian subject's blood sample comprising at least monocytes,

d) treating said extracorporeal quantity of said mammalian subject's blood comprising at least monocytes in said device by applying a physical force to the monocytes contained within said extracorporeal quantity of said mammalian subject's blood sample such that said monocytes are activated and induced to differentiate into immuno-stimulatory autologous dendritic cells by binding to said activated platelets and/or plasma components obtained in steps b) and c).

In yet another embodiment, the invention of claim 1 relates to a method of inducing differentiation of monocytes contained in an extracorporeal quantity of a mammalian subject's blood sample into immuno-stimulatory autologous dendritic cells, wherein said method comprises at least the steps of:

a) optionally passing platelets-rich plasma through a a device, which is configured to provide for a flow chamber through which said extracorporeal quantity of said mammalian subject's blood sample can be passed,

b) applying said extracorporeal quantity of said mammalian subject's blood sample comprising at least monocytes to a device, which is configured to provide for a flow chamber through which said extracorporeal quantity of said mammalian subject's blood sample can be passed,

c) treating said extracorporeal quantity of said mammalian subject's blood comprising at least monocytes in said device by applying a physical force to the monocytes contained within said extracorporeal quantity of said mammalian subject's blood sample such that said monocytes are activated and induced to differentiate into immuno-stimulatory autologous dendritic cells optionally by binding to said platelets-rich plasma of steps a).

As can be taken from the experiment described herein, the method for inducing differentiation of monocytes contained in an extracorporeal quantity of a mammalian subject's blood into immuno-stimulatory autologous dendritic cells works optimal, if platelets which are comprised within said extracorporeal quantity of said mammalian subject's blood are activated and if the extracorporeal quantity of said mammalian subject's blood comprising at least monocytes in said device is treated by applying a shear force to the monocytes contained within said extracorporeal quantity of said mammalian subject's blood such that said monocytes are activated and induced to differentiate into immuno-stimulatory autologous dendritic cells by binding to said activated platelets. However, activation of monocytes may also be achieved by direct interaction with plasma components, i.e. without interaction with activated platelets.

The steps of activating platelets and the subsequent activation and differentiation of monocytes into DC will be discussed in the following for the embodiment that (i) plasma components such as plasma proteins are passed through the flow chamber of the device so that these components adhere to the walls of the flow chamber, that (ii) platelets are passed through the flow chamber and are activated by binding to the plasma components and that (iii) monocytes-containing fractions such as an extracorporeal quantity of said mammalian subject's blood comprising at least monocytes are passed through the flow chamber and are activated for differentiation into DC by binding to the activated platelets. It is, however, to be understood that these activities also occur if the plasma fraction or plasma proteins or fragments thereof, the platelet fraction and the monocytes-containing fraction are passed simultaneously through the channels or channel-like structures as is the case for a whole blood fraction if obtained from the extracorporeal amount of blood as described below. It is further to be understood that the process may be performed even though not with same effectiveness by adhering only plasma components to the walls of the flow chamber and letting monocytes interact with the plasma components. Nevertheless, in the following these aspect will be discussed for a preferred embodiment, i.e. where steps (i), (ii), and (iii) are realized.

As regards the first step, plasma components including proteins like fibrinogen or fibronectin, or fragments thereof like the gamma component of fibrinogen may be provided either as fractions obtained from the extracorporeal amount of blood sample or in purified form from other resources e.g. in the form of recombinantly expressed proteins. Even though it seems that activation of platelets by plasma proteins such as fibrinogen and fibronectin is sufficient so that recombinantly expressed forms of these proteins are sufficient, it can be preferred to use plasma fractions which are obtained from the extracorporeal amount of blood sample and comprise these proteins as these plasma fractions have a more complex composition and may comprise all plasma components, which provide for an optimal activation of platelets.

Plasma protein fractions, plasma proteins or fragments thereof may be passed through the flow chamber, which may be made of plastic or non-plastic materials such as glass in order to adhere to the walls of the channels or channel-like structures. There is no requirement that the plasma fractions or plasma proteins are passed through the flow chamber at a specific shear force such as e.g. a specific pressure. However, in order to streamline the process, it is envisaged to pass the plasma fractions or plasma proteins through the flow chamber at a shear stress, which is comparable if not identical to the shear stress required for monocyte activation being described in more detail below. In general, the plasma fractions or plasma proteins are first pumped through the flow chamber to coat the surfaces thereof with plasma proteins, including fibronectin and fibrinogen. The flow rate of the plasma protein fractions, plasma proteins or fragments thereof through the flow chamber is controlled to obtain a desired level of protein adherence to the plastic surfaces. If desired, the flow can be stopped for a period of time and the plasma component can "soak" the surfaces of the flow chamber. By controlling the speed and timing of the pump that propels the plasma components through the flow chamber, the degree of coating of can be controlled. In one approach, the plasma fractions or plasma proteins are exposed to the surfaces of the flow chamber structures for a period between about 1 to 60 min, between about 1 to about 30 min, between about 1 to about 20 min, or between about 1 to about 10 min. To enhance plasma protein adherence to the surfaces of the flow chamber, the flow may be temporarily discontinued (for up to about 60 min), before resumption, or the flow rate may be slowed from the filling rate (up to 100 ml/minute) to as low as 5 ml/minute, during this phase of the procedure.

One can also envisage a scenario, where a device with a flow chamber is used for which the surfaces of the flow chamber have been pre-coated with e.g. purified plasma proteins or fragments thereof such as the gamma component of fibrinogen. Such pre-coated devices may be used if the whole process s conducted in a handheld device comprising a cartridge providing the flow chamber, which is configured for e.g. one time use. One can also envisage a scenario, where a device with a flow chamber is used for which the surfaces of the flow chamber have been pre-coated with e.g. platelets-rich plasma.

After the plasma fractions or plasma proteins or fragments thereof have been passed through the channels or channel-like structures and the surfaces thereof have been coated with plasma proteins, the platelet fraction is passed by e.g. pumping into and through the channels or channel-like structures. The flow rate and residence time of the platelets within the channels or channel-like structures is selected to allow the platelets to bind to the plasma components or proteins or fragments thereof which have adhered before to the surfaces of the channels or channel-like structures and to thereby activated.

The data presented herein suggest that activation of platelets by plasma components is a sequential process in which inactivated platelets first bind to the gamma component of fibronectin, get activated thereby and can then bind to the RGD motif (Arginine, Glycine, Aspartic Acid) which is found in many plasma proteins such as fibronectin or fibrinogen. If purified and/or recombinantly expressed plasma proteins or fragments thereof are used for activation of platelets, it can therefore be envisaged to pre-coat channels or channel-like structures with at least the gamma-component of fibrinogen and optionally additionally with RGD peptides. These plasma protein fragments and peptides may allow for efficient activation of platelets and at the same time for an optimal control of the coating process of the surfaces of the channels or channel-like structures. Of course, all of these components are present if a plasma fraction obtained from the extracorporeal amount of blood is used for coating and activation.

For efficient binding of the platelets to the plasma components and activation thereby, the flow rate may be adjusted upward or downward compared to the coating step of the plasma components, or flow may be stopped for a period of time, to obtain the desired level of platelets bound to the plasma components. The flow rates for plasma activation will typically be in the range of about 5 ml/min to about 200 ml/min, of about 10 ml/min to about 150 ml/min, of about 10 ml/min to about 100 ml/min, or of about 5 ml/min to about 50 ml/min. Typically, it will be desirable to allow between about 1 to 60 min, between about 1 to about 30 min, between about 1 to about 20 min, or between about 1 to about 10 min for the platelets to bind to the plasma components.

Even though shear stress does not seem to of the same importance for activation of platelets as for activation of monocytes, it can be preferred to pass the platelets fraction through the flow chamber under a shear force of about 0.1 to about 20.0 dynes/cm$^2$, of about 0.2 to about 15.0 dynes/cm$^2$, of about 0.3 to about 10.0 dynes/cm$^2$ such as from

about 0.2 to about 0.4, to about 0.5, to about 0.6, to about 0.7, to about 0.8, to about 0.9, to about 1, to about 2, to about 3, to about 4, to about 5, or to about 6 dynes/cm$^2$. Typical flow rates of the platelets-containing fraction may be in the range of about 5 ml/min to about 200 ml/min, of about 10 ml/min to about 150 ml/min, of about 10 ml/min to about 100 ml/min, or of about 5 ml/min to about 50 ml/min. The flow rates will depend to some extent on the size and geometry of the flow chamber and can particularly be used if flow chamber of the below-mentioned dimensions are used. In general, one will select flow rates to achieve the afore-mentioned shear stress values.

Thus, it is contemplated to pass the platelets-containing fraction through the channels or channel-like structures with a flow rate of about 10 ml/minute to about 200 ml/minute to produce a shear force of about 0.1 to about 10.0 dynes/cm$^2$.

After the platelets have been passed through the channels or channel-like structures and have been activated by the plasma proteins or fragments thereof, which have been disposed on the surfaces of the channels or channel-like structures thereof, the monocytes-containing fraction, e.g. the extracorporeal quantity of said mammalian subject's blood sample or the below-mentioned leukocyte or buffy coat fraction, which have been obtained from the extracorporeal amount of blood sample, is passed by e.g. pumping into and through the channels or channel-like structures, by applying a shear force. It is to be understood that activation of platelets through interaction with plasma components will lead to adherence of platelets to plasma components.

It is also to be understood that the same events as described above will happen if an extracorporeal quantity of a mammalian subject's blood sample comprising platelets and plasma components is passed through the flow chamber. In this case, plasma components will adhere to the walls to the flow chamber and then activate platelets. However, in this scenario the process may be less controllable and account may be taken of this by increasing the residence time of the extracorporeal quantity of a mammalian subject's blood sample comprising platelets and plasma components in the flow chamber.

It is further to be noticed that instead of activated platelets, factors derived from platelets may be used, which are sufficient to activate monocytes. These factors include e.g. fibronectin and may also include factors such as P-selectin, Integrin α5β1the C-type lectin receptor, CD61, CD36, CD47 and complement inhibitors such as CD55 and CD59, or TREM-like transcipt-1. Such platelet-derived factors may also be disposed directly on the surfaces of the flow chamber either as e.g. mixtures of purified components or mixtures of components obtained by e.g. lysis of platelets contained within the extracorporeal quantity of a mammalian subject's blood sample. In this case, the need for e.g. coating the surfaces of the flow chamber with plasma components may be bypassed.

The data presented herein suggest that once platelets have been activated, proteins such as P-selectin and RGD-containing ligands are expressed by the activated platelets, which can then interact with monocytes and activate their differentiation into immuno-stimulatory dendritic cells. Moreover, it was found that monocyte activation and dendritic cell induction by activated platelets do not occur under static conditions. Rather monocytes need to be passed through the channels or channel-like structures under application of a shear force. Given that platelets upon activation need about 60 to about 120 min to express factors such as P-selectin, which then activates monocytes, passing of monocytes may be delayed until platelets have started to express these factors, e.g. for about 60 to about 120 min. If an extracorporeal quantity of a mammalian subject's blood sample comprising monocytes, platelets and plasma components is passed through the flow chamber, this time period may have to be adjusted to longer times.

It is to be understood that interaction of monocytes with activated platelets, platelet-derived factors or plasma components is not sufficient for activation and differentiation of monocytes without the application of a shear force at the same time.

Application of a physical force for moving the monocytes-containing fraction through the flow chamber preferably means that a monocytes-containing fraction such as the extracorporeal quantity of a mammalian subject's blood sample is moved through the flow chamber under shear stress. Typically, monocytes-containing fraction may be passed through the flow chamber under a shear force of about 0.1 to about 20.0 dynes/cm$^2$, of about 0.2 to about 10.0 dynes/cm$^2$, such as from about 0.2 to about 0.3, to about 0.4, to about 0.5, to about 0.6, to about 0.7, to about 0.8, to about 0.9, to about 1, to about 1.5, or to about 2 dynes/cm$^2$. Typical flow rates of the monocytes-containing fraction may be in the range of about 5 ml/min to about 200 ml/min, of about 10 ml/min to about 150 ml/min, of about 10 ml/min to about 100 ml/min, or of about 5 ml/min to about 50 ml/min. The flow rates will depend to some extent on the size and geometry of the flow chamber and can particularly be used if channels or channel-like structures of the below-mentioned dimensions are used. In general, one will select flow rates to achieve the afore-mentioned shear stress values.

Thus, it is contemplated to pass the monocytes-containing fraction through the channels or channel-like structures with a flow rate of about 10 ml/minute to about 200 ml/minute to produce a shear force of about 0.1 to about 0.5 dynes/cm$^2$. In any case it must be made sure that a shear force is generated that allows binding of monocytes to activated platelets and differentiation of such activated monocytes into immuno-stimulatory DC.

The data presented herein suggests that monocyte-platelet interaction can be divided into short-acting interactions which are arbitrarily defined as contact occurring for less than 3 seconds by detection with a light microscope and long-acting interactions, defined as contact longer than 3 seconds by detection with a light microscope. It seems that the initial short-acting interactions are mediated by P-selectin which is expressed on activated platelets. These initial contacts can then subsequently trigger long-acting interactions mediated by RGD-containing proteins expressed by the activated platelets.

The activation of monocytes and differentiation into immuno-stimulatory DC may be positively influenced by allowing the monocytes to establish long-acting contacts with platelets, e.g. by giving the monocytes and platelets enough time to interact. As the monocytes flow through the channels or channel-like structures, they alternately bind to and disadhere from the platelets by the shearing force induced by the flow through the channels or channel-like structures. The residence time of the monocyte/platelet interaction may be controlled by varying the flow rate, e.g. by controlling the speed of the pump. For example, the pump may initially be operated at a slow speed/low flow rate to enhance monocyte/platelet interaction, and the speed/flow rate may then be increased to facilitate disadherence and collection of the treated monocytes from the treatment device. It seems that adherence of the monocytes to the platelets may be best accomplished at about 0.1 to about 2 dynes/cm$^2$, at about 0.1 to about 1 dynes/cm$^2$, and preferably at about 0.1 to about 0.5 dynes/cm$^2$, while disadherence and collection of the monocytes may be best accomplished at increased shear levels.

It is to be understood that activation of monocytes leads to immobilization, e.g. by interacting with activated platelets, platelets-derived factors or plasma components. In order to harvest the induced immuno-stimulatory autologous dendritic cells, one may increase the shear stress to e.g. 20 Dynes/cm$^2$ and/or may treat the immuno-stimulatory autologous dendritic cells with factors allowing disadherence from activated platelets, platelets-derived factors or plasma components by adding factors such as Plavic, Aspirin or other blood thinners.

Temperature is another factor to influence activation of monocytes and their differentiation into immuno-stimulatory autologous dendritic cells. The methods in accordance with the invention may be performed in a range of about 18°C to about 42°C, preferably in a range of about 22°C to about 41°C and more preferably in a range of about 37°C to about 41°C.

One parameter that can also be varied to tune activation of monocytes is the density by which the flow chamber is coated with plasma components and thus with platelets that bind to the plasma components. In general, the denser the surfaces of the flow chamber are coated with plasma components and platelets, the more efficient will be the monocyte activation.

It has been mentioned above that platelets are activated by binding to plasma components. The term "activated platelets" in accordance with the invention is used to refer to platelets which show an increased expression of P-selectin, αIIb-β3 integrin and/or RGD-containing proteins such as fibronectin, fibrinogen or vitronectin as a consequence of binding of platelets to plasma components such as fibronectin and/or fibrinogen. Expression may be determined by conventional methods such as RT-PCR, Western-Blotting or FACS analysis. The term "unactivated platelets" in accordance with the invention is used to refer to platelets for which binding to plasma proteins such as fibronectin or fibrinogen cannot be reduced by pre-incubating platelets with the gamma component of fibrinogen.

It has been mentioned above, that monocytes are activated and start to differentiate into immuno-stimulatory autologous dendritic cells by binding to activated platelets under shear stress conditions. The term "activated monocytes" in accordance with the invention is used to refer to monocytes which upon binding to activated platelets under shear stress conditions express increased levels of the open confirmation of β1-integrin and start expressing markers of maturing DC such as HLA-DR$^+$/CD83$^+$. As a control to determine whether interaction of monocytes with activated platelets leads to activation and differentiation of DC one can compare expression of HLA-DR$^+$/CD83$^+$ after binding of monocytes to activated platelets under shear stress condition either in the absence of anti-P-selectin antibodies (activation) or presence of anti-P-selectin antibodies (control). Expression may be determined by conventional methods such as RT-PCR, Western-Blotting or FACS analysis.

After monocytes have been activated by a method in accordance with the invention, they start differentiating into immuno-stimulatory autologous dendritic cells. The term "immuno-stimulatory autologous dendritic cells" in accordance with the invention is used as mentioned above. These immuno-stimulatory autologous dendritic cells can be identified by expression of markers described above. Immuno-stimulatory autologous dendritic cells can be further distinguished from immuno-suppressive or so-called truncated autologous dendritic cells in that no change in expression of GILZ is observed when obtaining autologous dendritic cells by a method in accordance with the invention.

The experimental findings described herein further immediately suggest various embodiments of this first aspect that can provide for different advantages.

The finding, that activation of monocytes and subsequent induction of differentiation of these monocytes into immuno-stimulatory autologous DC can be achieved in a miniaturized device, allows to conduct the process with smaller amounts of an extracorporeal blood sample. As mentioned above, the classical ECP procedure requires processing of 2.5 L to 6 L blood, which is typically obtained from patients by apheresis such as leukaphereses, to obtain a final volume of about 200 ml to 500 ml comprising leukocytes including monocytes as well as plasma components and platelets.

However, the methods in accordance with the invention may require substantial lower amount of blood samples thus bypassing the need of apheresis such as leukaphereses or other processes, which are a considerable burden to patients.

Thus, the present invention can be performed without the need for apheresis such as leukaphereses and the whole process of obtaining such immuno-stimulatory autologous dendritic cells may be performed in a handheld device.

Thus, in one embodiment of the first aspect of the invention, which may be combined with the above described embodiments, it is contemplated to perform the method in accordance with the first aspect, wherein said extracorporeal quantity of said mammalian subject's blood has not been obtained by apheresis such as leukaphereses.

Said extracorporeal quantity of said mammalian subject's blood may be between about 5 ml to about 500 ml, between about 10 ml to about 450 ml, between about 20 ml to about 400 ml, between about 30 ml to about 350 ml, between about 40 ml to about 300 ml, or between about 50 ml to about 200 ml or between about 50 ml to about 100 ml of extracorporeal blood of said mammalian subject to give a final volume between about 1 ml to about 100 ml, between about 1 ml to about 50 ml, between about 1 ml to about 40 ml, or between about 1 ml to about 30 ml an extracorporeal amount of a mammalian's blood sample.

The quantity of extracorporeal blood withdrawn and applied to the device may be whole blood. Alternatively, said extracorporeal quantity of said mammalian subject's blood may be obtained by isolating leukocytes from between about 5 ml to about 500 ml, between about 10 ml to about 450 ml, between about 20 ml to about 400 ml, between about 30 ml to about 350 ml, between about 40 ml to about 300 ml, or between about 50 ml to about 200 ml or between about 50 ml to about 100 ml of extracorporeal whole blood of said mammalian subject.

Said extracorporeal quantity of said mammalian subject's blood may also be obtained by isolating buffy coats from between about 5 ml to about 500 ml, between about 10 ml to about 450 ml, between about 20 ml to about 400 ml, between about 30 ml to about 350 ml, between about 40 ml to about 300 ml, or between about 50 ml to about 200 ml or between about 50 ml to about 100 ml of extracorporeal whole blood of said mammalian subject.

In all of the afore-mentioned cases (whole blood, leukocyte fraction, buffy coats), said extracorporeal amount of blood will typically comprise between about $1 \times 10^4$ to about $1 \times 10^8$ such as about $5 \times 10^6$ mononuclear cells/ml.

The person skilled in the art is familiar how to obtain whole blood, a leukocyte fraction thereof or a buffy coat fraction thereof (see e.g. Bruil et al., Transfusion Medicine Reviews (1995), IX (2), 145-166) an include filtration, differential centrifugation. A preferred method relies on filters as they are available from e.g. Pall. Such filters may be incorporated into the device such that processing of the extracorporeal sample can be done in the handheld device. As a source one can also use e.g. blood of the umbilical cord.

If one uses centrifugation, one may obtain whole blood through a syringe with e.g. a 17 or 18 gauge-gauge needle. Such a whole blood sample may be centrifuged to remove debris and other components. The whole blood sample may then be filtered through common filters, as they are available from Pall.

For obtaining a mononuclear leukocyte fraction, one may obtain a whole blood sample as described and then layer such a sample on e.g. Ficoll-Hypaque. Subsequently a centrifugation step is performed at e.g. about 100g to about 200 g such as 180 g and the mononuclear leukocyte fraction can then be collected from the interface and washed with common buffers such as HBSS. The washed mononuclear leukocyte fraction can then be resuspended in serum-free cell culture medium such as RPMI-1640 medium (GIBCO). Other methods for obtaining mononuclear leukocyte fractions include elutriation, filtration, density centrifugation, etc..

As pointed out above, crucial steps for the induction of DC formation seem to involve the activation of platelets by plasma components and the activation of monocytes by such activated platelets. In principle, one could pass a whole blood sample through the device under shear stress. The plasma components of such a sample will then bind to the surfaces of the flow chamber and allow for adherence and activation of platelets within such a sample by plasma-components. The monocytes of such a sample will then bind to the activated platelets and be activated themselves.

Similarly one may obtain combinations of the various components such as a platelet-rich plasma containing fraction which may be obtained by centrifuging a whole blood sample which has been obtained as described above at about 100 g to about 180 g such as about 150 g for about 10 min to about 20 min such as about 15 min to separate the debris of the whole blood sample. The platelet-rich plasma layer is then collected and recentrifuged at about 700 g to about 1000 g such as about 900 g for about 3 min to about 10 min such as about 5 min. The resultant pellet is then resuspended in serum-free cell culture medium.

However, in order to have the best control over the process, it may be desirable to first pass plasma components through the flow chamber and let them adhere, then platelets and then the monocytes-containing fraction. For this approach, it may be desirable to obtain a leukocyte fraction comprising a monocytes- or buffy-coat fraction comprising monocytes, which does not comprise plasma components and which does not comprise platelets. Such plasma- and platelet-free monocytes-containing fractions may be obtained as is described in the art. If leukocyte or buffy-coat fractions are obtained as described above, they will be sufficiently free of plasma or platelets for the purposes of the invention. For this approach, it may also be desirable to have platelet- and/or plasma-fractions.

Thus, the invention contemplate to use platelets which have been separated from the extracorporeal quantity of said mammalian subject's blood before said extracorporeal quantity of said mammalian subject's blood is applied to said device. These platelets may then be passed through the flow chamber, which has been coated with plasma components such as fibronectin.

In another embodiment, the invention considers to use plasma components, which have been separated from the extracorporeal quantity of said mammalian subject's blood before said extracorporeal quantity of said mammalian subject's blood is applied to said device. These plasma components may then be passed through flow chamber so that they can adhere.

Instead of using plasma components which have been obtained from the extracorporeal amount of blood, one may also use plasma components, which have been isolated from other sources such as e.g. by recombinant protein expression. Such plasma components include fibrinogen, fibronectin, P-selectin, and fragments thereof such as the gamma component of fibrinogen.

Even though it may be preferred to use an extracorporeal amount of blood, which has not been obtained by apheresis such as leukaphereses, using an extracorporeal amount of blood, which has been obtained by apheresis such as leukaphereses is not excluded by the invention.

Thus, in another embodiment of the first aspect of the invention it is contemplated to perform the method as described above, wherein said extracorporeal quantity of said mammalian subject's blood has been obtained by apheresis such as leukaphereses.

Apheresis such as leukaphereses may be performed as is known in the art. Thus, an extracorporeal quantity of blood such as 2.5 L to 6l may be obtained from a subject and treated by conventional leukaphereses to obtain three fractions, namely the plasma, the platelets and the buffy coats. The plasma, which contains proteins such as fibronectin and fibrinogen, is the lightest blood fraction, and therefore is the first portion of the blood selectively removed from the centrifuge and passaged through channels or channel-like structures. After the plasma has been pumped through the channels or channel-like structures and the surfaces thereof have been coated with plasma proteins, the second lightest component in the leukaphereses centrifuge, the platelet fraction, is pumped into and through the channels or channel-like structures. The third lightest fraction to be eluted from the leukaphereses centrifuge is the buffy coat, which contains the white blood cells, including the blood monocytes. The buffy coat including the monocytes is then pumped through the channels or channel-like structures. Blood sample may be obtained using the Therakos device, the Spectra cell separator (see Andreu et al., (1994), Transf. Sci., 15(4), 443-454), or the Theraflex device from Macopharma.

Thus, the invention in one embodiment the invention considers to use platelets which have been separated from the extracorporeal quantity of said mammalian subject's blood obtained by apheresis such as leukaphereses before said extracorporeal quantity of said mammalian subject's blood comprising monocytes is applied to said device.

In another embodiment the invention considers to use plasma components, which have been separated from the extracorporeal quantity of said mammalian subject's blood obtained by apheresis such as leukaphereses before said extracorporeal quantity of said mammalian subject's blood comprising monocytes and/or platelets is applied to said device.

Instead of using plasma components which have been obtained from the extracorporeal amount of blood, one may use also either plasma components which have been isolated from other sources such as e.g. by recombinant protein expression. Such plasma components include fibrinogen, fibronectin, or P-selectin. One can also use fragments of plasma proteins such as the gamma component of fibrinogen which corresponds to amino acids 400-411 (SEQ ID NO.: 105, His-His-Leu-Gly-Gly-Ala-Lys-Gln-Ala-Gly-Asp-Val). This gamma component is shown by the data presented herein to be able to activate platelets. It can therefore be preferred to use plasma fractions, which at least, if not predominantly comprise fibronectin. Similarly, it can be preferred to use e.g. recombinantly expressed and/or purified fibronectin or the gamma component thereof to activate platelets.

For both embodiments of the first aspect of the invention where the extracorporeal amount of blood has been obtained or has not been obtained by apheresis such as leukaphereses, it may be considered to pass all three fractions, namely plasma components, platelets and the monocytes-containing fraction at once, e.g. even in the form of a whole blood sample or by using only pre-purified fractions of whole blood, through the flow chamber even though the aforedescribed sequential passing of these fractions through the flow chamber may provide for better control over the process. Pre-purified fractions of whole blood may be obtained by e.g. centrifuging a blood bag and squeezing out the supernatant, which would be enriched in white blood cells and platelets.

As mentioned the flow rate through flow chamber and thus the resulting shear stress will effect the differentiation of the monocytes into immuno-stimulatory autologous dendritic cells. Aside from the flow rate, the design and the dimensions of the flow chamber may be varied to manipulate and even improve the application of a shear force to the monocytes.

A device having a flow chamber with channels or channel-like structures may be suitable. Such a flow chamber having the general architecture, albeit at smaller dimensions, of a device, which is used for the classical ECP procedure is depicted in Fig. 17.

However, other geometries such as those depicted in Fig. 18 a) to d) may also be used. Thus, the findings described herein allow to consider flow chambers of significantly simplified geometry, which also allows having better control over the process in terms of turbulences and shear stress occurring during the process.

A device having a multiplicity of flow chambers may be suitable. Such a flow chamber having the general architecture, albeit at smaller dimensions, of a device, which is used for the classical ECP procedure is depicted in Fig. 17.

Typically, a flow gradient will be created in the flow chamber such as channels as the monocytes-containing fraction is passed through. The monocytes will alternately bind to and disengage from the platelets and/or plasma components. Maturation of monocytes into immuno-stimulatory autologous dendritic cells is greatly enhanced by this interaction, with increased exposure to the platelets and/or plasma components thereby providing increased signaling of this maturational process.

In order to obtain a homogenous population of immuno-stimulatory autologous dendritic cells as possible, it is therefore desirable that the design and the dimensions of the flow chamber, such as channels is selected to avoid different flow zones in the flow chamber.

The flow chamber such as channels may in principle have any cross-sectional shape suitable for the above-described purposes. They thus may have a rectangular, round, elliptical, or other cross-sectional form. Even though the dimensions of such flow chamber will be discussed in the following mainly with respect to a rectangular cross-section, it can be preferred that flow chamber such as channels with an elliptical or round cross-section are used as such cross-sections should allow for e.g. more homogenous coating with plasma components and/or more continuous flow properties with less turbulences.

If having a rectangular cross-section, flow chamber such as channels may have dimensions of about 5 $\mu$m to up to about 500 $\mu$m of height and of about 5 $\mu$m to up to about 500 $\mu$m of width. The channels or channel-like structures may also have dimensions of about 10 $\mu$m to up to and including about 400 $\mu$m of height and of about 10 $\mu$m to up to and including about 400 $\mu$m of width, of about 10 $\mu$m to up to and including about 300 $\mu$m of height and of about 10 $\mu$m to up to and including about 300 $\mu$m of width, of about 10 $\mu$m to up to and including about 250 $\mu$m of height and of about 10 $\mu$m to up to and including about 250 $\mu$m of width, of about 10 $\mu$m to up to and including about 100 $\mu$m of height and of about 10 $\mu$m to up to and including about 100 $\mu$m of width, or of about 10 $\mu$m to up to and including about 50 $\mu$m of height and of about 10 $\mu$m to up to and including about 50 $\mu$m of width.

If flow chambers such as channels of elliptical cross-section are used, the aforementioned dimensions of height and width would have to be adapted correspondingly to allow for a comparable volume.

If flow chambers such as channels of round cross-sections are used, the diameter may typically be in the range of about 5 $\mu$m to up to and including about 500 $\mu$m, of about 10 $\mu$m to up to and including about 400 $\mu$m, of about 10 $\mu$m to up to and including about 300 $\mu$m, of about 10 $\mu$m to up to and including about 250 $\mu$m, of about 10 $\mu$m to up to and including about 100 $\mu$m, or of about 10 $\mu$m to up to and including about 50 $\mu$m.

Smaller dimensions are generally preferred for the flow chambers with a particular preference for height, widths or diameters of below 100 $\mu$m such as 50 $\mu$m the reason being that it is assumed that for such smaller dimensions interaction of monocytes with platelets is more efficient and uniform and flow properties at the surfaces and in the center of the flow chamber are more comparable.

The length of the flow chamber such as channels channel-like structures is usually selected such that the flow chamber allows for passage of the volume of extracorporeal blood. For example the flow chamber and the device may be configured to allow for passing of an overall volume of between about 1 ml to about 50 ml, between about 1 ml to about 40 ml, or between about 1 ml to about 30 ml.

The flow chamber may have internal sub structures to increase the surface area or to make the flow conditions less heterogeneous.

The flow chamber may be filled with particles to increase the surface area or to make the flow conditions less heterogeneous.

The material of the flow chamber may be plastic or non-plastic.

If non-plastic materials are considered, one may use glass.

The surface of the chamber may be coated covalently or via adsorption.

Materials for auxiliary tubing, chambers, valves etc. may be selected to for having reduced interactions with blood components.

Surfaces of auxiliary tubing, chambers, valves etc. may be treated/coated for having reduced interactions with blood components.

If plastic materials are considered, one may use acrylics, polycarbonate, polyetherimide, polysulfone, polyphenylsulfone, styrenes, polyurethane, polyethylene, teflon or any other appropriate medical grade plastic. In a preferred embodiment of the present invention, the flow chamber is made from an acrylic plastic.

The flow chamber may be made of a material that provides a degree of transparency such that the sample within the flow chamber such as the monocytes-containing fractions can be irradiated with visible or UV light, preferably with UV-A. As is shown by the experiments, exposure to UV-A and 8-MOP leads to increased expression of GILZ and thus to activation and differentiation of monocytes into immuno-suppressive autologous dendritic cells. Thus exposure to light such as UV-A and DNA-cross linking agents such as 8-MOP should be generally avoided when producing immuno-stimulatory autologous dendritic cells.

However, once monocytes have embarked on the maturation pathway long enough such that immuno-stimulatory autologous dendritic cells have formed as can be determined by the molecular markers mentioned above, one can envisage to administer DNA-cross linking agents such as 8-MOP and to expose the immuno-stimulatory autologous dendritic cells to e.g. UV-A to render other cells in the extracorporeal blood sample apoptotic. Such cells may be cytotoxic T-cells, virally infected cells or bacterial cells. Apoptosis of such cells may lead to antigen shedding. The immuno-stimulatory autologous dendritic cells can then taken up and process these antigens so that immuno-stimulatory autologous antigen-presenting cells are formed. These immuno-stimulatory autologous antigen-presenting cells can then e.g. be re-introduced into the respective individual to elicit an immune response against the respective tumor, viral or bacterial antigens. Once immuno-stimulatory autologous dendritic cells have formed, one may also separately introduce tumor cells, bacterial cells or virally infected cells of such an individual into the flow chamber and render these cells apoptotic by e.g. additionally adding DNA-cross linking agents such as 8-MOP and irradiate the mixture of immuno-stimulatory autologous dendritic cells and tumor cells, bacterial cells or virally infected cells to render the tumor cells, bacterial cells or virally infected cells apoptotic such that immuno-stimulatory autologous antigen-presenting cells can form. It is for these embodiments, that a design of the flow chamber allowing exposure to light such as UV-A is contemplated.

A typical flow chamber may have the geometry depicted in Fig. 19A). The flow path has dimensions of 20 mm by 80 mm. The chamber is made of polystyrene, PET (polyethylenteherephtalate), PMMA (poly (methyl mathacrylate)) and silicon. A blood sample may be spun at low speed through a Ficoll gradient to obtain e.g. 8 ml of sample with a concentration of white blood cells of e.g. $10^{10}$ cells/ml. The chamber may be pre-coated with platelets-rich plasma. The sample may be passed through the chamber at about 0.028 Pa for about ... min. The chamber may then be washed with about 3 ml RPMI at 0.028 Pa. A second wash with 30-55 ml RPMI may be performed at about 1.2 Pa. The collected activated monocytes will then be combined and used for further analysis.

Once immuno-stimulatory autologous dendritic cells have been obtained by methods in accordance with the invention, they can be generally further processed for specific purposes. These newly formed immuno-stimulatory dendritic cells can for example be incubated under standard conditions to allow completion of their maturation. Culturing of these immuno-stimulatory dendritic cells can be performed under standard conditions, e.g. at 37° C and 5% $CO_2$ in standard mediums for culturing of human cells such as in RPMI-1640 medium (obtainable e.g. from GIBCO), supplemented with 15% AB serum (obtainable from e.g. Gemini Bio-Products).

In this way mature immuno-stimulatory dendritic cells can be obtained without the need for rather expensive cocktails of cytokines for induction of monocyte to DC differentiation. Even though not necessary, it can be considered to cultivate such immuno-stimulatory dendritic cells in a buffered culture medium with one or more cytokines, such as GM-CSF and IL-4, during the incubation period. Maturation cocktails (typically consisting of combinations of ligands such as CD40L, cytokines such as interferon gamma, TNF alpha, interleukin 1 or prostaglandin E2 or the factors mentioned above) may be added as well. In one aspect, one preferentially produce immuno-stimulatory dendritic cells over immuno-suppressive dendritic cells by cultivating the dendritic cells over extended periods of time such as e.g. at least 1, at least 2, at least 3, at least 4, or at least 5 days. This may help the initially formed immuno-stimulatory dendritic cells to further embark on their maturation pathway. However, according to the invention, immuno-stimulatory dendritic cells are obtained without cocktails of cytokines for induction of monocyte to DC differentiation..

The immuno-stimulatory dendritic cells in accordance with the present invention can be tested for the functionality in assays as described herein. For example, one can adapt the assay described in Bioley et al., The Journal of Immunology 2006), 177:6769-6779. In such an adapted assay immuno-stimulatory dendritic cells, which are obtained by the methods disclosed herein e.g. by passing white blood cells as described above through a device depicted in Fig. 19, are co-incubated with CD4$^+$ and CD8$^+$ cells of the same donor and the Melan-A/MART-1$_{26-35}$ Peptide, described by Bioley et al. Detection of Melan-A/MART-1$_{26-35}$ positive CD4$^+$ cells and CD8$^+$ cells allows confirmation of functional immuno-stimulatory dendritic cells which are obtained in accordance with the present invention.

Further such immuno-stimulatory dendritic cells can then be manipulated ex vivo, prior to re-administration to the subject, in order to tailor them for the desired therapeutic purpose.

Thus, prior to re-administration to the subject, such immuno-stimulatory dendritic cells can e.g. be processed ex vivo, such as by loading them with immunogenic antigens, e.g. those expressed on apoptotic tumor cells or pathogenic infectious agents, or enhancing their maturation in order to increasing their efficiency in cancer immunotherapy. This will lead to immuno-stimulatory antigen-presenting cells displaying the antigen on their surfaces. One of the most preferred embodiments of the present invention contemplates to separate as much as possible the generation of immuno-stimulatory dendritic cells as described herein, the generation of disease-antigens and the loading of these antigen-presenting cells with the disease-antigens. Thus, other than e.g. in ECP these different processes do not occure in a continuos manner, e.g. by avoiding application of photoactivatable agents such as 8-MOP and UVA. Rather, immuno-stimulatory antigen-presenting cells such as dendritic cells are made from monocytes by applying shear forces in the absence of e.g. 8-MOP and UVA and/or cytokine coctails. These immuno-stimulatory dendritic cells may then be co-incubated with disease-antigens which were obtained separately to effect efficient loading of the immuno-stimulatory dendritic cells and display of the antigens on their surface. It is the insight provided by the findings herein that allows to separate the multiple processes occuring during ECP and to fine-tune generation of immuno-stimulatory dendritic cells by e.g. avoiding or reducing formation of immuno-suppressive dendritic cells.

The immuno-stimulatory dendritic cells may in particular be loaded with disease effector agents to produce antigen presenting dendritic cells, which upon reintroduction into the subject can launch an immune response against the disease effector genes.

As used herein, the term "disease effector agents" refers to agents that are central to the causation of a disease state in a subject. For example immuno-stimulatory dendritic cells may be loaded with antigens, which are known being expressed in cancerous tissue. To this end, such antigens may be expressed in immuno-stimulatory dendritic cells such that they are displayed on the MHC class 1 and MHC class 2 molecules. By priming immuno-stimulatory dendritic cells with such antigens, subjects may be vaccinated against the later occurrence of e.g. a cancer or an infection. If an antigen is used, which is already expressed by cancerous tissue obtained from the subject, from whom the extracorporeal amount of blood was taken to generate immuno-stimulatory dendritic cells, the antigen-loaded, reintroduced immuno-stimulatory antigen-presenting cells may launch an immune response against the cancer.

In certain circumstances, these disease effector agents are disease-causing cells which may be circulating in the bloodstream, thereby making them readily accessible to extracorporeal manipulations and treatments. Examples of such disease causing cells include e.g. malignant T-cells, malignant B cells, and virally or bacterially infected white or red blood cells which may harbor or express microbial (e.g. viral, bacterial, fungal, mycobacterial, protozoal) peptides or proteins or other pathogen-associated molecules. Exemplary disease categories giving rise to disease-causing cells include lymphoproliferative disorders such as leukemia, lymphoma, and myeloma, as well as infections including malaria, human-immunodeficiency virus (HIV), Epstein-Barr virus (EBV), cytomegalovirus (CMV), hepatitis B virus (HBV), hepatitis C virus (HCV), Lyme disease, leprosy, tuberculosis, and other blood borne pathogens.

Other disease-causing cells include those isolated from surgically excised specimens from solid tumors, such as lung, colon, brain, kidney or skin cancers. These cells can be manipulated extracorporeally in analogous fashion to blood leukocytes, after they are brought into suspension or propagated in tissue culture. Alternatively, in some instances, it has been shown that the circulating blood of patients with solid tumors can contain malignant cells that have broken off from the tumors and entered the circulation. These circulating tumor cells can provide an easily accessible source of cancer cells, which may be isolated, rendered apoptotic and engulfed by the dendritic cells in accordance with the method described and claimed herein.

Disease effector agents may also be obtained from such disease-causing cells, which have been rendered apoptotic by e.g. cytotoxic agents. It is to be understood that apoptotic cells may send different signals through effector agents depending on whether they are derived from normal or abnormal cells, such as healthy or malignant cells. Combining and cultivating immuno-stimulatory dendritic cells with such apoptotic cells may also be used to load immuno-stimulatory dendritic cells with disease-causing antigens and generate immuno-stimulatory antigen-presenting cells.

In addition to disease-causing cells, disease effector agents falling within the scope of the invention further include microbes such as bacteria, fungi and viruses, which express disease-associated antigens. It should be understood that viruses can be engineered to be "incomplete", i.e., produce distinguishing disease-causing antigens without being able to function as an actual infectious agent, and that such "incomplete" viruses fall within the meaning of the term "disease effector agents" as used herein.

Immunogenic antigens may also be obtained by treating cancer specimens or cancer cells with agents which are know to induce immunogenic antigens such as Bortezomib.

Cancers which may be in particular treatable by the above described approaches include CTLC, Melanoma, Prostate cancer, of HNSCC as e.g. disease-antigens are know for some of these disease or as e.g. animal models of some of these diseases can be used initially.

As mentioned above, by loading immune-stimulatory autologous dendritic cells obtainable by the methods described herein with antigens allows producing immuno-stimulatory autologous antigen-presenting cells.

In order to avoid e.g. protein degradation of delivered antigen and inefficient processing of soluble antigens by dendritic cells, leading to poor T-cell responses, it is contemplated to enhance formation of immuno-stimulatory autologous antigen-presenting cells by encapsulation of antigens in polymeric nanoparticles (NPs), which may be made from biodegradable polymers such as polylactic acid (Waeckerle-Men et al., Adv Drug Deliv Rev (2005), 57:475-82). Such NPs may be further modified with targeting moieties for DEC-205 such as an DEC-205 antibody to improve receptor-mediated endocytosis and antigen presentation.

Thus the present disclosure contemplates to use encapsulation of antigens in polymeric nanoparticles to optionally further enhance formation of immuno-stimulatory autologous antigen-presenting dendritic cells.

The immuno-stimulatory dendritic cells obtained in accordance with the invention and disease effector agents are incubated for a period of time sufficient to maximize the number of functional antigen presenting dendritic cells in the incubated cell population. Typically, the treated blood cell concentrate and disease effector agents are incubated for a period of from about 1 to about 24 hours, with the preferred incubation time extending over a period of from about 12 to about 24 hours. Additional incubation time may be necessary to fully mature the loaded immuno-stimulatory antigen-presenting cells prior to reintroduction to the subject. Preferably, the blood cell concentrate and disease effector agents are incubated at a temperature of between 35° C and 40° C. In a particularly preferred embodiment, the incubation is performed at about 37°C.

Inducing monocyte differentiation according to the method described above provides immuno-stimulatory dendritic cells in numbers which equal or exceed the numbers of dendritic cells that are obtained by expensive and laborious culture of leukocytes in the presence of cytokines such as GM-CSF and IL-4 for a couple of days. The large numbers of functional dendritic cells generated by the method described above provide a ready means of presenting selected material, such as, for example, apoptotic cells, disease agents, antigens, plasmids, DNA or a combination thereof, and are thereby conducive to efficient immunotherapy. Antigen preparations selected to elicit a particular immune response may be derived from, for example, tumors, disease-causing non-malignant cells, or microbes such as bacteria, viruses and fungi. The antigen-loaded dendritic cells can be used as immunogens by reinfusing the DC into the subject or by otherwise administering the cells in accordance with methods known to elicit an immune response, such as subcutaneous, intradermal or intramuscular injection. As described below, it is also possible to generate antigen-loaded dendritic cells by treating and co-incubating monocytes and disease effector agents, which are capable of expressing disease associated antigens.

As mentioned above, immuno-stimulatory dendritic cells can be obtained by a method in accordance with the invention in the absence of a photoactivatable agent and without exposure to light such as visible and preferably UV-A.

The present invention thus aims at obtaining individual-specific functionally and maturationally synchronized autologous immuno-stimulatory dendritic cells.

In a second aspect, the present disclosure relates to autologous immuno-stimulatory dendritic cells obtainable by a method described herein, preferably for use in immunization against cancer antigens, viral antigens, bacterial antigens or fungal antigens.

The invention is now described with respect to some specific examples which, however, are for illustrative purposes and not to be construed in a limiting manner.

**Experiments**

**Experiment 1 - Shear stress and platelet activation for inducing monocyte activation**

**Materials and Methods**

*Procurement of leukocytes and platelets*

All samples were acquired from young, healthy subjects not taking medications, including aspirin, known to influence platelet function. Samples were obtained under the guidelines of the Yale Human Investigational Review Board, and informed consent was provided according to the Declaration of Helsinki. Peripheral blood specimens were collected through a 19-gauge needle from the antecubital vein into syringes containing heparin, then layered on Ficoll-Hypaque (Gallard-Schlessinger, Carle Place, N.Y.). Following centrifugation at 180g, the interface containing the mononuclear leukocyte fraction was collected and washed twice in HBSS, then resuspended in RPMI-1640 medium (GIBCO) to a final concentration of $5 \times 10^6$ mononuclear cells/ml. Cells were utilized within one hour of being acquired.

*Preparation of Platelet-rich-Plasma*

Whole blood was centrifuged at 150 g for 15 min at room temperature. The platelet-rich-plasma (PRP) layer was collected and centrifuged at 900 g for 5 min, and the platelet pellet resuspended in RPMI 1640 to the desired concentration.

*Preparation of Parallel-Plates*

Two likes of parallel-plate flow chambers were used to model the flow dynamics of ECP. Experiments involving the assessment of cell phenotype post-flow were conducted using the larger Glycotech system (Glycotech, Rockville, MD). This system consisted of a volumetric flow path measuring 20000 × 10000 × 254 microns (length × width × height). The bottom plate in this system was composed of a 15mm petri dish (BD Biosciences, Durham, NC) separated by a gasket and vacuum-connected to an acrylic flow deck, which formed the upper plate. For experiments requiring the plates to be pre-coated with platelets, prior to assembling the flow chamber, 20 drops of the desired concentration of PRP was placed in the center of the petri dish and platelets allowed to settle for 20 minutes at room temperature. The petri dish was washed twice with 2ml of RPMI, and the flow chamber then assembled.

For experiments not involving the collection and phenotyping of cells post-flow, Vena8 biochips (Cellix Ltd, Dublin, Ireland) were used to generate laminar flow. The volumetric flow path for a channel of the Vena8 biochips measured 20000 × 400 × 100 microns (length × width × height). Protein coating of these chips is described in the appropriate section below.

*Experiments using Parallel Plates*

The parallel-plate flow chamber was mounted on the stage of a phase contrast optical microscope (CK40, Olympus, Japan) with a 10× objective. All runs were performed at room temperature. A uniform laminar flow field was simulated by use of a syringe pump (KD Scientific, New Hope, PA) capable of generating near-constant volumetric flow rates. The components of the configuration were devised to minimize tubing. Prior to infusing cell suspensions through the plates, the system was washed with 5 ml of RPMI at a flow rate producing a wall shear stress of approximately 1 dyne/cm$^2$. Cell suspensions of interest were then passed through the chamber at a fixed flow rate and wall shear stress.

All experiments were viewed in real time, recorded at 15.2 frames per second using a DP 200 digital camera and software (DeltaPix, Maalov, Denmark), and analyzed using Image J software (NIH).

*Overnight culture*

When overnight culture was required, cells were centrifuged and resuspended in RPMI-1640 medium (GIBCO), supplemented with 15% AB serum (Gemini Bio-Products) to a final concentration of $5 × 10^6$ cells/ml. Cells were cultured overnight for 18 hours in 12-well polystyrene tissue culture plates (2 ml per well) at 37° C in 5% CO2.

*Immunophenotyping*

Monoclonal antibodies for immunophenotyping included CD14 (LPS receptor; monocytes), CD11c (integrin subunit; monocytes and DC), HLA- DR (class II MHC molecule), CD83 (DC marker), CD62p (P-selectin; activated platelets), and CD61 (integrin subunit; platelets). Antibodies were obtained from Beckman Coulter (CD14, CD11c, HLADR, CD83) or Sigma (CD62p, CD61) and used at their pre-determined optimal dilutions. Background staining was established with appropriate isotype controls, and immunofluorescence was analyzed using a FC500 flow cytometer (Beckman Coulter). Two-color membrane staining was performed by adding the pre-determined optimal concentrations of both antibodies directly conjugated to FITC or PE and incubating for 20 min at 4°C, followed by washing to remove unbound antibodies. Combined membrane and cytoplasmic staining was performed following manufacturer's instructions for cell fixation and permeabilization (Intraprep kit, Beckman Coulter).

*Quantitative real-time PCR*

Gene expression was compared between cells exposed during flow through the parallel plates to low (10 ± 5/low power field [lpf]) versus high (102 ± 32/lpf) levels of platelets, followed by overnight culture. Cell RNA was isolated using RNeasy Mini Kit columns with on-column DNase I treatment (QIAGEN). RNA yield and purity were measured using a NanoDrop ND-1000 Spectrophotometer and an Agilent 2100 Bioanalyzer. RNA was reverse transcribed to cDNA using the High Capacity cDNA Reverse Transcription Kit (Applied Biosystems). Reverse transcription was carried out in a 96-well thermocycler (MJ Research PTC-200) in the following conditions: 25°C, 10 minutes, 37°C, 120 minutes, 85°C, 5 seconds. TaqMan real-time PCR was used to detect transcripts of DC-LAMP, CD40, ADAM Decysin, Lox1, CCR7, CD80, CD83, CD86, FPRL2, and GPNMB. Primers and probes for each sequence were obtained as inventoried Taqman Gene Expression Assays (Applied Biosystems). HPRT1 was used as a reference gene.

*Co-cultures of Platelets with Monocytes*

Experiments involving co-cultures of monocytes with additional platelets were performed as described in the Overnight Culture section, with a few necessary modifications. Following Ficoll-Hypaque separation, mononuclear cells were resuspended in 30% AB serum/RMPI to a final concentration of $10 × 10^6$ cells/ml, of which 1 ml was allocated to each well of a 16- well plate. An additional 1ml of platelets (suspended in RPMI, at 2x the desired final concentration) or RPMI without platelets was then added to each well. To activate platelets, 500 $\mu$l containing 2 units of thrombin was added to half the wells, and 500 $\mu$l of RPMI was added to the others to balance the volume. Cells were then incubated as described previously.

*Platelet Adhesion Studies*

Platelet adhesion experiments were performed using the Vena8 flow chamber described above. Fibrinogen and fibronectin (Sigma) were dissolved in PBS to a final concentration of 200 mcg/ml. Channels of the Vena8 chips were incubated at room temperature in a humidified chamber for 2 hours with the protein solution, autologous plasma, or PBS alone. The channels were washed with 5x the volume RPMI. Platelet-rich-plasma was then perfused through the protein-coated channel at the indicated shear-stress, held constant. For each channel, still images were acquired exactly 90 seconds into the experiment at 4 pre-defined low power fields located along the flow path (fields were centered at 2500, 7500, 12500, and 17500 microns from the start point of infusion).

Some experiments involved pre-treating platelet-rich-plasma with protein fragments prior to infusion through the channels. Small RGD peptides, containing the amino-acid sequence Arg-Gly-Asp-Ser; DRG peptides, contain the amino-acid sequence Ser-Asp- Gly-Arg; or fragment 400-411 of fibrinogen, containing the amino-acid sequence His-His-Leu-Gly-Gly-Ala-Lys-Gln-Ala-Gly-Asp-Val, were incubated at a concentration of 2mM with PRP for 20 minutes at room temperature. The PRP was then perfused through the channels as previously described.

*Receptor-Ligand Studies*

Platelet-coated Vena8 channels were pre-treated with either 40 $\mu$g/ml anti-P-selectin (R&D Systems) or 40 $\mu$g/ml of an isotype control for 30 minutes at room temperature, then washed with 5x the volume RPMI. Mononuclear cell suspensions were pre-treated with either RGD or DGR peptides at a concentration of 2.5 mM. Video samples lasting 400 frames (26.3 seconds) were recorded 60 seconds after commencement of flow using a lower power field of view spanning 400 microns and centered at 7500 microns from the flow start point.

$\beta$-1 integrin conformation was assessed using the Glycotech flow chamber. 15mm platelet-coated petri dishes (described above) were pre-treated with 40 $\mu$g/ml anti-P-selectin or an isotype control for 20 minutes at room temperature, then washed with 5x the volume RPMI. Immediately following perfusion through the platelets, cells were immunophenotyped with anti-CD29 HUTS-21 (BD Biosciences), an antibody that specifically binds to the active (open) conformation of $\beta$1 integrins.

**Results**

*Monocytes in flow transiently interact with immobilized platelets*

ECP was initially developed as a means to enable extracorporeal chemotherapeutic exposure of pathogenic leukocytes to ultraviolet A (UVA)-activated 8-methoxypsoralen (8-MOP), a DNA-cross-linking drug. Therefore, ECP involves the flow of leukapheresed blood between large transparent plastic parallel-plates separated by 1 mm. To permit detailed analysis of the flow dynamics involved during ECP, independent of UVA/8-MOP exposure, the flow conditions of ECP were reproduced using miniature parallel plates with surface area of only 0.8 mm$^2$, separated by 100 microns. This model permitted visualization using digital microscopy. Studies using the model revealed the following sequence (determined by video analysis): initial adherence of platelets from the flow stream to the plate, followed by transient binding of passaged monocytes to the immobilized platelets.

*DC induction correlates with the number of monocyte-platelet interactions*

Based on the initial qualitative observations described above, platelets were hypothesized to induce monocyte-to-DC differentiation under conditions of flow. To test the influence of platelets on monocyte-to-DC differentiation, monocytes were passed between parallel plates pre-coated with autologous platelets at low (10 $\pm$ 5/low power field [lpf]), medium (44 $\pm$ 20/lpf), and high (102 $\pm$ 32/lpf) densities. Cells were passed through the plates at a flow rate producing a wall shear stress of 0.5 dyne/cm$^2$, analogous to the wall shear stress in post-capillary venules. The number of monocyte-platelet interactions per unit time increased in proportion to augmented density of platelets (determined by video analysis). An average of 52.3 + 15 monocyte-platelet interactions per lpf per second were observed with the high-density plate, dropping to 18.3 $\pm$ 14 and 3.4 $\pm$ 1 interactions per second with the medium and low-density plates, respectively (Figure 1a).

Following overnight incubation, a correlation was found between the percentage of cells which developed a DC phenotype and the frequency of monocyte-platelet physical interactions observed the previous day (Figure 1b). An increasing number of monocyte-platelet interactions correlated with increasing proportion of cells expressing markers consistent with DC differentiation, membrane HLA-DR and CD83. An average of 14.2% of monocytes exposed to the high-density platelet-coated plate were HLA-DR+/ CD83+ after overnight incubation, compared to 4.9% and 0.8% of monocytes exposed to plates coated with medium and low levels of platelets, respectively.

*Monocyte exposure to platelets results in changes in gene expression*

To supplement the described changes in monocyte phenotype observed following platelet exposure, RT-PCR was performed to assess for changes in gene expression. Monocytes were passed through parallel plates coated with high or low densities of platelets as described in the previous section. Following overnight incubation, RNA was extracted and RT-PCR performed to determine level of expression for 10 genes associated with DC (Figure 2). CD40, a costimulatory molecule with known expression on mature DC (Cella et al., 1996, see reference list), was found to be upregulated by over 567% in monocytes exposed to high densities of platelets relative to monocytes exposed to low levels. LAMP3, a marker specific to DC differentiation (de Saint-Vis at al., 1998, see reference list), was upregulated by 398%. CD80 is a costimulatory molecule known to be upregulated upon APC activation (Slavik et al., 1999, see reference list), upregulated by 220% in monocytes exposed to high levels of platelets. CCR7, a chemokine receptor known to play a role in DC migration to lymphoid organs, was upregulated by 376%. LOX1, CD83, CCR7, and ADAM Decysin, all genes associated with DC (Berger et al., 2010, see reference list), were also upregulated in the monocytes exposed to high levels of platelets. FPRL2, GPNMB, and CD86 were all downregulated in monocytes exposed to high levels of platelets. FPRL2 is a receptor that when activated is known to inhibit DC maturation (Kang et al., 2005, see reference list) GPNMB is a protein involved in decreasing cytokine production (Ripoll et al., 2007, see reference list); CD86 is a costimulatory molecule expressed by APCs.

*DC induction in the presence of platelets does not occur under static conditions*

Platelets could potentially influence monocytes through direct receptor-ligand interaction, or via cytokines and other secreted mediators. To determine whether the platelet induction of monocyte-to-DC differentiation requires flow dynamics, we tested the role of platelets under static conditions. Monocytes were co-cultured with low (<50,000/mm$^3$), medium (100-200,000/mm$^3$) and high (>400,000/mm$^3$) concentrations of platelets, with platelets in either an inactive or active state (induced by the addition of thrombin). After overnight incubation in static conditions (shear stress = 0), we found that neither activated nor non-activated platelets were capable of inducting DC differentiation of monocytes in the absence of flow (see Figure 3).

*Platelets suspended in flow bind to serum proteins adsorbed onto the plate*

Several proteins abundantly present in plasma, including fibronectin and fibrinogen, are well known adsorb onto glass and plastic surfaces; the contribution of adherent plasma proteins on platelet adhesion and activation was therefore assessed. Parallel plates were pre-coated either with fibrinogen, fibronectin, plasma, or saline. Unactivated platelets were then passed through at shear rates producing wall shear stresses ranging from 0.2 to 6.0 dyne/cm$^2$. The highest concentrations of platelets adhered to plates coated with fibrinogen (Figure 4). Adhesion to fibronectin-coated, plasma-coated, and uncoated plates was observed as well, but to a significantly lower extent ($p < 0.05$). In the absence of flow, platelet adherence was equivalent on all protein substrates.

Both fibrinogen and fibronectin contain segments with the amino acid sequence arginine(R)-gylcine(G)-aspartate(D), RGD. RGD segments are well-known to interact with many integrin receptors, particularly the I/A domain of beta subunits, which are exposed when the integrins are in the active conformation (Xiong et al., 2002, see references). In experiments using fibrinogen-coated plates, platelet adhesion was not significantly altered by pre- incubation of platelets with RGD peptides; however, adhesion was significantly decreased ($p < 0.05$) by pre-incubation of platelets with peptide fragments corresponding to amino acids 400-411 of fibrinogen, the gamma component of the protein (Figure 5 a). In experiments using fibronectin-coated plates, pre-incubating platelets with RGD peptides decreased adhesion significantly, while pre-incubating platelets with peptide fragments corresponding to amino acids 400-411 of fibrinogen had no effect, (Figure 5b). Interestingly, it should be noted that unlike the I/A domain of integrins, which is known to interact with RGD domains of proteins, the region of the integrin found to interact with the gamma component of fibrinogen is exposed in the integrin's inactive state (Weisel et al., 1992, see references). Therefore, this data suggests that unactivated platelets in flow bind to the gamma-component of fibrinogen-coated plates. The potential for platelets in the unactivated state to bind fibrinogen may explain the greater level of platelet adhesion seen on fibrinogen-coated plates explained in the previous paragraph.

*Platelets are activated by adhesion to the plate*

Platelets physiologically circulate in an inactive state, with an array of proteins stored in intracellular granules. Upon encountering stimuli such as damaged endothelium or thrombin, platelets become activated and almost instantaneously translocate these intracellular proteins to the plasma membrane (Kaplan et al., 1979, see references). It was postulated that platelet adhesion to the plastic plate/absorbed proteins caused platelet activation similar to that caused by well-known stimuli. To test this hypothesis, surface expression of P-selectin, a well-known marker of platelet activation, was assessed before and after adhesion. Prior to adhesion, $6 \pm 3\%$ of platelets were found to express P-selectin, with a mean fluorescence intensity (MFI) of $12.4 \pm 6.9$; following adhesion, P-selectin positivity increased to $64 \pm 13\%$ (MFI $98.2 \pm 14$). The positive control, platelets activated with thrombin, was $71 \pm 18\%$ P-selectin positive (MFI $108.3 \pm 23$). Expression of P-selectin was further assessed at 30, 60, and 90 minutes following platelet adhesion; P-selectin expression remained stable at all time points, with $72 \pm 11\%$ of platelets P-selectin positive 90 minutes after adhesion, indicating that platelets remain in an active state for the duration of the procedure. Similar trends were found in assessment of $\alpha$IIb-$\beta$3, a fibrinogen-binding integrin, with surface expression of this protein increasing from $4 \pm 3\%$ prior to adhesion, to $49 + 18\%$ post-adhesion.

*Monocytes interact with P-selectin and RGD-containing ligands expressed on activated platelets*

The monocyte-platelet interactions observed on video were divided into two categories: (1) short-acting, arbitrarily defined as contact occurring for less than 3 seconds (46 frames), and (2) long-acting, defined as contact longer than 3 seconds, including stable binding. Since it had been previously determined that the platelets in the ECP system were in an activated state, and that activated platelets express an array of proteins including P- selectin and RGD containing proteins (e.g. fibronectin, fibrinogen, and vitronectin), it was sought to determine the involvement, if any, of these proteins in either short or long- duration interactions. Plates were pre-coated with platelets, and four conditions tested: (1) platelets pre-treated with an irrelevant isotype control, and monocytes untreated (P+RGD+); (2) platelets pre-treated with an irrelevant isotype control, and monocytes pre-incubated with RGD peptides (P+RGD-); (3) platelets pre-treated with anti-P-selectin, and monocytes untreated (P-RGD+); (4) platelets pre-treated with anti- P-selectin, and monocytes pre-treated with RGD peptides (P-RGD-). It was assumed that pre-treating monocytes with RGD peptides should result in a decreased in the number of free RGD- recognizing receptors available to interact with RGD-containing proteins expressed by the platelets. Thus, the four conditions tested represent every permutation of potential interaction with two platelet ligands, P-selectin and RGD-containing-proteins. As shown by Figure 6, both short-acting and long-acting interactions were maximal when neither RGD nor P-selectin were blocked (P+RGD+); the level of interaction in all other conditions was expressed as a percentage of this maximum. Blocking with anti-P-selectin alone (P-RGD+) resulted in a decrease of both short and long monocyte- platelet interactions to almost zero ($p < 0.01$; Figure 6, also confirmed by video analysis). In contrast, blocking RGD alone (P+RGD-) did not significantly alter the number of short-duration interactions, but decreased the long-duration monocyte-platelet interactions by 44% ($p < 0.05$; Figure 6). Blocking both P-selectin and RGD simultaneously (P-RGD-) resulted in a pattern similar to that seen when only P-selectin was blocked, with both long and short duration interactions reduced to near zero. The most appropriate conclusions, based on the pattern of interactions observed in each of the four conditions, are as follows: (1) P-selectin is predominantly responsible for the short-duration interactions; (2) RGD-containing proteins expressed by the platelet are involved in long-duration interactions, but not short-duration interactions; (3) monocyte interaction with P-selectin must occur upstream of monocyte interaction with RGD-containing proteins expressed by platelets. This last conclusion is based on the observation that conditions of P-RGD+ decreased both short and long duration interactions to near zero, while P+RGD-conditions only decreased long-duration interactions. If the interactions were not sequential, conditions of P-RGD+ should have produced similar results to P+RGD+ in terms of long-duration interactions. Furthermore, the ordering of the interactions, i.e. that P-selectin acts upstream of RGD-interactions, is apparent by the finding that conditions of P+RGD- only influenced long duration interactions, while conditions of P-RGD+ produced similar results to those of P-RGD-.

*Monocyte exposure to P-selectin results in downstream monocyte integrin-activation*

Integrin receptors, in their open conformation, are known to interact with RGD-containing ligands (Ruoslathi et al., 1996, see references). Using an antibody that recognizes an epitope exposed only when the $\beta$1 integrin is in its open conformation, we assessed the conformation of monocyte integrins before and after flow through the model. Figure 7 shows that as the number of short-acting monocyte-platelet interactions increased, there was corresponding increase in the percentage of monocytes expressing integrins in their open conformation immediately post-flow. The black line shows that an average of 71% of monocytes which had received a high number of platelet-interactions (> 61+ 19/lpf $\times$ sec) expressed $\beta$1 in the active form, compared to 9% of monocytes which had received a low number of platelet interactions (< 5.1 + 2 /1pf $\times$ sec). These results were not significantly affected by pre-treating the adherent platelets with an irrelevant isotype control (gray line). In contrast, pre-treating platelets with anti-P-selectin reduced the monocyte-platelet interactions to near zero, and monocytes emerging from flow in these conditions (dashed line) displayed low levels of active $\beta$1 integrins, irrespective of the density of platelets to which they were exposed. It is noteworthy that all cell populations prior to passage through the plates demonstrated similar low levels of baseline integrin activation (<10%); therefore, differences seen in short-duration monocyte-platelet interactions were not the result of differences in integrin conformation preflow.

*Monocyte exposure to P-selectin is required for DC differentiation*

Given the dependence of monocyte-platelet interactions on platelet P-selectin, we set out to determine if there was a relationship between monocyte exposure to P-selectin at time 0, and the phenotype later developed by the monocyte after overnight incubation, time 18-hours (Figure 8). Monocytes were passed though parallel plates coated with high densities (108 ± 36/lpf) of platelets that were either untreated (unblocked), or pretreated with either anti-P-selectin or an isotype control. 15.5 ± 4 % of monocytes exposed to unblocked platelets became membrane HLA-DR+/CD83+ (markers of maturing DC) after overnight incubation, and 13 ± 4 % of the those exposed to platelets blocked with the irrelevant isotype control. In contrast, only 3 ± 2% of the monocytes exposed to platelets blocked with anti-P-selectin became HLA-DR+/CD83+ after overnight incubation.

**Experiment 2 - Identification of molecular markers for immuno-suppressive dendritic cells**

**Materials and Methods**

*Sample Collection and Monocyte Enrichment*

Peripheral blood specimens were acquired from healthy subjects under the guidelines of the Yale Human Investigational Review Board, and informed consent was provided according to the Declaration of Helsinki. PBMC were isolated by centrifugation over a Ficoll-Hypaque gradient (Isolymph, CTL Scientific). Monocytes were enriched from freshly isolated PBMC by: 1) plastic adherence for dexamethasone dose-titration experiments (purity: 71.6 ± 5.6% CD14+); 2) CD14 magnetic bead positive selection (Miltenyi Biotec) for PUVA dose-titration experiments (purity: 88.1 ± 3.5% CD14+), and; 3) Monocyte Isolation Kit II (Miltenyi Biotec) for LPS stimulation experiments (purity: 83.8 ± 3.8% CD14+).

*Generation of Monocyte-Derived DC (MoDC)*

Monocytes were cultured at a density of $5 \times 10^6$ cells/mL in 6- and 12-well polystyrene tissue culture plates at 37°C and 5% $CO_2$ in RPMI-1640 (Gibco) supplemented with heat-inactivated 15% AB serum (Gemini) and 1% penicillin/streptomycin (now referred to as complete media). 800 IU/mL recombinant human GM-CSF (R&D Systems) and 1000 IU/mL recombinant human IL-4 (R&D Systems) were added to cultures for 36 hr to induce monocyte to DC differentiation as described.

*8-MOP and UVA Light Treatment*

Cultures were incubated with 8-MOP (Uvadex, 20 μg/mL) for 30 min in the dark, and then irradiated with a desktop UVA light box containing a series of 12 linear fluorescent tubes. The tubes emitted UVA light ranging from 320 to 400 nm. The UVA irradiance (power, $W/m^2$) was measured using a photodiode. Given a measured irradiance and the absorption properties of the various components of the system, it was possible to determine the time (sec) needed to expose the cells to deliver a given dose of UVA radiation ($J/cm^2$).

*MoDCILymphocyte Co-Cultures*

Non-adherent cells (purity: 66.0 ± 4.5% CD3+) removed during plastic adherence will now be generally referred to as lymphocytes. Lymphocytes were treated with 8-MOP (100 ng/mL) and UVA (1 $J/cm^2$), washed with PBS, and co-cultured in complete media at 37°C and 5% $CO_2$ with either PUVA-treated or untreated-MoDC in a ratio of 5 or 10 lymphocytes to 1 MoDC. MoDC treated for 24 hr with 100 nM dexamethasone (Sigma) served as the positive control group. After 24 hr, cells were harvested and MoDC were re-purified. To ensure that RNA was not isolated in significant amounts from lymphocytes, it was critical to re-purify MoDC from all cultures using CD11c magnetic bead (Miltenyi Biotec) positive selection (purity: 96.4 ± 1.0% CD11c+). CD11c+ MoDC were re-plated at 0.5-1.0 $\times$ 10^6 cells/mL in complete media and stimulated with 100 ng/mL LPS (Sigma). 24 hr after LPS stimulation, cells were harvested for RNA isolation and immunophenotyping, and supernatants were collected for cytokine quantification. As negative controls, parallel groups did not receive LPS.

*siRNA Experiments*

Silencer select pre-designed and validated GILZ siRNA (Invitrogen), with off-target prediction algorithms, was used to knockdown GILZ expression. Mo-DC were transfected using Lipofectamine RNAiMAX Reagent (Invitrogen). $RNA_i$ duplex and lipofectamine reagent were incubated together for 20 min, then added to MoDC cultures and incubated for 2 hr at 37°C and 5% $CO_2$. Transfected MoDC were treated in an identical fashion as described for the MoDC/lymphocyte co-cultures. MoDC were also transfected with scramble siRNA.

*Immunophenotyping*

Monoclonal antibodies included HLA-DR, CD80, CD83, CD3, CD86, CD14, CD11c and GILZ. Antibodies were obtained from Beckman-Coulter and eBioscience and were used at their pre-determined optimal dilutions. Apoptosis was assessed using the Annexin-V Apoptosis Detection Kit (eBioscience), with Annexin-V recognizing phosphatidylserine (PS) on the surface of apoptotic cells. 7-AAD substituted for PI as the cell viability dye. Cells displaying an Annexin-V[+]/7-AAD[-] phenotype were classified as early apoptotic cells, and cells displaying an Annexin-V[+]/7-AAD[+] phenotype were classified as late apoptotic cells. Dual membrane and intracytoplasmic staining was performed using the IntraPrep fix and permeabilization kit (Beckman-Coulter). Background staining was established with appropriate isotype and fluorescence minus one controls. Immunofluorescence was analyzed using a FACSCalibur L (BD Biosciences) within 2 hr of fixation with 2% paraformaldehyde. A minimum of 10,000 events were collected for each group.

*Quantitative Real-Time PCR*

RNA was isolated from CD11c[+]MoDC using QIAShredder columns (QIAGEN) and the RNeasy Mini Kit (QIAGEN) with on-column Dnase I treatment (QIAGEN). RNA yield and purity were assessed using a NanoDrop ND-1000 spectrophotometer. cDNA was obtained using the High Capacity cDNA Reverse Transcription Kit (Applied Biosystems) in a 96-well thermocycler (MJ Research PTC-200). TaqMan real-time PCR was used to detect transcripts of GILZ, CD80, and CD86. Primers and probes were obtained as pre-designed and validated Taqman Gene Expression Assays (Applied Biosystems). SYBR green real-time PCR (Applied Biosystems) was used to detect transcripts of IL-12, IL-10, IL-6, TNF-alpha, and TGF-$\beta$. Primers were designed to span intron junctions using Primer3Plus. Primer melting curves were obtained to confirm a single product. HPRT-1 and GAPDH were used as reference genes. Samples were run in triplicate on a 7500 Real Time PCR System (Applied Biosystems). The delta-delta C(t) method was used to calculate the fold change.

*Cytokine Quantification*

Culture supernatants were analyzed in a multiplex format utilizing magnetic beads to IL-6, IL-8, IL-10, IL-12p70, IFN-$\gamma$, TNF-$\alpha$, RANTES, MCP-1, and MIP-1$\beta$ (BioRad Laboratories). For siRNA experiments, supernatants were analyzed with enzyme-linked immunosorbent assay (ELISA) kits for IL-10 (R&D Systems) and IL-12p70 (Enzo Life Science). All samples and standards were run in duplicate and analyzed using the LUMINEX 200 (LUMINEX), or the BioTek EL800 (BioTek).

*Statistical Analysis*

Student's t-tests were used for statistical comparisons between groups, with *p*-values < 0.05 considered statistically significant. Differential gene expression was considered statistically significant with a $\geq$ 2.5-fold change and a *p*-value < 0.05.

**Results**

*Expression of GILZ is rapidly down-regulated as monocytes differentiate into immature MoDC*

Freshly isolated CD14[+] monocytes express GILZ, but rapidly down-regulate GILZ by more than 99% as they differentiate into immature MoDC (Figure 10A). A reduction in GILZ mRNA was confirmed by a 61% decrease in GILZ protein levels (Figure 10B). GILZ down-regulation correlated with reduced expression of CD14 (monocyte-specific marker, see Zhou et al., references), and increased expression of cytoplasmic CD83, (immature MoDC marker, see Klein et al., references). Importantly, MoDC remained immature, expressing low membrane CD83 (mature DC marker, see Renzo et al., references, p = 0.16). MoDC up-regulate GILZ after treatment with dexamethasone (dex) in a dose-dependent manner (Figure 10C). Treatment with 100 nM dex for 24 hr was selected as the positive control for inducing GILZ expression in MoDC (Dex-DC) (Figure 10D).

8-MOP or UVA treatment alone did not effect GILZ expression (Figure 10E). However, when MoDC were treated with the combination of 8-MOP and UVA light (PUVA-DC), GILZ expression increased 5.5-fold. The induction of GILZ exhibited a slow time course, peaking 24 hr after treatment, and remaining significantly elevated for 72 hr (Figure 10F). In comparison, Dex-DC up-regulated GILZ as little as 2 hr after treatment.

*Immature MoDC treated with the combination of 8-MOP and UVA light up-regulate GILZ and assume a tolerogenic, immuno-suppressive phenotype*

It was next examined if there was a PUVA dose-dependent effect on GILZ expression. MoDC treated with 1 $J/cm^2$ UVA and 100 or 200 ng/mL 8-MOP up-regulated GILZ 2.9- and 4.4-fold respectively (Figure 11A). A similar dose-dependent phenomenon was observed with 2 $J/cm^2$, starting at an 8-MOP concentration of 50 ng/mL. Treatment with 0.5 $J/cm^2$ had no effect on GILZ expression until the 8-MOP concentration reached 200 ng/mL, and treatment with 4 $J/cm^2$ resulted in high levels of non-specific cell death (data not shown). The number of photo-adducts formed per $10^6$ base pairs is directly related to the product of the 8-MOP concentration and UVA dose, see Gasparro et al., references. As the product of 8-MOP and UVA reached 100, GILZ was up-regulated 3-fold, and as the product increased to 200 and 400, GILZ was up-regulated 4.8- and 8.6-fold respectively (Figure 11B).

The percentage of early apoptotic CD11c+ cells was minimally ($p > 0.05$) higher at 2 $J/cm^2$ as compared to 1 $J/cm^2$ for all doses of 8-MOP tested (Figure 11C). At 2 $J/cm^2$ and 200 ng/mL, there was an increase in the percentage of early apoptotic CD11c+ cells as compared to untreated MoDC (Figure 11C). The percentage of late apoptotic CD11c+ cells remained less than 13% at 1 $J/cm^2$, and less than 16% at 2 $J/cm^2$ for all doses of 8-MOP tested (Figure 11D). Moreover, dot plots highlight the relative resistance of MoDC to the pro-apoptotic effect of escalating doses of PUVA (Figure 11E). The number of cells recovered from cultures did not statistically differ in any group treated with 1 or 2 $J/cm^2$ (data not shown), and greater than 90% CD11c+ cells (range 91.0-97.5%) were harvested after treatment.

In contrast, lymphocytes display Annexin-V as early as 2 hr after treatment with 1 $J/cm^2$ and 100 ng/mL (data not shown). In contrast to MoDC treated with 100 ng/mL and 1 $J/cm^2$ (Figure 11F), 24 hr after treatment with the same dose of PUVA, the percentage of early apoptotic lymphocytes increased from 6.6% in untreated MoDC to 44.3% in PUVA-DC, and the percentage of late apoptotic lymphocytes increased from 4.5% to 33.7% (Figure 11G). Given that $64.3 \pm 3.2\%$ of lymphocytes were Annexin-V+ 24 hr after treatment, PUVA-treated lymphocytes are subsequently referred to as apoptotic lymphocytes (ApoL).

The PUVA dose-dependent induction of GILZ correlated with a decrease in cell surface expression of CD80, CD86, and CD83 (Figure 12A, 3B). Down-regulation of these markers paralleled the induction of GILZ (see Figure 11B), beginning at 8-MOP concentrations of 100 ng/mL for both 1 and 2 $J/cm^2$. As the product of 8-MOP and UVA exceeded 100, CD83, CD80 and CD86 expression were reduced by 31%, 30% and 54% respectively, and HLA-DR expression increased by 38%.

*MoDC exposed to apoptotic lymphocytes up-regulate GILZ and are resistant to LPS-induced full maturation*

To dissect the individual contributions of PUVA and exposure to apoptotic cells, MoDC were first co-cultured with varying ratios of ApoL. GILZ was up-regulated in an ApoL dose-dependent fashion (Figure 13A). When PUVA-DC were exposed to ApoL, GILZ was expressed at higher levels than in PUVA-DC cultured alone (Figure 13B). PUVA-DC exposed to ApoL also expressed GILZ at higher levels than in untreated MoDC exposed to ApoL (6.7-fold and 3.6-fold higher, respectively). There was a corresponding 1.5-fold increase in the GILZ protein level in all groups in which GILZ mRNA was up-regulated (Figure 13C). Induction of GILZ was not related to an increase in the number of early or late apoptotic CD11c+ cells, as there were < 12% early apoptotic (range 3.8-11.4%) and late apoptotic (range 6.3-11.5%) CD11c+ cells in all groups demonstrating up-regulation of GILZ.

MoDC expressing GILZ greater than 2.5-fold above untreated MoDC were resistant to full maturation by LPS and exhibited a semi-mature, tolerogenic phenotype. LPS stimulation increased CD80 expression in MoDC up-regulating GILZ to only 50% of the levels seen after LPS stimulation in untreated MoDC (Figure 13D, range 0.48-0.57%), and increased CD86 expression to only 45% of untreated MoDC (Figure 13D, range 0.42-0.47%). Similar results were obtained for HLA-DR and CD83 (Figure 14E, range 47-65% and 23-57% of untreated MoDC after LPS respectively). In addition, MoDC up-regulating GILZ expressed 6% of the CD80 mRNA of untreated MoDC (range 4.5-7.5%), and expressed 50% of the CD86 mRNA of untreated MoDC (range 12.4-85.1%), as assessed by qRT-PCR.

*MoDC expressing GILZ display a tolerogenic cytokine profile, and knockdown of GILZ reduces the IL-10 to IL-12p70 ratio*

Supernatants were harvested from co-cultures as described in Figure 13B. Dex-DC up-regulated GILZ 4.29-fold (see Figure 13B), increased production of IL-10 (Figure 14A), and decreased production of all pro-inflammatory cytokines (Figure 14B, 14C) and chemokines (Figure 14D, 14E) tested. In comparison, PUVA-DC up-regulated GILZ 2.78-fold (see Figure 13B), increased production of II,-10, and decreased production of all pro-inflammatory cytokines and chemokines tested, except TNF-$\alpha$ and IFN-$\gamma$. PUVA-DC or untreated MoDC, exposed to ApoL expressed GILZ at higher levels that PUVA-DC cultured alone (3.6- and 6.7-fold higher, respectively; see Figure 13B). These two groups increased production of IL-10, and decreased production of all pro-inflammatory cytokines and chemokines tested. Cytokine levels were confirmed at the RNA level, with MoDC that up-regulated GILZ also demonstrating up-regulation of IL-10 mRNA 8-fold above untreated MoDC (range 5.5-11.8, $p < 0.01$). Reductions in IL-12, TNF-$\alpha$, and IL-6 were also confirmed at the RNA level (data not shown). TGF-$\beta$ was up-regulated 2.5-fold in MoDC up-regulating GILZ (data not shown). TGF-$\beta$ was not included in the multiplex analysis and therefore was only analyzed at the mRNA level.

The IL-10 to IL-12p70 ratio is a useful indicator of tolerogenicity, since tolerogenic DC are characterized by an increased IL-10 to IL-12p70 ratio, see Steinman et al., references). The ratio of IL-10 to IL-12p70 increased from 6.7 in untreated MoDC to 67.7 in Dex-DC. Similarly, the IL-10 to IL-12p70 ratio increased to 38.7 in PUVA-DC, and to 89.4 and 114.9 in untreated MoDC and PUVA-DC exposed to ApoL, respectively ($p < 0.05$).

To assess whether induction of GILZ was mediating the tolerogenic cytokine profile, MoDC were transfected with siRNA to knockdown GILZ expression. Transfection with GILZ siRNA reduced GILZ expression in Mo-DC by 68% (Figure 15A, range 59-79%). Transfection with scramble siRNA did not significantly change GILZ expression. There was also no significant difference in the number of cells recovered from any groups transfected with siRNA as compared to non-transfected groups (data not shown).

Treated MoDC up-regulating GILZ 2.5-fold higher than untreated MoDC produced higher levels of IL-10 (Figure 15B), and knockdown of GILZ reduced IL-10 production by 39% (range 34-48%, $p < 0.05$). Treated MoDC up-regulating GILZ 2.5-fold higher than untreated MoDC also produced lower amounts of IL-12p70 (Figure 15C), and knockdown of GILZ increased IL-12p70 production by 188% (range 149-214%, $p < 0.05$). Treatment with scramble siRNA had no appreciable effect on the production of IL-10 or IL-12p70. Knockdown of GILZ reduced the IL-10 to IL-12p70 ratio that had been elevated after GILZ induction. Dex-DC treated with GILZ siRNA demonstrated a reduction in the IL-10 to IL-12p70 ratio from 15.3 in non-transfected MoDC to 3.9 in transfected Dex-DC. In PUVA-DC the ratio decreased from 8.4 in non-transfected MoDC to 2.9 in PUVA-DC, and in untreated MoDC and PUVA-DC exposed to ApoL, reductions in the ratio from 18.1 to 7.8 and 28.4 to 8.3, respectively, were observed.

These results demonstrates that like other immunosuppressive mediators, PUVA induces the expression of GILZ and generates tolerogenic immuno-suppressive dendritic cells, characterized by low expression of the co-stimulatory molecules CD80 and CD86, and the maturation marker CD83. GILZ induction is necessary for the polarization towards a tolerogenic cytokine profile, characterized by increased IL-10 production, and decreased pro-inflammatory cytokine and chemokine production, including IL-12p70. These results further implicate GILZ as the molecular switch mediating the immunosuppressive effects of apoptotic cells.

## Experiment 3 - Identification of further molecular markers for immuno-stimulatory dendritic cells

### Materials and Methods

#### Patient samples

Leukocytes from patients undergoing ECP using the UVAR XTS Photopheresis System (Therakos) were obtained under the guidelines of the Yale Human Investigational Review Board. Informed consent was provided according to the Declaration of Helsinki. Aliquots were procured at 3 time points: before treatment (Pre ECP), immediately after 8-MOP/ultraviolet A (UVA) exposure (ECP Day 0) or after 18-hour incubation of treated blood mononuclear leukocytes (ECP Day 1) in a 1-L platelet storage bag (PL-2410; Baxter).

#### Normal subjects

To determine whether ECP induces monocytes from healthy subjects to convert to DC, mononuclear leukocytes from normal subjects were examined in 2 ways. Leukapheresed leukocytes from normal subjects (N = 3) were studied pretreatment (pre-ECP), immediately after ECP (ECP Day 0), and 18 hours after ECP (ECP Day 1). A desktop apparatus, incorporating a UVA light source and a plastic exposure plate, enabled laboratory reproduction of the clinical ECP system and sample access for parallel RNA isolation, immunophenotyping, and functional studies. Alternatively, a unit of blood from normal subjects was drawn into a transfer bag and passed through the ECP treatment apparatus in an identical fashion to that of treated patients (N = 3). The cells obtained from the unit of normal blood were used for microarrays and antigen presentation assays.

*Psoralen addition*

As is routinely done during ECP, the standard 8-MOP concentrated solution (Therakos) was added directly to the clinical ECP apparatus and to the laboratory model system. That mode of introduction enabled precise 100-200 ng/mL concentrations throughout the clinical procedures and experimentation.

*Overnight culture*

In ECP, it is not possible to examine phenotypic and functional changes in treated monocytes, because those cells are immediately reinfused into patients. Therefore, after ECP, cells were cultured for 18 hours (RPMI 1640/15% autologous serum) to study induced monocyte gene activation, maturation and function. Prior to (pre-ECP) and immediately after ECP (ECP Day 0), patient and normal subject samples were isolated by centrifugation over a Ficoll-Hypaque gradient. The cells were resuspended in RPMI-1640 medium (Gibco), supplemented with 7.5% AB serum, 7.5% autologous serum (Gemini Bio-Products) and cultured (for patients) in 6-well polystyrene tissue-culture plates at a density of $5* 10^6$ cells/mL and in Baxter platelet storage bags (for normal subjects 37°C, 5% $CO_2$). After overnight culture (ECP Day 1), cells were harvested before undergoing monocyte enrichment. To generate DC for comparative phenotypic analysis, cells were cultured in RPMI 1640 15% serum in the presence of 1 mL of GMCSF and IL4 (25 ng/mL; R&D Systems) for 6 days.

*Magnetic bead enrichment of the monocyte population*

To enable determination of whether ECP activates genes directing monocytes into the dendritic cell maturational pathway, it was necessary to develop a gentle negative monocyte enrichment method that eliminates contribution of lymphocytes to the transcriptome analysis while minimizing monocyte physical or cell membrane perturbation. Monocytes were enriched from the mononuclear cell pool by single passage through affinity columns. This negative selection method limited physical perturbation, whereas lymphocytes adherent to magnetic microbeads (Miltenyi Biotec), conjugated to relevant monoclonal antibodies (anti-CD4, CD8, CD19), were depleted. However, enrichment of ECP Day 1 monocytes beyond 60%-80% proved challenging, because diminished surface display of lymphocyte markers by ECP-damaged lymphocytes permitted a fraction of T and B cells to escape retention in the columns. Repetitive passes through the affinity column, to further enhance monocyte purity, was not an option because that approach compounds the physical perturbation of passively filtered monocytes. Fortuitously, a series of analyses revealed that ECP's preferential damage of lymphocytes precluded the necessity of full purification of monocytes for accurate assessment of level of DC gene activation. Due to their extreme sensitivity to UVA-activated 8-MOP, 99% of ECP-processed lymphocytes were apoptotic after overnight incubation (as determined by staining with APO2-PE, Trypan blue, and/or annexin-fluorescein isothiocynate FITC/ propidium iodide). Because ECP causes global lymphocyte apoptosis, 90%-95% of viable mononuclear leukocytes in the ECP day 1 fraction were monocytes. This phenomenon accounts for the observation that multiple step magnetic bead removal of apoptotic lymphocytes, performed as follows and yielding monocyte purity of greater than 95%, does not alter levels of observed gene expression in the studied cell populations. To accomplish that comparison we modified the monocyte purification procedure by adapting a negative selection protocol using magnetic beads and the EasySep magnet. Peripheral blood mononuclear cells were centrifuged at low speed (120g for 10 minutes) to remove platelets. Cells were then labeled using the Monocyte Isolation Kit II (Miltenyi Biotec) following the manufacturers procedure with the follow- ing modifications: (1) buffer consisted of ice-cold phosphate-buffered saline containing 2% autologous serum and 1mM EDTA (ethylenediaminetetraacetic acid); (2) blocking time was increased to 10 minutes; (3) labeling with the Biotin-Antibody Cocktail was increased to 20 minutes; and (4) cells were washed once between labeling with the Biotin-Antibody Cocktail and the Anti-Biotin Microbeads. To avoid stimulating the monocytes by passing them over a column, the magnetically labeled cells were instead separated from the unlabeled monocytes using the EasySep magnet (StemCell Technologies). Cells, in 2 mL of buffer in a 5-mL polystyrene tube, were placed in the magnet for 10 minutes, and then the unlabeled cells were carefully poured off into a new tube. This procedure was repeated 2x, to maximally enhance monocyte purity. At this point, because the purity was still insufficient, cells were relabeled with the Monocyte Isolation Kit II reagents and placed in the EasySep magnet for an additional 10 minutes, and the unlabeled monocytes were eluted. Final purity (X=96%+4.5) was assessed by flow cytometric analysis of CD14 staining.

*Immunophenotyping*

Monoclonal antibodies specific for monocytes and dendritic cells, included: CD14 (lipopolysaccharide [LPS] receptor, monocytes); CD36 (receptor for apoptotic cells, monocytes); human leukocyte antigen DR-1 (HLA-DR; class II major histocompatibility complex [MHC] molecule); CD83 (dendritic cell marker); cytoplasmic dendritic cell-lysosome-associated membrane protein (DC-LAMP; dendritic cell marker); and CD80 and CD86 (B7.1 and B7.2 costimulatory molecules). Antibodies were obtained from Beckman Coulter and used at their predetermined optimal dilutions. Background staining was established with appropriate isotype controls, and immunofluorescence was analyzed using a FC500 flow cytometer (Beckman Coulter). Combined membrane and cytoplasmic staining was performed following manufacturer's instruc- tions for cell fixation and permeabilization (Intraprep kit; Beckman Coulter).

*Antigen presentation assay*

Volunteer freshly isolated, magnetic bead-enriched, antigen-experienced CD4$^+$ populations ($2*10^6$/mL, 50 $\mu$L/well) were added to monocytes ($2*10^6$/mL, 50 $\mu$L/well) in the presence of tetanus toxoid (10 $\mu$g/mL, 100 $\mu$L/well) and RPMI medium 1640/15% autologous serum. After 5 days of culture, the cells received 1 $\mu$Ci of [$^3$H]-thymidine and were incubated overnight, harvested, and counted in a Beta liquid scintillation counter (PerkinElmer). Results are presented as the mean and standard deviation of 5 replicate cultures.

*MLR/CML assay*

To assess whether ECP-processed monocytes are functionally capable of stimulating MHC class I-restricted cytotoxicity by CD8 T cells, mononuclear leukocytes from 3 normal subjects were studied. One unit of anti-coagulated blood, freshly procured from each of 3 HLA-A2-positive volunteers, served as sources of stimulator monocyte/dendritic cells, before and after being processed through the clinical ECP apparatus in a manner identical to the actual ECP procedure. Mononuclear fractions were isolated from the blood immediately prior to ECP processing (pre-ECP) and immediately after ECP (ECP D0). After gamma irradiation (3000 rad, Cesium source) to ensure unidirectional T-cell stimulation, the Pre ECP fraction was serially diluted in RPMI 1640/15% autologous serum, and 100 $\mu$L containing from 25 000 to 250 cells was plated in round-bottom microtiter plate wells, in 5 replicates. The ECP D0 fraction was incubated for 18 hours in large well plates and harvested by scraping the wells to free adherent cells. The re-suspended cells were then serially diluted and plated as above. An A-2-negative normal donor served as the source of responder CD4 and CD8 T cells, purified by positive selection on Miltenyi magnetic bead columns (average purity 98%). Responder T cells (50 000/well in 100 $\mu$L) were then added to the wells containing either Pre-ECP or ECP-D0 stimulators, and the plates were cultured for 7 days at 37°C in a CO2 incubator. For target cells, the A-2-positive T-B hybridoma lymphoblast line, 174 x CWM.T1, was labeled with $^{51}$Cr and added to the MLR cultures at $10^4$ cells/well. After 4-hour incubation, plates were centrifuged, and 100 $\mu$L of supernatant was removed from each well for counting in a gamma counter. "Percent-specific lysis" was defined as 100 times the following fraction:

$$\text{Mean cpm (sample) - Mean cpm (T cell only)}$$

$$\text{Mean cpm (detergent maximum release) - mean cpm (T cells only)}$$

*RNA isolation and microarray hybridization*

Total RNA was isolated using RNeasy Mini Kit columns with on-column DNase I treatment (QIAGEN). RNA yield and purity were measured using the NanoDrop ND-1000 Spectrophotometer and the Agilent 2100 Bioana- lyzer. Fragmented cRNAs were hybridized on Affymetrix HG U133 Plus 2.0 human chips, and screening for approximately 47 400 human genes and ESTs was performed by the Yale University W. M. Keck Resource Laboratory. The microarray results are available on Gene Expression Omnibus under accession number GSE23604.

*Data analysis*

Raw data without normalization generated from Affymetrix GeneChip Operating Software Version 1.2 (GCOS 1.2; Affymetrix) were analyzed using GeneSpring software 7.2 (Agilent Technologies-Silicon Genetics). Data were normalized using Robust Multi-Array. Only probe sets with a minimal fold change of >2.0 combined with an average signal intensity of 500 or higher in either leukapheresis or treated samples were included in the analysis. Differential gene expression was considered as a ≥2-fold change and P ≤ .05. Principal component analysis (PCA) of the induced transcriptomes was performed by standard methodology. Signal transduction pathway involvement was identified with MetaCore Software Version 1.0 (GeneGo).

*Quantitative real-time PCR*

Microarray expression of selected genes was confirmed in aliquots of the same RNA samples, using quantitative real-time polymerase chain reaction (PCR). RNA was reverse transcribed to cDNA using the High Capacity cDNA Reverse Transcription Kit (Applied Biosystems). Reverse transcription was carried out in a 96-well thermocycler (MJ Research PTC-200) in the following conditions: 25°C, 10 minutes, 37°C, 120 minutes, 85°C, 5 seconds. TaqMan real-time PCR was used to detect transcripts of DC-LAMP, CCR7, CD80, CD86, and CD14. Primers and probes for each sequence were obtained as inventoried Taqman Gene Expression Assays (Applied Biosystems). HPRT1 was used as a reference gene.

**Results**

*Large ECP-induced changes in individual gene expressions*

The stimulation by ECP of individual gene activation in monocytes was expressed as the ratio of ECP Day 1 to pre-ECP expression for the relevant gene. To preclude inadvertent gene induction during monocyte enrichment, a negative column purification method was used, whereby lymphocytes were retained, and monocytes were passively filtered. The results revealed that the ECP-processed monocytes from both patients and normal subjects remain sufficiently viable to reproducibly express a shared transcriptome signature.

Genes were considered significantly up- or down-regulated by ECP if fold change was ≥2 and significance was $P \leq .05$ compared with pre ECP. Levels of RNA transcripts from approximately 3000 genes were significantly changed in each patient group and in normal subjects (Table 2). Overall, 1129 genes were up- or down-regulated in common by ECP-processed monocytes from both CTCL and GVHD patients and from normal subjects, indicating commonality in ECP-induced gene activation.

Table 2: Number of Monocyte Genes with Altered Expression after ECP.

| Monocyte Source | Total | Up-regulated | Down-regulated |
|---|---|---|---|
| Normal Subjects (alone): N=6 | 3,666 | 1494 (41%) | 2172 (59%) |
| CTCL (alone): N=3 | 4,315 | 2613 (61%) | 1702 (38%) |
| GVHD (alone): N=3 | 4,350 | 2658 (61%) | 1692 (39%) |
| Number of genes significantly induced or suppressed by ECP. | | | |

Increased expression of numerous genes associated with dendritic cell differentiation, adhesion, and function (Table 3) further support ECP stimulation of entry of monocytes into that pathway.

Table 3: : ECP-Enhanced Expression of DC Marker Genes, Ratio* of Post-ECP/Pre-ECP Levels

| Gene | Attributes | CTCL and GVHD (N-6) Induced Expression Ratio | Normal Subjects (N-6) Induced Expression Ratio |
|---|---|---|---|
| DC-LAMP | DC Lysomal Protein | 27.6 $p=1.2\times10^{-09}$ | 17.2 $p=1.4\times10^{-07}$ |
| GPNMB | Transmembrane glycoprotein | 205.7 $p=9.6\times10^{-15}$ | 123.3 $p=2.8\times10^{-14}$ |
| CD80 | Co-stimulatory molecule, B7.1 | 13.4 $p=2.3\times10^{-13}$ | NC |
| CD86 | Co-stimulatory molecule, B7.2[8] | NC | 5.0 $p=1.4\times10^{-05}$ |
| CD40 | Involved in DC survival | 2.3 $p=5.7^{-04}$ | NC |
| Decysin | ADAM-like, Expressed in LPS matured DC | 26.5 $p=1.0\times10^{-09}$ | 7.1 $p=5.6\times10^{-04}$ |

(continued)

| Gene | Attributes | CTCL and GVHD (N-6) Induced Expression Ratio | Normal Subjects (N-6) Induced Expression Ratio |
|---|---|---|---|
| CCR7 | Lymph node homing molecule | 2.6<br>$p=7.0\times10^{-03}$ | NC |
| CD83 | DC maturation molecule | NC | 2.3<br>$p=0.03$ |
| OLR1 | Lox1, lectin-like receptor | 13.6<br>$p=3.3\times10^{-05}$ | 100.1<br>$p=8.3\times10^{-08}$ |
| CLEC5A | MDL-1 | 10.9<br>$p=9.5\times10^{-07}$ | 45.5<br>$p=1.6\times10^{-08}$ |
| FPRL2 | Formyl peptide receptor-like-2 | 33.9<br>$p=2.1\times10^{-08}$ | 43.2<br>$p=1.9\times10^{-08}$ |
| SDC2 | Syndecan, cell surface proteoglycan | 21.7<br>$p=9.3\times10^{-08}$ | 98.9<br>$p=3.3\times10^{-09}$ |
| THBS1 | Thrombospondin 1 | 6.2<br>$p=7.8\times10^{-08}$ | 10.4<br>$p=4.7\times10^{-09}$ |
| *Ratio = (Pre-ECP Gene Expression) to (Post-ECP Gene Expression), Fold increase in expression of multiple genes involved in DC maturation and function induced by ECP. Impact of treatment on gene expression is displayed as an *Induced Expression Ratio* (ratio of post-ECP to pre-ECP expression for the relevant gene). RNA was isolated from 3 CTCL patients and 3 GVHD patients and 6 normal subjects at the relevant time points. |||||

Further genes, the expression of which was found to be increased and which can be considered to be molecular markers of immune-stimulatory dendritic cells are depicted in Table 1.

As would be expected during monocyte-to-dendritic cell maturation, CD14 (monocyte marker) expression was diminished, as assessed by measuring the mean fluorescence intensity on the monocyte populations of all patients and normal subjects, after overnight culture of ECP-processed monocytes. This result was confirmed in RT-PCR studies of the patients' post-ECP cells (results not shown). Further factors, the expression of which was reduced indicating monocyte-to-dendritic cell maturation are shown in Table 4.

Table 4: ECP-Reduced Expression of Monocyte Marker Genes , Ratio* of Post-ECP/Pre-ECP Levels

| Gene | Attributes | CTCL and GVHD (N=6) Induced Expression Ratio | Normal Subjects (N=6) Induced Expression Ratio |
|---|---|---|---|
| CD33 | Cell surface protein expressed on monocytes | -2.2<br>$p=4.5\times10^{-04}$ | NC |
| CD36 | Receptor for apoptotic cells | -7.4<br>$p=7.9\times10^{-05}$ | NC |
| FCGR1A | Receptor for IgGFc fragment 1A | -6.9<br>$p=6.6\times10^{-05}$ | -4.4<br>$p=2.1\times10^{-03}$ |
| *Ratio = (Pre-ECP Gene Expression) to (Post-ECP Gene Expression), Fold decrease in expression of genes distinctive of monocytes induced by ECP, as the monocytes differentiate into DC. Impact of treatment on gene expression is displayed as an *Induced Expression Ratio* (ratio of post-ECP to pre-ECP expression for the relevant gene). RNA was isolated from 3 CTCL patients and 3 GVHD patients and 6 normal subjects at the relevant time points. |||||

Further factors, the expression of which was reduced and thus indicating monocyte-to-immuno suppressive dendritic cell maturation are shown in Table 5.

Table 5: ECP-Enhanced Expression of Immunosuppression-Associated Genes, Ratio* of Post-ECP/Pre-ECP Levels.

| Gene | Attributes Normal | CTCL and GVHD (N-6) Induced Expression Ratio | Normal Subjects (N-6) Induced Expression Ratio |
|---|---|---|---|
| IDO | Indoleamine | 27.8<br>$p=4.0\times10^{-10}$ | 9.4<br>$p=1.1\times10^{-06}$ |
| KMO | kynurenine 3-hydroxylase | 6.0<br>$p=2.5\times10^{-06}$ | NC |
| IL10 | Interleukin 10 | 6.3<br>$p=9.2\times10^{-06}$ | 8.6<br>$p=5.7\times10^{-06}$ |

*Ratio = (Pre-ECP Gene Expression) to (Post-ECP Gene Expression), ECP-induced fold increase in expression of genes which contribute to DC capacity suppress T cell-mediated immunologic reactions. Impact of treatment on gene expression is displayed as an *Induced Expression Ratio* (ratio of post-ECP to pre-ECP expression for the relevant gene). RNA was isolated from 3 CTCL patients and 3 GVHD patients and 6 normal subjects at the relevant time points.

**Experiment 4** - **Surface molecule markers and functional mediators of immuno-stimulatory DC.**

Further analysis of the ECP-induced dendritic cells transcriptome was performed to identify a subset of surface molecule gene products as markers and functional mediators of immuno-stimulatory dendritic cells. Of 466 genes up-regulated in ECP-induced dendritic cells were cross referenced to approximately 2000 known or presumed full-length human transmembrane genes to identify 87 shared surface proteins.

**Materials and Methods**

*Procurement of leukocytes and platelets*

All samples were acquired from young, healthy subjects not taking medications, including aspirin, known to influence platelet function. Samples were obtained under the guidelines of the Yale Human Investigational Review Board, and informed consent was provided according to the Declaration of Helsinki. Peripheral blood specimens were collected through a 19-gauge needle from the antecubital vein into syringes containing heparin, then layered on Ficoll-Hypaque (Gallard-Schlessinger, Carle Place, N.Y.). Following centrifugation at 180g, the interface containing the mononuclear leukocyte fraction was collected and washed twice in HBSS, then resuspended in RPMI-1640 medium (GIBCO) to a final concentration of $5 \times 10^6$ mononuclear cells/ml. Cells were utilized within one hour of being acquired.

*Preparation of Platelet-rich-Plasma*

Whole blood was centrifuged at 150 g for 15 min at room temperature. The platelet-rich-plasma (PRP) layer was collected and centrifuged at 900 g for 5 min, and the platelet pellet resuspended in RPMI 1640 to the desired concentration.

*Preparation of Plates*

Plate passage was conducted using a Glycotech system (Glycotech, Rockville, MD). This system consisted of a volumetric flow path measuring $20000 \times 10000 \times 254$ microns (length × width × height). The bottom plate in this system was composed of a 15mm petri dish (BD Biosciences, Durham, NC) separated by a gasket and vacuum-connected to an acrylic flow deck, which formed the upper plate. For precoating with platelets, prior to assembling the flow chamber, 20 drops of the desired concentration of PRP was placed in the center of the petri dish and platelets allowed to settle for 20 minutes at room temperature. The petri dish was washed twice with 2ml of RPMI, and the flow chamber then assembled.

*Overnight culture*

When overnight culture was required, cells were centrifuged and resuspended in RPMI-1640 medium (GIBCO), supplemented with 15% AB serum (Gemini Bio-Products) to a final concentration of $5 \times 106$ cells/ml. Cells were cultured

EP 3 741 843 B1

overnight for 18 hours in 12-well polystyrene tissue culture plates (2 ml per well) at 37° C in 5% CO2.

*Immunophenotyping*

Monoclonal antibodies for immunophenotyping included CD14 (LPS receptor; monocytes), CD11c (integrin subunit; monocytes and DC), HLA-DR (class II MHC molecule), CD83 (DC marker), CD62p (P-selectin; activated platelets), and CD61 (integrin subunit; platelets). Antibodies were obtained from Beckman Coulter (CD14, CD11c, HLADR, CD83) or Sigma (CD62p, CD61) and used at their pre-determined optimal dilutions. Background staining was established with appropriate isotype controls, and immunofluorescence was analyzed using a FC500 flow cytometer (Beckman Coulter). Two-color membrane staining was performed by adding the pre-determined optimal concentrations of both antibodies directly conjugated to FITC or PE and incubating for 20 min at 4°C, followed by washing to remove unbound antibodies. Combined membrane and cytoplasmic staining was performed following manufacturer's instructions for cell fixation and permeabilization (Intraprep kit, Beckman Coulter).

**Results**

Plate-passed and/or PBMC D1 populations showed significant upregulation of analyzed surface expression of SIRPa, ICAM1, CXCL16, LIGHT, PLAUR (CD87, plasminogen activator, urokinase receptor), MSR1, Neu1 (sialidase), CD137L, and CATB (CTSB, cathepsin B).

**Experiment 5** - **Determining expression of molecular markers and FSC/SSC complexity after passing monocytes through flow chamber**

**Materials and Methods**

Monocytes were passed through a device depicted in Fig. 19. In brief, a blood sample was spun at low speed through a Ficoll gradient to obtain e.g. 8 ml of sample with a concentration of peripheral blood mononuclear cells (PBMC) of e.g. $10^{10}$ cells/ml. The chamber was pre-coated with platelets. The sample was passed through the chamber at about 0.028 Pa. The chamber and then washed with about 3 ml RPMI at 0.028 Pa. A second wash with 30-55 ml RPMI was performed at about 1.2 Pa. The collected activated monocytes were combined, incubated for a day and used for further analysis (PP D1 PBMC). As a control PBMCs were not passed through the device and incubated for a day (D1 PBMC). As another control immature fast DC were obtained by directly cultivating PBMC in the presence of GM-CSF and IL-4 (immature Fast DC). Further, PBMC were analyzed directly after harvest through a Ficoll gradient (Fresh (Ficoll) PBMC).
The cells and controls were then analyzed for expression of HLA-DR, CD86, ICAM-1, and PLAUR. They were further analyzed for FSC/SSC complexity. The results are depicted for HLA-DR in Figure 20 and for FSC/SSC complexity in Figure 21 and 22. A summary is shown in Figure 23.

**Results**

The results show that cells subjected to centrifugation through a Ficoll gradient alreads seem to experience enough physical forces to start differentiating as becomes apparent from incubating these cells for one day (D1 PBMC). However, activation and differentiation is more pronounced upon plate passage through the device (PP D1 PDMC). The dendritic cells obtained by methods in accordance with the invention in the absence of e.g. 8-MOP and UV-A moreover have a more complex and distinct pattern than immature Fast DC obtained with cytokine cocktails.

References:

1. Berger C, Hoffmann K, Vasquez JG, Mane S, Lewis J, Filler R et al. Rapid generation of maturationally synchronized human dendritic cells: contribution to the clinical efficacy of extracorporeal photochemotherapy. Blood 2010; 116(23): 4838-4847.

2. Cella M, Scheidegger D, PalmerLehmann K, Lane P, Lanzavecchia A, Alber G. Ligation of CD40 on dendritic cells triggers production of high levels of interleukin-12 and enhances T cell stimulatory capacity: T-T help via APC activation. Journal of Experimental Medicine 1996; 184(2): 747-752.

3. de Saint-Vis B, Vincent J, Vandenabeele S, Vanbervliet B, Pin JJ, Ait-Yahia S et al. A novel lysosome-associated membrane glycoprotein, DC-LAMP, induced upon DC maturation, is transiently expressed in MHC class II compartment. Immunity 1998; 9(3): 325-336.

4. Slavik JM, Hutchcroft JE, Bierer BE. CD80 and CD86 are not equivalent in their ability to induce the tyrosine phosphorylation of CD28. Journal of Biological Chemistry 1999; 274(5): 3116-3124.

5. Kang HK, Lee HY, Kim MK, Park KS, Park YM, Kwak JY et al. The synthetic peptide Trp- Lys-Tyr-Met-Val-D-Met inhibits human monocyte-derived dendritic cell maturation via formyl peptide receptor and formyl peptide receptor-like 2. Journal of Immunology 2005; 175(2): 685-692.

6. Ripoll VM, Irvine KM, Ravasi T, Sweet MJ, Hume DA. Gpnmb is induced in macrophages by IFN-gamma and lipopolysaccharide and acts as a feedback regulator of proinflammatory responses. Journal of Immunology 2007; 178(10): 6557-6566.

7. Chen SQ, Springer TA. Selectin receptor-ligand bonds: Formation limited by shear rate and dissociation governed by the Bell model. Proceedings of the National Academy of Sciences of the United States of America 2001; 98(3): 950-955.

8. Thomas WE. Understanding the counterintuitive phenomenon of catch bonds. Current Nanoscience 2007; 3: 63-83.

9. Xiong JP, Stehle T, Zhang RG, Joachimiak A, Frech M, Goodman SL et al. Crystal structure of the extracellular segment of integrin alpha V beta 3 in complex with an Arg-Gly-Asp ligand. Science 2002; 296(5565): 151-155.

10. Weisel JW, Nagaswami C, Vilaire G, Bennett JS. Examination of the Platelet Membrane Glycoprotein-IIB-IIIA Complex and its Interaction with Fibrinogen and Other Ligands by Electron-Microscopy. Journal of Biological Chemistry 1992; 267(23): 16637-16643.

11. Kaplan KL, Broekman MJ, Chernoff A, Lesznik GR, Drillings M. Platelet alpha-granule proteins - studies on release and subcellular-localization. Blood 1979; 53(4): 604-618.

12. Ruoslahti E. RGD and other recognition sequences for integrins. Annual Review of Cell and Developmental Biology 1996; 12: 697-715.

13. Zhou LJ, Tedder TF. CD14+ blood monocytes can differentiate into functionally mature CD83+ dendritic cells. Proc. Natl. Acad. Sci. U.S.A. 1996;93(6):2588-2592.

14. Klein E. CD83 localization in a recycling compartment of immature human monocyte-derived dendritic cells. International Immunology. 2005;17(4):477-487.

15. Renzo MD, Rubegni P, Pasqui AL, et al. Extracorporeal photopheresis affects interleukin (IL)-10 and IL-12 production by monocytes in patients with chronic graft-versus-host disease. Br J Dermatol. 2005;153(1):59-65.

16. Gasparro FP, Bevilacqua PM, Goldminz D, et al. Repair of 8-MOP photoadducts in human lymphocyte. In DNA Damage and Repair in Human Tissues (edited by B. M. Sutherland and A.D. Woodhead). Plenum Press, New York.

17. Steinman RM, Hawiger D, Nussenzweig MC. Tolerogenic dendritic cells. Annu. Rev. Immunol. 2003;21:685-711.

**Claims**

1. A method for inducing differentiation of monocytes contained in an extracorporeal quantity of a mammalian subject's blood sample into immuno-stimulatory dendritic cells, said method comprising at least the steps of:

   a) subjecting said extracorporeal quantity of said mammalian subject's blood sample to a shear force by passing the sample through a flow chamber;
   b) wherein the method is performed in the absence of photoactivatable agents such as 8-MOP and UVA; and
   c) wherein differentiation of monocytes from said blood sample into immuno-stimulatory dendritic cells is identified by increased expression of at least one molecular marker HLA-DR, CD83, CD86, ICAM-1 or PLAUR when comparing the immuno-stimulatory autologous dendritic cells to the monocytes within the extracorporeal quantity of a mammalian subject's blood sample.

2. Method according to claim 1, wherein the method is performed in the absence of a molecular cocktail comprising cytokines.

3. Method according to claim 1 or 2, wherein the flow chamber allows for fixed or tunable adjustment of the flow rate.

4. Method according to any of claims 1 to 3, wherein the flow chamber allows for adjustment of at least one parameter selected from the group comprising temperature and light exposure.

5. Method according to any of claims 1 to 4,
   wherein said monocytes are activated and induced to differentiate into immuno-stimulatory dendritic cells through interaction with activated platelets and/or plasma components.

6. Method according to any of claims 1 to 5,
   wherein said method additionally comprises

   (i) before method step a), the step of passing platelets through the flow chamber, which may be comprised within said extracorporeal quantity of said mammalian subject's blood or which may be provided separate from said mammalian subject's blood sample comprising at least monocytes; or
   (ii) before method step a), the step of passing plasma components though the flow chamber, which may be comprised within said extracorporeal quantity of said mammalian subject's blood sample or which may be provided separate from said mammalian subject's blood sample; or
   (iii) before method step a), the steps of (i) and (ii).

7. Method according to any of claims 1 to 6,
   wherein activation of said monocytes and differentiation into immuno-stimulatory autologous dendritic cells can be influenced by the design and dimensions of the flow chamber, the flow rate at which the monocytes are passed through the flow chamber, the temperature at which the monocytes, platelets, platelets-derived factors and/or plasma components are passed through the flow chamber, the exposure of the monocytes to light, the order by which the monocytes, platelets, platelets-derived factors and/or plasma components are passed through the flow chamber, the density by which plasma components are coated to the surfaces of the flow chamber, the density by which platelets and/or platelets derived factors adhere to the surfaces and or to the plasma components of the flow chamber, and/or the density by which monocytes adhere to the platelets and/or platelets derived factors and or plasma components adhered to the surfaces of the flow chamber.

8. Method according to claims 1 or 7, wherein said extracorporeal quantity of said mammalian subject's blood sample has not been obtained by apheresis; wherein preferably:

   i) said extracorporeal quantity of said mammalian subject's blood sample is between about 10 ml to about 500 ml of extracorporeal whole blood of said mammalian subject; and/or
   ii) said extracorporeal quantity of said mammalian subject's blood sample is obtained by isolating leukocytes from about 10 ml to about 500 ml of extracorporeal whole blood of said mammalian subject; and/or
   iii) said extracorporeal quantity of said mammalian subject's blood sample is obtained by isolating buffy coats from about 10 ml to about 500 ml of extracorporeal whole blood of said mammalian subject; and/or
   iv) said extracorporeal quantity of said mammalian subject's blood sample does not comprise plasma components; and/or
   v) said extracorporeal quantity of said mammalian subject's blood sample does not comprise platelets, wherein preferably said platelets have been separated from said extracorporeal quantity of said mammalian subject's blood before said extracorporeal quantity of said mammalian subject's blood said is applied to said flow chamber.

9. Method of claims 1 or 8,
   wherein said extracorporeal quantity of said mammalian subject's blood has been obtained by apheresis, wherein preferably:

   i) said extracorporeal quantity of said mammalian subject's blood is obtained by isolating leukocytes by apheresis;

and/or

ii) said extracorporeal quantity of said mammalian subject's blood is obtained by isolating buffy coats by apheresis; and/or

iii) said extracorporeal quantity of said mammalian subject's blood does not comprise plasma components; and/or

iv) said extracorporeal quantity of said mammalian subject's blood does not comprise platelets, wherein preferably said platelets have been separated from said extracorporeal quantity of said mammalian subject's blood before said extracorporeal quantity of said mammalian subject's blood said is applied to said flow chamber.

10. Method according to any of claims 1 to 9, wherein said flow chamber has dimensions of about 1 μm to up to about 400 μm of height and of about 1 μm to up to about 400 μm of width;

wherein preferably said flow chamber has dimensions of about 5 μm to up to and including about 300 μm of height and of about 5 μm to up to and including about 300 μm of width;

wherein more preferably said flow chamber has dimensions of about 10 μm to up to and including about 250 μm of height and of about 10 μm to up to and including about 250 μm of width; wherein even more preferably said flow chamber has dimensions of about 50 μm to up to and including about 200 μm of height and of about 50 μm to up to and including about 200 μm of width; and wherein even more preferably said flow chamber has dimensions of about 50 μm to up to and including about 100 μm of height and of about 50 μm to up to and including about 100 μm of width.

11. Method of claim 10, wherein said flow chamber is configured to take up a volume of between about 1 ml to about 50 ml of said extracorporeal amount of said mammalian subject's blood sample.

12. Method of any of claims 1 to 11, wherein

i) the material of said flow chamber is not plastic; and wherein preferably said non-plastic material is glass; or

ii) the material of said flow chamber is plastic; wherein preferably said plastic material is selected from the group consisting of acrylics, polycarbonate, polyetherimide, polysulfone, polyphenylsulfone, styrenes, polyurethane, polyethylene, teflon or any other appropriate medical grade plastic.

13. Method of any of claims 6 to 12, wherein activation of said platelets is achieved by disposing plasma components, which are comprised within said extracorporeal quantity of said mammalian subject's blood sample, on the surface of said flow chamber such that at least some of said platelets can interact with said plasma components and are immobilized on the surface of said flow chamber; wherein preferably:

i) activation of said platelets is achieved by disposing proteins selected from the group comprising fibrinogen, fibronectin, and the gamma component of fibrinogen on the surface of said flow chamber such that at least some of said platelets can interact with said proteins and are immobilized on the surface of said flow chamber; and wherein more preferably activation of said platelets is achieved by disposing fibrinogen on the surface of said flow chamber such that at least some of said platelets can interact with said fibronectin and are immobilized on the surface of said flow chamber; and/or

ii) said platelets are passed through said flow chamber under a shear force of 0.1 to 10.0 dynes/cm$^2$, preferably in a range of about 0.1 to about 2.0 dynes/cm$^2$; and/or

iii) activation of platelets can be monitored by expression of P-selectin and/or αIIb-β3 integrin.

14. Method of any of claims 1 to 13, wherein

i) said monocytes are passed through said flow chamber with a flow rate of about 10 ml/minute to about 200 ml/minute to produce a shear force of about 0.1 to about 20.0 dynes/cm$^2$; and/or

ii) said monocytes are activated and induced to differentiate into immuno-stimulatory autologous dendritic cells by passing said monocytes through said flow chamber under a shear force of about 0.1 to about 10.0 dynes/cm$^2$, preferably force of about 0.1 to about 1.0 dynes/cm$^2$ such that said monocytes can bind to said activated platelets.

15. Method of any of claims 1 to 14 for obtaining autologous immuno-stimulatory dendritic cells.

16. Method of any of claims 1 to 14 for obtaining allogenic immuno-stimulatory dendritic cells.

17. Method of any of claims 1 to 15 for obtaining individual-specific functionally and maturationally synchronized autologous immuno-stimulatory dendritic cells.

**Patentansprüche**

1. Verfahren zum Induzieren der Differenzierung von Monozyten, die in einer extrakorporalen Menge einer Blutprobe eines Säugetieres enthalten sind, in immunstimulierende dendritische Zellen, wobei das Verfahren mindestens die folgenden Schritte umfasst:

   a) Unterwerfen der extrakorporalen Menge der Blutprobe des Säugetiers einer Scherkraft, indem die Probe durch eine Strömungskammer geleitet wird;
   b) wobei das Verfahren in Abwesenheit von photoaktivierbaren Mitteln wie 8-MOP und UVA durchgeführt wird; und
   c) wobei die Differenzierung von Monozyten aus der Blutprobe in immunstimulierende dendritische Zellen durch eine erhöhte Expression von mindestens einem molekularen Marker HLA-DR, CD83, CD86, ICAM-1 oder PLAUR identifiziert wird, wenn die immunstimulierenden autologen dendritischen Zellen mit den Monozyten innerhalb der extrakorporalen Menge der Blutprobe eines Säugetieres verglichen werden.

2. Verfahren nach Anspruch 1, wobei das Verfahren in Abwesenheit eines molekularen Cocktails aus Zytokinen durchgeführt wird.

3. Verfahren nach Anspruch 1 oder 2, wobei die Strömungskammer eine feste oder verstellbare Einstellung der Strömungsgeschwindigkeit ermöglicht.

4. Verfahren nach einem der Ansprüche 1 bis 3, wobei die Strömungskammer die Einstellung mindestens eines Parameters aus der Gruppe Temperatur und Lichtexposition ermöglicht.

5. Verfahren nach einem der Ansprüche 1 bis 4,
   wobei die Monozyten durch Wechselwirkung mit aktivierten Blutplättchen und/oder Plasmabestandteilen aktiviert und zur Differenzierung in immunstimulierende dendritische Zellen veranlasst werden.

6. Verfahren nach einem der Ansprüche 1 bis 5,
   wobei das Verfahren zusätzlich umfasst

   (i) vor dem Verfahrensschritt a) den Schritt des Durchleitens von Blutplättchen durch die Strömungskammer, die in der extrakorporalen Menge des Blutes des Säugetieres enthalten sein können oder die getrennt von der Blutprobe des Säugetieres, die zumindest Monozyten enthält, bereitgestellt werden können; oder
   (ii) vor dem Verfahrensschritt a) den Schritt des Durchleitens von Plasmakomponenten durch die Strömungskammer, die in der extrakorporalen Menge der Blutprobe des Säugetiers enthalten sein können oder die getrennt von der Blutprobe des Säugetiers bereitgestellt werden können; oder
   (iii) vor dem Verfahrensschritt a) die Schritte (i) und (ii).

7. Verfahren nach einem der Ansprüche 1 bis 6,
   wobei die Aktivierung der Monozyten und die Differenzierung in immunstimulierende autologe dendritische Zellen beinflusst werden kann durch das Design und die Abmessungen der Strömungskammer, die Strömungsgeschwindigkeit, mit der die Monozyten durch die Strömungskammer geleitet werden, die Temperatur, bei der die Monozyten, Blutplättchen, von Blutplättchen abgeleiteten Faktoren und/oder Plasmakomponenten durch die Strömungskammer geleitet werden, die Exposition der Monozyten gegenüber Licht, die Reihenfolge, in der die Monozyten, Blutplättchen, Blutplättchen abgeleitete Faktoren und/oder Plasmakomponenten durch die Strömungskammer geleitet werden, die Dichte, mit der Plasmakomponenten auf die Oberflächen der Strömungskammer aufgebracht werden, die Dichte, mit der Blutplättchen und/oder von Blutplättchen abgeleitete Faktoren an den Oberflächen und/oder an den Plasmakomponenten der Strömungskammer haften, und/oder die Dichte, mit der Monozyten an den Blutplättchen und/oder von Blutplättchen abgeleiteten Faktoren haften und/oder Plasmakomponenten, die an den Oberflächen der Strömungskammer haften.

8. Verfahren nach Anspruch 1 oder 7, wobei die extrakorporale Menge der Blutprobe des Säugetiers nicht durch Apherese gewonnen wurde; wobei vorzugsweise:

i) die extrakorporale Menge der Blutprobe des Säugetiers zwischen etwa 10 ml und etwa 500 ml extrakorporales Vollblut des Säugetiers beträgt; und/oder

ii) die extrakorporale Menge der Blutprobe des Säugetiers durch Isolierung von Leukozyten aus etwa 10 ml bis etwa 500 ml extrakorporalem Vollblut des Säugetiers gewonnen wird; und/oder

iii) die extrakorporale Menge der Blutprobe des Säugetiers durch Isolierung von *Buffy Coats* aus etwa 10 ml bis etwa 500 ml extrakorporalem Vollblut des Säugetiers gewonnen wird; und/oder

iv) die extrakorporale Menge der Blutprobe des Säugetiers keine Plasmakomponenten enthält; und/oder

v) die extrakorporale Menge der Blutprobe des Säugetiers keine Blutplättchen enthält, wobei vorzugsweise die Blutplättchen von der extrakorporalen Menge des Blutes des Säugetiers abgetrennt wurden, bevor die extrakorporale Menge des Blutes des Säugetiers in die Strömungskammer eingebracht wird.

9. Verfahren nach Anspruch 1 oder 8,
   wobei die extrakorporale Menge des Blutes des Säugetiers durch Apherese gewonnen wurde, wobei vorzugsweise:

   i) die extrakorporale Menge des Blutes des Säugetiers durch Isolierung von Leukozyten durch Apherese gewonnen wird; und/oder

   ii) die extrakorporale Menge des Blutes des Säugetieres durch Isolierung von *Buffy Coats* durch Apherese gewonnen wird; und/oder

   iii) die extrakorporale Menge des Blutes des Säugetiers keine Plasmakomponenten enthält; und/oder

   iv) die extrakorporale Menge des Blutes des Säugetieres keine Blutplättchen enthält, wobei vorzugsweise die Blutplättchen von der extrakorporalen Menge des Blutes des Säugetieres abgetrennt wurden, bevor die extrakorporale Menge des Blutes des Säugetieres in die Strömungskammer eingebracht wird.

10. Verfahren nach einem der Ansprüche 1 bis 9, wobei die Strömungskammer Abmessungen von etwa 1 $\mu$m bis zu etwa 400 $\mu$m Höhe und von etwa 1 $\mu$m bis zu etwa 400 $\mu$m Breite aufweist;
    wobei die Strömungskammer vorzugsweise Abmessungen von etwa 5 $\mu$m bis zu und einschließlich etwa 300 $\mu$m Höhe und von etwa 5 $\mu$m bis zu und einschließlich etwa 300 $\mu$m Breite aufweist; wobei die Strömungskammer vorzugsweise Abmessungen von etwa 10 $\mu$m bis zu und einschließlich etwa 250 $\mu$m Höhe und von etwa 10 $\mu$m bis zu und einschließlich etwa 250 $\mu$m Breite aufweist; wobei die Strömungskammer noch bevorzugter Abmessungen von etwa 50 $\mu$m bis zu und einschließlich etwa 200 $\mu$m Höhe und von etwa 50 $\mu$m bis zu und einschließlich etwa 200 $\mu$m Breite aufweist; und wobei die Strömungskammer noch bevorzugter Abmessungen von etwa 50 $\mu$m bis zu und einschließlich etwa 100 $\mu$m Höhe und von etwa 50 $\mu$m bis zu und einschließlich etwa 100 $\mu$m Breite aufweist.

11. Verfahren nach Anspruch 10, wobei die Strömungskammer so konfiguriert ist, dass sie ein Volumen von etwa 1 ml bis etwa 50 ml der extrakorporalen Menge der Blutprobe des Säugetiers aufnimmt.

12. Verfahren nach einem der Ansprüche 1 bis 11, wobei

    i) das Material der Strömungskammer nicht aus Plastik ist; und wobei das nicht-Plastik Material vorzugsweise Glas ist; oder

    ii) das Material der Strömungskammer ist Plastik, wobei das Plastikmaterial vorzugsweise aus der Gruppe ausgewählt ist, die aus Acryl, Polycarbonat, Polyetherimid, Polysulfon, Polyphenylsulfon, Styrolen, Polyurethan, Polyethylen, Teflon oder einem anderen geeigneten Kunststoff medizinischer Qualität besteht.

13. Verfahren nach einem der Ansprüche 6 bis 12, wobei die Aktivierung der Blutplättchen dadurch erreicht wird, dass Plasmakomponenten, die in der extrakorporalen Menge der Blutprobe des Säugetiers enthalten sind, auf die Oberfläche der Strömungskammer aufgebracht werden, so dass zumindest einige der Blutplättchen mit den Plasmakomponenten interagieren können und auf der Oberfläche der Strömungskammer immobilisiert werden; wobei vorzugsweise:

    i) die Aktivierung der Blutplättchen durch Aufbringen von Proteinen, die aus der Gruppe ausgewählt sind, die Fibrinogen, Fibronektin und die Gammakomponente von Fibrinogen umfasst, auf die Oberfläche der Strömungskammer erreicht wird, so dass zumindest einige der Blutplättchen mit den Proteinen in Wechselwirkung treten können und auf der Oberfläche der Strömungskammer immobilisiert werden; und wobei die Aktivierung der Blutplättchen vorzugsweise durch Aufbringen von Fibrinogen auf die Oberfläche der Strömungskammer erreicht wird, so dass zumindest einige der Blutplättchen mit dem Fibronektin in Wechselwirkung treten können und auf der Oberfläche der Strömungskammer immobilisiert werden; und/oder

    ii) die Blutplättchen unter einer Scherkraft von 0,1 bis 10,0 dyn/cm$^2$, vorzugsweise in einem Bereich von etwa 0,1 bis etwa 2,0 dyn/cm$^2$, durch die Strömungskammer geleitet werden und/oder

iii) die Aktivierung der Blutplättchen durch die Expression von P-Selektin und/oder αIIb-β3-Integrin überwacht werden kann.

14. Verfahren nach einem der Ansprüche 1 bis 13, wobei

i) die Monozyten mit einer Strömungsgeshcwindigkeit von etwa 10 ml/Minute bis etwa 200 ml/Minute durch die Strömungskammer geleitet werden, um eine Scherkraft von etwa 0,1 bis etwa 20,0 dyn/cm$^2$ zu erzeugen; und/oder

ii) die Monozyten aktiviert und zur Differenzierung in immunstimulierende autologe dendritische Zellen veranlasst werden, indem die Monozyten unter einer Scherkraft von etwa 0,1 bis etwa 10,0 dyn/cm$^2$, vorzugsweise einer Kraft von etwa 0,1 bis etwa 1,0 dyn/cm$^2$ durch die Strömungskammer geleitet werden, so dass die Monozyten an die aktivierten Blutplättchen binden können.

15. Verfahren nach einem der Ansprüche 1 bis 14 zur Gewinnung autologer immunstimulierender dendritischer Zellen.

16. Verfahren nach einem der Ansprüche 1 bis 14 zur Gewinnung von allogenen immunstimulierenden dendritischen Zellen.

17. Verfahren nach einem der Ansprüche 1 bis 15 zur Gewinnung individualspezifischer, funktionell und reifungssynchronisierter autologer immunstimulatorischer dendritischer Zellen.

## Revendications

1. Procédé d'induction de la différenciation de monocytes contenus dans une quantité extracorporelle de l'échantillon sanguin d'un sujet mammifère en cellules dendritiques immunostimulatrices, ledit procédé comprenant au moins les étapes de :

   a) soumettre ladite quantité extracorporelle dudit échantillon sanguin de sujet mammifère à une force de cisaillement en passant l'échantillon à travers une chambre d'écoulement ;
   b) ledit procédé étant effectué en absence d'agents photoactivables tels que le 8-MOP et les UVA ; et
   c) la différenciation de monocytes dudit échantillon sanguin en cellules dendritiques immunostimulatrices étant identifiée par l'expression accrue d'au moins un marqueur moléculaire HLA-DR, CD83, CD86, ICAM-1 ou PLAUR lorsqu'on compare les cellules dendritiques autologues immunostimulatrices aux monocytes dans la quantité extracorporelle de l'échantillon sanguin d'un sujet mammifère.

2. Procédé selon la revendication 1, ledit procédé étant effectué en absence d'un cocktail moléculaire comprenant des cytokines.

3. Procédé selon la revendication 1 ou 2, dans lequel la chambre d'écoulement permet l'ajustement fixe ou accordable du débit.

4. Procédé selon l'une quelconque des revendications 1 à 3, dans lequel la chambre d'écoulement permet l'ajustement d'au moins un paramètre choisi dans le groupe comprenant la température et l'exposition à la lumière.

5. Procédé selon l'une quelconque des revendications 1 à 4,
   dans lequel lesdits monocytes sont activés et induits pour se différencier en cellules dendritiques immunostimulatrices par l'interaction avec les plaquettes activées et/ou des composants du plasma.

6. Procédé selon l'une quelconque des revendications 1 à 5,
   ledit procédé comprenant de plus

   (i) avant l'étape a) du procédé, l'étape de passage de plaquettes à travers la chambre d'écoulement, qui peuvent être comprises dans ladite quantité extracorporelle dudit sang de sujet mammifère ou qui peuvent être fournies séparément dudit échantillon de sang de sujet mammifère comprenant au moins des monocytes ; ou
   (ii) avant l'étape a) du procédé, l'étape de passage de composants du plasma à travers la chambre d'écoulement, qui peuvent être compris dans ladite quantité extracorporelle dudit échantillon de sang de sujet mammifère ou qui peuvent être fournis séparément dudit échantillon de sang de sujet mammifère ; ou

(iii) avant l'étape a) du procédé, les étapes de (i) et de (ii).

7. Procédé selon l'une quelconque des revendications 1 à 6, dans lequel l'activation desdits monocytes et la différenciation en cellules dendritiques autologues immunostimulatrices peuvent être influencées par la conception et les dimensions de la chambre d'écoulement, le débit auquel les monocytes sont passés à travers la chambre d'écoulement, la température à laquelle les monocytes, les plaquettes, les facteurs dérivés de plaquette et/ou les composants de plasma sont passés à travers la chambre d'écoulement, l'exposition des monocytes à la lumière, l'ordre par lequel les monocytes, les plaquettes, les facteurs dérivés de plaquette et/ou les composants de plasma sont passés à travers la chambre d'écoulement, la densité par laquelle les composants de plasma sont revêtus sur les surfaces de la chambre d'écoulement, la densité par laquelle les plaquettes et/ou les facteurs dérivés de plaquette adhèrent aux surfaces et ou aux composants de plasma de la chambre d'écoulement et/ou la densité par laquelle les monocytes adhèrent aux plaquettes et/ou aux facteurs dérivés de plaquette et ou les composants de plasma adhérés aux surfaces de la chambre d'écoulement.

8. Procédé selon les revendications 1 ou 7, dans lequel ladite quantité extracorporelle dudit échantillon sanguin de sujet mammifère n'a pas été obtenu par aphérèse ; dans lequel de préférence :

i) ladite quantité extracorporelle dudit échantillon sanguin de sujet mammifère est compris entre environ 10 ml à environ 500 ml de sang entier extracorporel dudit sujet mammifère ; et/ou

ii) ladite quantité extracorporelle dudit échantillon sanguin du sujet mammifère est obtenue en isolant les leucocytes d'environ 10 ml à environ 500 ml de sang entier extracorporel dudit sujet mammifère ; et/ou

iii) ladite quantité extracorporelle dudit échantillon sanguin de sujet mammifère est obtenue en isolant les revêtements buffy d'environ 10 ml à environ 500 ml de sang entier extracorporel dudit sujet mammifère ; et/ou

iv) ladite quantité extracorporelle dudit échantillon sanguin de sujet mammifère ne comprend pas de composants de plasma ; et/ou

v) ladite quantité extracorporelle dudit échantillon sanguin de sujet mammifère ne comprend pas les plaquettes, de préférence lesdites plaquettes ayant été séparées de ladite quantité extracorporelle dudit sang de sujet mammifère avant que ladite quantité extracorporelle dudit sang de sujet mammifère ne soit appliquée à ladite chambre d'écoulement.

9. Procédé selon les revendications 1 ou 8, dans lequel ladite quantité extracorporelle dudit sang de sujet de mammifère a été obtenue par aphérèse, de préférence :

i) ladite quantité extracorporelle dudit sang de sujet mammifère a été obtenue en isolant les leucocytes par aphérèse ; et/ou

ii) ladite quantité extracorporelle dudit sang de sujet mammifère est obtenue en isolant les revêtements buffy par aphérèse ; et/ou

iii) ladite quantité extracorporelle dudit sang de sujet mammifère ne comprend pas de composants de plasma ; et/ou

iv) ladite quantité extracorporelle dudit sang de sujet mammifère ne comprend pas de plaquettes, de préférence lesdites plaquettes ayant été séparées de ladite quantité extracorporelle dudit sang de sujet mammifère avant que ladite quantité extracorporelle dudit sang de sujet mammifère ne soit appliquée à ladite chambre d'écoulement.

10. Procédé selon l'une quelconque des revendications 1 à 9, dans lequel ladite chambre d'écoulement a des dimensions d'environ 1 $\mu$m à environ 400 $\mu$m compris de hauteur et d'environ 1 $\mu$m à environ 400 $\mu$m compris de largeur ; dans lequel de préférence ladite chambre d'écoulement a des dimensions d'environ 5 $\mu$m à environ 300 $\mu$m compris de hauteur et d'environ 5 $\mu$m à environ 300 $\mu$m compris de largeur ; dans lequel davantage de préférence ladite chambre d'écoulement a des dimensions d'environ 10 $\mu$m à environ 250 $\mu$m compris de hauteur et d'environ 10 $\mu$m à environ 250 $\mu$m compris de largeur ; dans lequel encore davantage de préférence ladite chambre d'écoulement a des dimensions d'environ 50 $\mu$m à environ 200 $\mu$m compris de hauteur et d'environ 50 $\mu$m à environ à 200 $\mu$m compris de largeur ; et dans lequel encore davantage de préférence ladite chambre d'écoulement a des dimensions d'environ 50 $\mu$m à environ 100 $\mu$m compris de hauteur et d'environ 50 $\mu$m à comprenant environ 100 $\mu$m compris de largeur.

11. Procédé selon la revendication 10, dans lequel ladite chambre d'écoulement est configurée pour prélever un volume compris entre environ 1 ml à environ 50 ml de ladite quantité extracorporelle dudit échantillon sanguin de sujet mammifère.

**12.** Procédé selon l'une quelconque des revendications 1 à 11, dans lequel

i) le matériau de ladite chambre d'écoulement n'est pas du plastique ; et de préférence ledit matériau non plastique est du verre ; ou

ii) le matériau de ladite chambre d'écoulement est du plastique ; de préférence ledit matériau en plastique étant choisi dans le groupe constitué par les acryliques, le polycarbonate, le polyétherimide, la polysulfone, la polyphénylsulfone, les styrènes, le polyuréthane, le polyéthylène, le téflon ou toute autre matière plastique de grade médical approprié.

**13.** Procédé selon l'une quelconque des revendications 6 à 12, dans lequel l'activation desdites plaquettes est obtenue en disposant les composants de plasma, qui sont compris dans ladite quantité extracorporelle dudit échantillon sanguin de sujet mammifère, sur la surface de ladite chambre d'écoulement de telle manière qu'au moins certaines desdites plaquettes peuvent interagir avec lesdits composants de plasma et sont immobilisées sur la surface de la chambre d'écoulement ; de préférence :

i) l'activation desdites plaquettes est obtenue en disposant des protéines choisies dans le groupe comprenant le fibrinogène, la fibronectine et le composant gamma du fibrinogène sur la surface de ladite chambre d'écoulement de telle manière qu'au moins certaines desdites plaquettes peuvent interagir avec lesdites protéines et sont immobilisées sur la surface de ladite chambre d'écoulement ; et davantage de préférence l'activation desdites plaquettes est obtenue en disposant le fibrinogène sur la surface de ladite chambre d'écoulement de telle manière qu'au moins certaines desdites plaquettes peuvent interagir avec ladite fibronectine et sont immobilisées sur la surface de ladite chambre d'écoulement ; et/ou

ii) lesdites plaquettes sont passées à travers ladite chambre d'écoulement sous une force de cisaillement de 0,1 à 10,0 dynes/cm$^2$, de préférence dans une plage d'environ 0,1 à environ 2,0 dynes/cm$^2$ ; et/ou

iii) l'activation des plaquettes peut être contrôlée par l'expression de la P-sélectine et/ou de l'$\alpha$IIb-$\beta$3-intégrine.

**14.** Procédé selon l'une quelconque des revendications 1 à 13, dans lequel

i) lesdits monocytes sont passés à travers ladite chambre d'écoulement avec un débit d'environ 10 ml/minute à environ 200 ml/minute pour produire une force de cisaillement d'environ 0,1 à environ 20,0 dynes/cm$^2$ ; et/ou

ii) lesdits monocytes sont activés et induits pour se différencier en cellules dendritiques autologues immunostimulatrices en passant lesdits monocytes à travers ladite chambre d'écoulement sous une force de cisaillement d'environ 0,1 à environ 10,0 dynes/cm$^2$, de préférence une force d'environ 0,1 à environ 1,0 dyne/cm$^2$ de telle manière que lesdits monocytes peuvent se lier auxdites plaquettes activées.

**15.** Procédé selon l'une quelconque des revendications 1 à 14 pour obtenir des cellules dendritiques immunostimulatrices autologues.

**16.** Procédé selon l'une quelconque des revendications 1 à 14 pour obtenir des cellules dendritiques immunostimulatrices allogènes.

**17.** Procédé selon l'une quelconque des revendications 1 à 15 pour obtenir des cellules dendritiques immunostimulatrices autologues fonctionnellement spécifiques individuelles et synchronisées de manière mature.

Figure 1

a)

b)

Figure 2

Figure 3

Figure 4

Figure 5

a)

Fibrinogen

b)

Fibronectin

Figure 6

Figure 7

Figure 8

Figure 9

Figure 10

Figure 11

Figure 12

Figure 13

Figure 14

Figure 15

Figure 16

Figure 17

Figure 18

a)

b)

Figure 18 cont.

c)

d)

Figure 19

A)

B)

Figure 20

Figure 21

Figure 22

Figure 23

| | HLA-DR | CD86 | ICAM-1 | PLAUR | FSC/SSC Complexity |
|---|---|---|---|---|---|
| Fresh (Ficoll) PBMC | 0 | 0 | 0 | 0 | 0 |
| D1 PBMC | ++ | 0 | ++ | ++ | + |
| PP D1 PBMC | +++ | + | ++++ | ++++ | +++ |
| Immature Fast DC | ++++ | +++ | ++ | – | ? |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011137365 A

- US 6524855 B

**Non-patent literature cited in the description**

- **GIRARDI et al.** *Transfusion and Apheresis Science,* 2002, vol. 26, 181-190
- **BERGER et al.** *Blood,* 2010, vol. 116, 4838-4847
- **ANDREU et al.** *Trans. Sci.,* 1994, vol. 15 (4), 443-454
- **BRUIL et al.** *Transfusion Medicine Reviews,* 1995, vol. IX (2), 145-166
- **ANDREU et al.** *Transf. Sci.,* 1994, vol. 15 (4), 443-454
- **BIOLEY et al.** *The Journal of Immunology,* 2006, vol. 177, 6769-6779
- **WAECKERLE-MEN et al.** *Adv Drug Deliv Rev,* 2005, vol. 57, 475-82
- **BERGER C ; HOFFMANN K ; VASQUEZ JG ; MANE S ; LEWIS J ; FILLER R et al.** Rapid generation of maturationally synchronized human dendritic cells: contribution to the clinical efficacy of extracorporeal photochemotherapy. *Blood,* 2010, vol. 116 (23), 4838-4847
- **CELLA M ; SCHEIDEGGER D ; PALMERLEHMANN K ; LANE P ; LANZAVECCHIA A ; ALBER G.** Ligation of CD40 on dendritic cells triggers production of high levels of interleukin-12 and enhances T cell stimulatory capacity: T-T help via APC activation. *Journal of Experimental Medicine,* 1996, vol. 184 (2), 747-752
- **DE SAINT-VIS B ; VINCENT J ; VANDENABEELE S ; VANBERVLIET B ; PIN JJ ; AIT-YAHIA S et al.** A novel lysosome-associated membrane glycoprotein, DC-LAMP, induced upon DC maturation, is transiently expressed in MHC class II compartment. *Immunity,* 1998, vol. 9 (3), 325-336
- **SLAVIK JM ; HUTCHCROFT JE ; BIERER BE.** CD80 and CD86 are not equivalent in their ability to induce the tyrosine phosphorylation of CD28. *Journal of Biological Chemistry,* 1999, vol. 274 (5), 3116-3124
- **KANG HK ; LEE HY ; KIM MK ; PARK KS ; PARK YM ; KWAK JY et al.** The synthetic peptide Trp-Lys-Tyr-Met-Val-D-Met inhibits human monocyte-derived dendritic cell maturation via formyl peptide receptor and formyl peptide receptor-like 2. *Journal of Immunology,* 2005, vol. 175 (2), 685-692

- **RIPOLL VM ; IRVINE KM ; RAVASI T ; SWEET MJ ; HUME DA.** Gpnmb is induced in macrophages by IFN-gamma and lipopolysaccharide and acts as a feedback regulator of proinflammatory responses. *Journal of Immunology,* 2007, vol. 178 (10), 6557-6566
- **CHEN SQ ; SPRINGER TA.** Selectin receptor-ligand bonds: Formation limited by shear rate and dissociation governed by the Bell model. *Proceedings of the National Academy of Sciences of the United States of America,* 2001, vol. 98 (3), 950-955
- **THOMAS WE.** Understanding the counterintuitive phenomenon of catch bonds. *Current Nanoscience,* 2007, vol. 3, 63-83
- **XIONG JP ; STEHLE T ; ZHANG RG ; JOACHIMIAK A ; FRECH M ; GOODMAN SL et al.** Crystal structure of the extracellular segment of integrin alpha V beta 3 in complex with an Arg-Gly-Asp ligand. *Science,* 2002, vol. 296 (5565), 151-155
- **WEISEL JW ; NAGASWAMI C ; VILAIRE G ; BENNETT JS.** Examination of the Platelet Membrane Glycoprotein-IIB-IIIA Complex and its Interaction with Fibrinogen and Other Ligands by Electron-Microscopy. *Journal of Biological Chemistry,* 1992, vol. 267 (23), 16637-16643
- **KAPLAN KL ; BROEKMAN MJ ; CHERNOFF A ; LESZNIK GR ; DRILLINGS M.** Platelet alpha-granule proteins - studies on release and subcellular-localization. *Blood,* 1979, vol. 53 (4), 604-618
- **RUOSLAHTI E.** RGD and other recognition sequences for integrins. *Annual Review of Cell and Developmental Biology,* 1996, vol. 12, 697-715
- **ZHOU LJ ; TEDDER TF.** CD14+ blood monocytes can differentiate into functionally mature CD83+ dendritic cells. *Proc. Natl. Acad. Sci. U.S.A.,* 1996, vol. 93 (6), 2588-2592
- **KLEIN E.** CD83 localization in a recycling compartment of immature human monocyte-derived dendritic cells. *International Immunology,* 2005, vol. 17 (4

- **RENZO MD ; RUBEGNI P ; PASQUI AL et al.** Extracorporeal photopheresis affects interleukin (IL)-10 and IL-12 production by monocytes in patients with chronic graft-versus-host disease. *Br J Dermatol.,* 2005, vol. 153 (1), 59-65

- Repair of 8-MOP photoadducts in human lymphocyte. **GASPARRO FP ; BEVILACQUA PM ; GOLDMINZ D et al.** DNA Damage and Repair in Human Tissues. Plenum Press
- **STEINMAN RM ; HAWIGER D ; NUSSENZWEIG MC.** Tolerogenic dendritic cells. *Annu. Rev. Immunol.,* 2003, vol. 21, 685-711